# EUROPEAN PATENT APPLICATION

(11) **EP 0 735 015 A2**
(43) Date of publication of application: **02.10.1996**
(21) Application number: 96300655.6
(22) Date of filing: 30.01.1996
(51) Int. Cl.: C07B 59/00, C09K 19/04

(54) **A liquid crystal compound having (a) deuterium atom (s) and a liquid crystal composition comprising the said compound**

(30) Priority: 31.03.1995 JP 100105/95
(71) Applicant: CHISSO CORPORATION, Osaka (JP)
(72) Inventor: Koizumi, Yasuyuki, Ichiharashi Chibaken (JP); Demus, Dietrich, D-06118 Halle (DE); Matsui, Shuichi, Ichiharashi, Chibaken (JP); Miyazawa, Kazutoshi, Ichiharashi, Chibaken (JP); Sekiguchi, Yasuko, Ichiharashi, Chibaken (JP); Nakagawa, Etsuo, Ichiharashi, Chibaken (JP)
(74) Representative: Ruffles, Graham Keith

(57) **Abstract**

Object It is possible to control a pitch without any increase of a viscosity and any decrease of a voltage holding ratio etc. in various liquid crystal compositions.

Structure Provision of a liquid crystal compound with (an) optically active carbon atom(s) bonded to (a) deuterium atom(s) and a liquid crystal compound having characteristics suitable for various liquid crystal display methods at the same time.

Effect It is possible to prepare a liquid crystal composition without use of a chiral dopant. It is possible to control a pitch length and a spiral direction by selection of a stereochemistry in the compound and (a) position(s) of the deuterium atom(s).

## Description

### BACKGROUND OF THE INVENTION

### 1. Field of the Invention

This invention relates to a novel liquid crystal compound expressing preferable physical properties as an electrooptic display material, and a liquid crystal composition with such preferable physical properties containing the said novel liquid crystal compound.

### 2. Description of Related Art

Liquid crystal display elements are used for clocks, electronic computers, various metering machineries, panels for automobiles, word processors, electronic notebooks, printers, computers and television sets etc. The liquid crystal display elements utilize optical anisotropy and dielectric anisotropy properties of the liquid crystal compounds, and there are known a twist nematic type (TN type), a supertwist nematic type (STN type), a dynamic scattering type (DS type), a guest-host type (G-H type), a thin film transistor type (TFT type), and a ferroelectric liquid crystal (FLC) etc. as displaying methods. Also, as methods for driving them, there are known a static driving method, a time divided driving method, an active matrix driving method, and 2-frequency driving method etc.

Into the liquid crystal compositions used for these various display methods, there is generally added a chiral dopant with the intentions to keep constant a twist direction of liquid crystal molecules in a cell for liquid crystal display constant, to control a reverse-twist action of the liquid crystal molecules and to keep constant a display quality. The following optical active liquid crystal compounds are used in addition to cholesteric liquid crystals as the chiral dopants to be used (Kagaku Sosetsu, Vol. 22, Chemistry of Liquid Crystal p.50).

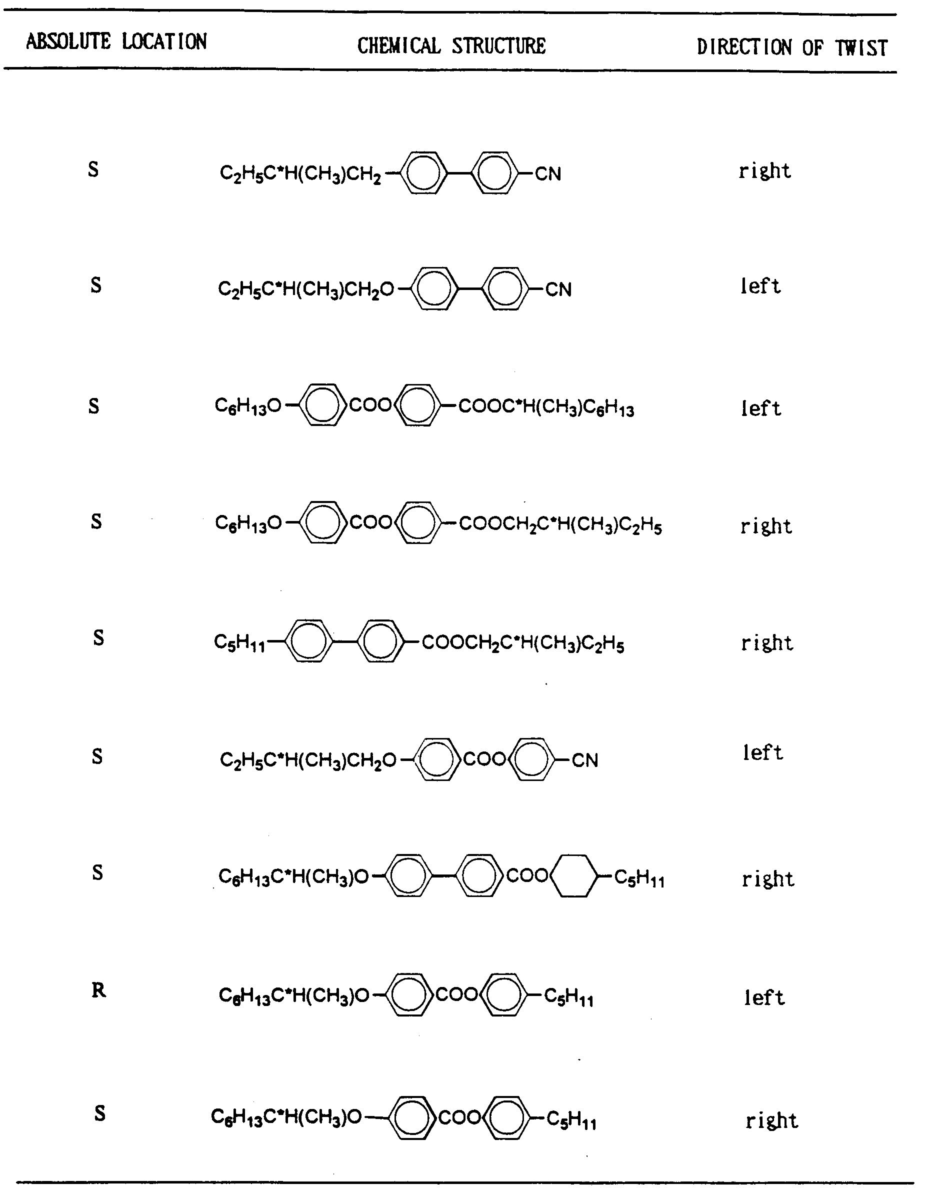

All structures of these chiral dopants have branched methyl groups in optically active groups, and also have ester bonds in molecules and cyano groups etc. in end groups. Thus, there are disadvantages such that 1) they show higher viscosities than those of nematic liquid crystals, and 2) they are less stable chemically because of the contained ester bonds and cyano groups. 3) the clearing temperatures due to the branched structure of the compounds are decreased. Therefore, if they are used in large amounts as the chiral dopants, they will have disadvantages to elevate viscosities of original nematic liquid crystals, by which a voltage holding ratio (VHR) or a resistance value are lowered.

Additionally, there is known that a liquid crystal temperature range and a temperature characteristic of threshold voltage (Vth) in the obtained composition may be strongly changed.

Furthermore, a pitch of the liquid crystal composition in which the above-mentioned optical active liquid crystal compound being used as the chiral dopant is strongly changed according to the temperature and unevenness of a display quality is produced due to the used temperature. In order to correct those phenomena, multiple chiral dopants having different temperature characteristics have been tried to use (Emoto and el al., Toku-Kai-Sho 63-22893), but an operation for realizing the aimed temperature characteristic is very complex and thus chiral dopants having good temperature characteristics about the pitch are expected.

As described above, there has been desired a development of a liquid crystal compound to improve the above-mentioned problems.

### Problems to be solved by the Invention

We inventors studied seriously in order to solve the above-mentioned problems, and thus found a compound having a novel structure and improved characteristics compared to the known liquid crystal compounds.

That is, we found a compound with (an) optical active carbon atom(s) bonded to (a) deuterium atom(s) and improved characteristics compared to the known liquid crystal compounds, to complete this invention.

That is, an object of the invention is that in various liquid crystal composition exemplified to TN, STN and TFT to provide a liquid crystal compound, a liquid crystal composition and a liquid crystal display element with use of them, by which a pitch control can be carried out easily without any change such as increase of viscosity, decrease of clearing temperature, decrease of voltage holding ratio or decrease of specific resistance value, and also which have optimal characteristics for various liquid crystal display methods.

### SUMMARY OF THE INVENTION

The invention comprises the following items (1) to (12).
(1) An optically active compound with (an) optically active carbon atom(s) bonded to (a) deuterium atom(s).
(2) A liquid crystal compound expressed by the general formula (1) 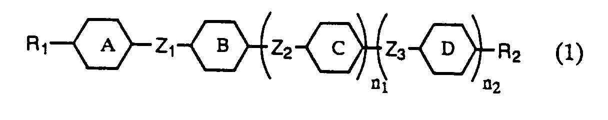 in which,
   each of R₁ and R₂ independently denotes a hydrogen atom, a cyano group, a halogen atom, or an alkyl group or a halogenated alkyl group with 1 to 20 carbon atoms, wherein one or more than one methylene group in the said groups may be substituted by an oxygen atom, a sulfur atom, CH=CH, C≡C, CO, CF=CF, CF₂, or a cycloalkane ring or a cycloalkene ring with 3 to 5 carbon atoms, but two or more than two methylene groups cannot be continuously substituted by (an) oxygen atom(s) or (a) sulfur atom(s),
   each of Z₁, Z₂ and Z₃ independently denotes a covalent bond or an alkylene group with 1 to 4 carbon atoms, wherein one or more than one methylene group in the said groups may be substituted by an oxygen atom, a sulfur atom, CH=CH, C≡C, CO, CF=CF, CF2, or a cycloalkane ring or a cycloalkene ring with 3 to 5 carbon atoms, but two or more than two methylene groups cannot be continuously substituted by (an) oxygen atom(s) or (a) sulfur atom(s),
   n₁ and n₂ are 0 or 1,
   each of rings A, B, C and D independently denotes a benzene ring, a bicyclo[1.1.1]pentane ring, a bicyclo[2.1.1]hexane ring, a bicyclo[2.2.1]heptane ring, a bicyclo[2.2.2]octane ring, a naphthalene ring, a 1,2,3,4-tetrahydronaphthalene ring, a perhydronaphthalene ring, a fluorene ring, a phenanthrene ring, a 9,10-dihydrophenanthrene ring, an indane ring, an indene ring or a cycloalkane ring or (a) cycloalkene ring with 4 to 20 carbon atoms, wherein the carbon atom in the said rings may be substituted by a oxygen atom, a sulfur atom or a nitrogen atom,
   one or more than one hydrogen atom in R₁, R₂, Z₁, Z₂, Z₃, rings A, B, C or D is substituted by (a) deuterium atom(s), and the carbon atom bonded to the deuterium atom(s) is (are) (an) asymmetric carbon atom(s) and optically active, and the hydrogen atom(s) in the structure may be substituted by (a) halogen atom(s) or a cyano group.
(3) A liquid crystal compound according to the above-mentioned item (2), wherein one or more than one hydrogen atom in R₁ or R₂ is (are) substituted by (a) deuterium atom(s).
(4) A liquid crystal compound according to the above-mentioned item (2), wherein one or more than one hydrogen atom in Z₁, Z₂ or Z₃ is (are) substituted by (a) deuterium atom(s).
(5) A liquid crystal compound according to the above-mentioned item (2), wherein one or more than one hydrogen atom in rings A, B, C or D is (are) substituted by (a) deuterium atom(s).
(6) An intermediate for synthesizing a liquid crystal compound expressed by the general formula (1), wherein one or more than one hydrogen atom is substituted by (a) leaving group(s) such as an iodine atom, a bromine atom, a chlorine atom, a fluorine atom, a hydroxyl group, an alkanoyloxy group, a methanesulfonyl group, a benzenesulfonyl group or a 4-substituted benzenesulfonyloxy group, and wherein the carbon atom(s) bonded to the leaving group(s) is (an) asymmetric carbon atom(s).
(7) A liquid crystal composition which is consistuted by using at least one liquid crystal compound according to any of the above-mentioned items (1) to (5).
(8) A liquid crystal composition characterized in that at least one liquid crystal compound according to any of the above-mentioned items (1) to (5) is contained as the first component and at least one compound selected from the group consisting of general formulae (2), (3) and (4) 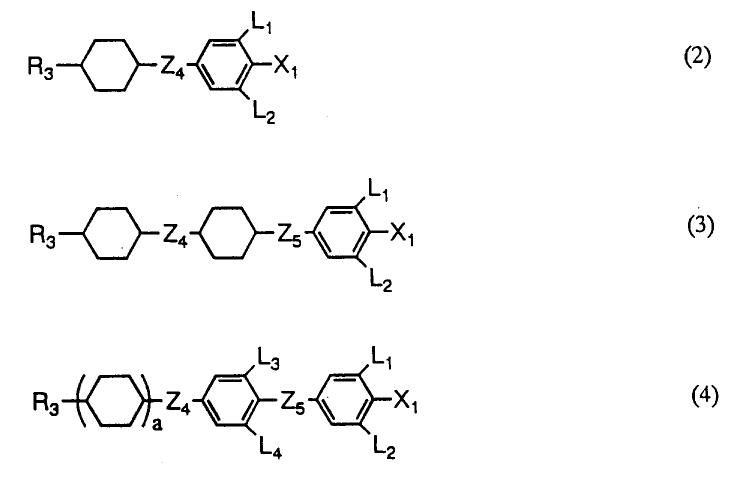 in which, R₃ denotes an alkyl group with 1 to 10 carbon atoms, X₁ denotes F, Cℓ, OCF₃, OCF₂H, CF₃, CF₂H or CFH₂, each of L₁, L₂, L₃ and L₄ independently denotes H or F, each of Z₄ and Z₅ independently denotes (CH₂)₂, CH=CH or a covalent bond, and a denotes 1 or 2,
   is contained as the second component.
(9) A liquid crystal composition characterized in that at least one liquid crystal compound according to any of the above-mentioned items (1) to (5) is contained as the first component and at least one compound selected from the group consisting of general formulae (5), (6), (7), (8) and (9) 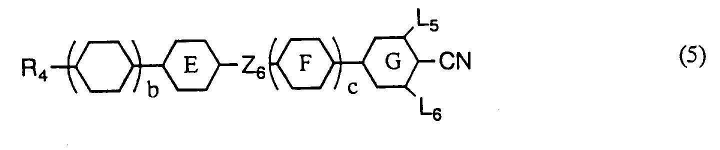 in which, R₄ denotes F, an alkyl group with 1 to 10 carbon atoms or an alkenyl group with 2 to 10 carbon atoms, wherein an optional methylene group in the said alkyl group or the alkenyl group may be substituted by (an) oxygen group(s) but two or more than two methylene groups cannot be continuously substituted by (an) oxygen atom(s), ring E denotes a cyclohexane ring, a benzene ring, a pyrimidine ring or a 1,3-dioxane ring, ring F denotes a cyclohexane ring, a benzene ring or a pyrimidine ring, ring G denotes a cyclohexane ring or a benzene ring, Z₆ denotes (CH₂)₂, COO or a covalent bond, each of L₅ and L₆ independently denotes H or F, and each of b and c independently denotes 0 or 1, 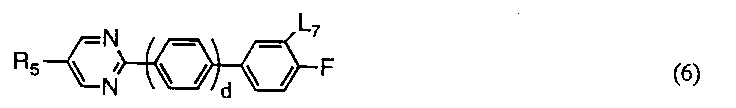 in which, R₅ denotes an alkyl group with 1 to 10 carbon atoms, R₇ denotes H or F, and d denotes 0 or 1, 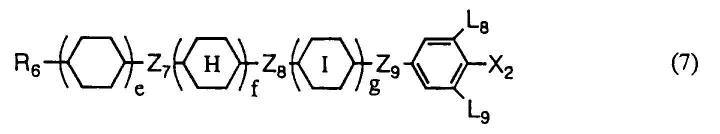 in which, R₆ denotes an alkyl group with 1 to 10 carbon atoms, each of rings H and I independently denotes a cyclohexane ring or a benzene ring, each of Z₇ and Z₈ independently denotes COO or a covalent bond, Z₉ denotes COO or C≡C, each of L₈ and L₉ independently denotes H or F, X₂ denotes F, OCF₃, OCF₂H, CF₃, CF₂H or CFH₂, but in the case that X₂ denotes OCF₃, OCF₂H, CF₃, CF₂H or CFH₂, both of L₈ and L₉ denote H, and each of e, f and g independently denotes 0 or 1, 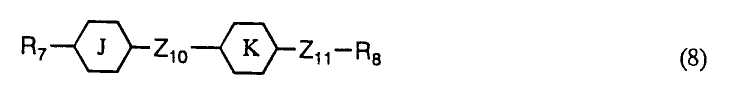 in which, each of R₇ and R₈ independently denotes an alkyl group with 1 to 10 carbon atoms or an alkenyl group with 2 to 10 carbon atoms, wherein (an) optional methylene group(s) thereof may be substituted by (an) oxygen atom(s) but two or more than two methylene groups cannot be continuously substituted by oxygen atoms, ring J denotes a cyclohexane ring, a benzene ring or a pyrimidine ring, ring K denotes a cyclohexane ring or a benzene ring, Z₁₀ denotes C≡C, COO, (CH₂)₂, CH=CH-C≡C or a covalent bond, and Z₁₁ denotes COO or a covalent bond, 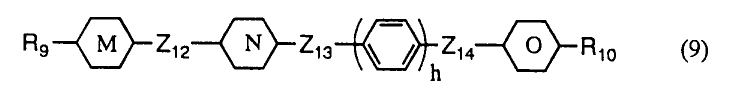 in which, each of R₉ and R₁₀ independently denotes an alkyl group with 1 to 10 carbon atoms or an alkenyl group with 2 to 10 carbon atoms, wherein (an) optional methylene group(s) thereof may be substituted by (an) oxygen atom(s) but two or more than two methylene groups cannot be continuously substituted by oxygen atoms, ring M denotes a cyclohexane ring, a benzene ring or a pyrimidine ring, ring N denotes a cyclohexane ring or a benzene ring in which one or more than one hydrogen atom may be substituted by F or (a) pyrimidine ring(s), ring O denotes a cyclohexane ring or a benzene ring, each of Z₁₂ and Z₁₄ independently denotes COO, (CH₂)₂ or a covalent bond, and Z₁₃ denotes CH=CH,
   C≡C, COO or a covalent bond, and h denotes 0 or 1, is contained as the second component.
(10) A liquid crystal composition characterized in that at least one liquid crystal compound according to any of the above-mentioned items (1) to (5) is contained as the first component, at least one compound selected from the group consisting of general formulae (2), (3) and (4) is contained as a part of the second component, and at least one compound selected from the group consisting of general formulae (5), (6), (7), (8) and (9) is contained as another part of the second component.
(11) A liquid crystal display element which is constituted by using a liquid crystal composition according to any of the above-mentioned items (7) to (10).
(12) A method for producing a compound of the general formula (1), characterized in that a racemic compound in which (a) hydrogen atom(s) being substituted by (a) hydroxyl group(s) is resoluted optically to obtain an optically active alcoholic substance, and that a hydroxyl part thereof is converted to (a) halogen or leaving group(s), and thereafter substituted by (a) deuterium atom(s).

As compounds of general formula (1) used in the invention, the followings are preferably mentioned:

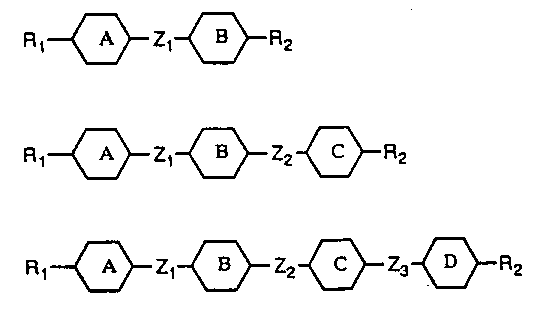

### Brief Description of the Drawing

### Fig 1

It shows a relationship between an amount of a chiral dopant according to the invention added and a viscosity of a liquid crystal composition.

### DETAILED DESCRIPTION OF THE INVENTION

It is known to substitute a hydrogen atom by a deuterium atom in the liquid crystal compound, and there have been reported some examples as described below:
a) H. Gasparoux and et al., Ann. Rev. Phys. Chem., 27, 175 (1976),
b) G. W. Gray and et al., Mol. Cryst. Liq. Cryst., 41, 75 (1977),
c) A. J. Leadbetter and et al., J. Phys. [Paris] coll. C₃, 40, 125 (1979),
d) A. Kolbe and et al, Z. Naturforsch., 23a, 1237 (1968),
e) J. D. Rowell and et al, J. Chem. Phys., 43, 3442 (1965),
f) W. D. Philips and et al, J. Chem. Phys., 41, 2551 (1964),
g) A. F. Matins and et al, Mol. Cryst. Liq. Cryst., 14, 85 (1971),
h) E. T. Samulski and et al., Phys. Rev. Lett., 29, 340 (1972),
i) Takehara and et al., Toku-Kai-Hei 6-312949.

As to these reports, however, the literature (a) disclosed a liquid crystal compound in which (a) hydrogen atom(s) on (a) benzene ring(s) at (a) carboxylic acid position(s) of 4-alkoxybenzoic acid being substituted by (a) deuterium atom(s), the literatures (b) ∼ (h) disclosed liquid crystals compound in which (a) hydrogen atom(s) in (a) terminal alkyl group(s) being substituted by (a) deuterium atom(s), and the literature (i) disclosed a liquid crystal compound in which (a) hydrogen atom(s) in (a) cyclohexane ring(s) being substituted by (a) deuterium atom(s), thus those literatures didn't disclose any liquid crystal compound having (an) asymmetric and optically active carbon atom(s) bonded to (a) deuterium atom(s).

The compound of the general formula (1) according to the invention is a liquid crystal compound having (an) asymmetric and optically active carbon atom(s) bonded to (a) deuterium atom(s), the said compound being found first by the present inventors.

The term "a liquid crystal compound" used in the invention means a compound having characteristics suitable for producing a constitutive element of a liquid crystal composition.

The compound of the general formula (1) according to the invention is a compound having a characteristic as a chiral dopant in addition to the similar characteristics to the corresponding compounds without any substituted deuterium atom.

As described above, in contrast to the conventional dopants which are more unstable chemically and also changed more in their characteristics such as a viscosity, a voltage holding ratio (VHR), a specific resistance value and a liquid crystal temperature range than the conventional nematic liquid crystal compounds, the compound of the general formula (1) according to the invention shows quite equal physical properties to those of a compound without any substitution by a deuterium. Hitherto, the chiral dopants are used for the intention to control a reverse-twist of liquid crystal molecules in spite of the above-mentioned difficult points about changes in characteristics, but now by using the compound according to the invention it becomes possible first to prepare a liquid crystal composition without any conventional chiral dopant.

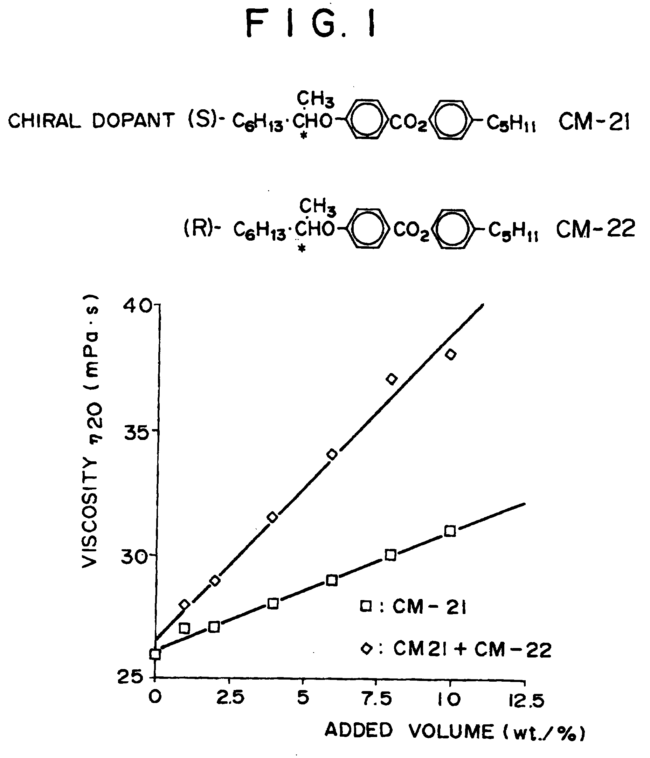

Generally, increase in viscosity by addition of the chiral dopant is due to a helical twist structure formed by the addition. However, even if any helical twist structure is not formed, viscosity is increased only by adding the chiral dopant. Relationships between added amounts of chiral dopants CM-21 or CM-22 made by Chisso Co., Ltd. into liquid crystal compositions and viscosities are shown in Fig.1.

As being made obviously from the above-mentioned matter, the viscosity of the composition in which CM-21 and reverse-twisted CM-22 are added in equal amounts is increased even if any helical twist structure being not formed. Namely, as described above, it is recognized that the viscosity is increased only by adding the conventional chiral dopant with a branched methyl group in its structure.

On the other hand, since the compound of the general formula (1) according to the invention has no branched methyl group, the above-mentioned increase in the viscosity cannot be caused.

When the compound of the general formula (1) according to the invention is used, it is possible to control optionally a pitch length (a twist power) or a pretilt angle by selecting a stereochemistry of the compound, (a) position(s) of (a) deuterium atom(s), a number of substitutions or an optical purity thereof.

Furthermore, the compound of the general formula (1) according to the invention is an optical active liquid crystal compound and thus it is useful as a constitutive component for a ferroelectric liquid crystal composition proposed by N. A. Clark and et al. (Appl. Phys. Lett., 36, 899 (1980))

A liquid crystal composition consists of several to more than 20 liquid crystal compounds in order to express optimal characteristics required for respective display elements. Thus, as characteristics of the liquid crystal compound, there are required a compatibility with the other liquid crystal compound, in particular recently a good compatibility at a low temperature for use in various environments. Furthermore, it is required that the liquid crystal composition should not express any deposition of crystal or a smectic phase, and also required a less temperature-dependency of viscosity at low temperature as possible, in order to become usable at a wide temperature range. Therefore, high compatibility at a low temperature of the used liquid crystal compound with the other liquid crystal compound is very important.

The compound of the general formula (1) according to the invention is a practical liquid crystal material which is superior in compatibility with the other liquid crystal material compared to the corresponding compound without substitution by a deuterium atom, wherein any deposition of crystal is not found at a low temperature range, and also any undesirable smectic phase is difficultly expressed. Furthermore, by using compounds having the same structure but with reverse stereochemistry which are included in the compounds of the general formula (1) according to the invention in equal quantities admixed each other, the compatibility at a low temperature is increased.

Although various characteristics are required for a liquid crystal material according to display methods and driving methods, the common and important characteristics are that 1) a temperature range of a liquid crystal phase being wide, and that 2) a viscosity being low. Amongst, characteristic 1) includes such matters that an upper limitation temperature of a nematic phase being high, and that a melting point being low and thus a phase separation such as a deposition of crystal at a low temperature range being difficultly formed.

Although properties of the liquid crystal compounds used in these various liquid crystal display elements are different according to their useful applications, all liquid crystal compounds are required to be stable against external environmental factors such as water, air, heat and light, and also required to show their liquid crystal phases in a wide temperature range as possible preferably near the room temperature.

Although any compound according to the invention shows preferable physical properties, it is possible to prepare different liquid crystal compositions having characteristics according to objects by using the compounds in which R₁, R₂, rings A, B, C and D, Z₁, Z₂ and Z₃ are selected appropriately.

A three rings-type compound ((3) and (4) of the general formula [2]) or a four rings-type compound (formula [3]) can be used particularly in the case of the composition in which the liquid crystal temperature range should be higher temperatures, but otherwise a two rings-type ((2) of formula [2]) or a three rings-type compound ((3) and (4) of the general formula [2]) can be used.

In the case that a low viscosity being an object, a compound having much unsaturated hydrocarbon sites in R₁, R₂, Z₁, Z₂ or Z₃ and much cyclohexane rings etc. in rings A, B, C and D is preferably used.

Similarly, in the case that a stable compound against external factors being an object, a compound having much unsaturated hydrocarbon sites or fluorine atoms, chlorine atoms, halogenated alkyl groups in R₁, R₂, Z₁, Z₂ or Z₃ may be used.

In the case that a particularly high dielectric anisotropy value being required, a compound having a positive dielectric anisolopy value (a P type compound) is used similar to the conventionally used composition, and the P type compound can be provided by selecting a halogen atom, a cyano group, or a halogenated alkyl group, a halogenated alkoxy group as R₁ in the formula (1). In the case that more dielectric anisotropy value being required, the object may be attained by introducing halogen atoms on rings A, B, C and D with directing dipoles in the same direction.

In order to obtain a compound in which a dielectric anisotropy value being negative (a N type compound), a group with less dipole moment such as alkyl group and alkoxy group may be introduced in R₁.

A refractive index anisotropy value may be controlled optionally by selecting kinds of rings A, B, C and D in (1). That is, in the case that more refractive index anisotropy value being required, a compound having much benzene rings may be used, and in the case that less refractive index anisotropy value being required, a compound having many saturated cyclic hydrocarbons such as cyclohexane rings may be used.

For a composition used in a STN driving system, a sharp threshold characteristic is required to realize a high image quality. This sharpness is a function of elastic constant ratio K33/K11, and it is known that the composition with the higher constant shows more sharp threshold characteristics (F. Leenhouts and et al., Proceedings of the Japan Display, 388 (1986)).

The elastic constant ratio can be also controlled by selecting R₁, R₂, rings A, B, C and D, Z₁, Z₂ and Z₃. That is, in the case that a high elastic constant ratio being required, a compound having a cyano group as R1 and an unsaturated hydrocarbon site in R₂, Z₁, Z₂ and Z₃ may be used. For example, the compound numbers 12, 115, 126 and 147 in Example 6 are preferable.

On the other hand, a liquid crystal composition designed for an active matrix liquid crystal display containing an integrated non-linear element for switching individual image points, particularly for TFT, should have a high positive dielectric anisotropy, as well as a high voltage holding ratio, a quite high specific resistance value and a good UV stability.

The active matrix liquid crystal displays are suitable particularly for television sets, or high information displays for computers, as well as for high information displays in automobiles and aircrafts. However, if a liquid crystal compound or a liquid crystal composition not possessing a high voltage holding ratio, a quite high specific resistance and a good UV stability is used, a contrast will be lowered according to decrease in an electric resistance of a liquid crystal panel, which results in a problem of "after image removal". A high electric resistance of the liquid crystal composition is a very important factor influencing on an useful life, particularly in the case of a low voltage driving. Therefore, the high voltage holding ratio, the quite high specific resistance and the good UV stability are very important characteristics required for the liquid crystal compound used.

In the case that the high voltage holding ratio and the good UV stability being required, an alkyl group, a fluoroalkyl group, a chloroalkyl group, a fluoroalkoxy group or a chloroalkoxy group may be selected as R₁, R₂, R₃, Z₁, Z₂ or Z₃, and a cycloalkane ring or a benzene ring may be selected as ring A, B, C or D. For example, the compound numbers 38, 117, 118, 120, 124, 151, 152, and 182 in Example 6 are mentioned.

The liquid crystal composition according to the invention preferably contains one or more than one compound shown by (1) at a proportion of 0.1 to 99.9% by weight, in order to express excellent characteristics.

In more detail, the liquid crystal composition provided by the invention is accomplished by introducing the first component containing at least one compound (1) and then mixing an optionally selected compound from the other compound group of the general formula (1) or the general formulae (2) to (9) according to the object of the liquid crystal composition.

The liquid crystal composition provided by the invention may comprise the first component containing at least one liquid crystal compound shown by the general formula (1), but one obtained by mixing thereto at least one compound selected from the group consisting of the above-mentioned general formulae (2), (3) and (4) (referred to the second A component hereinafter), and/or at least one compound selected from the group consisting of the above-mentioned general formulae (5), (6), (7), (8) and (9) (referred to the second B component hereinafter) are preferable, and furthermore the known compound can be mixed as the third component with the intention to control a threshold voltage, a liquid crystal phase temperature range, a refractive index anisotropy value, a dielectric anisotropy value and viscosity etc.

As preferable examples of the compounds included in the general formulae (2), (3) and (4) of the above-mentioned second A components, there may be listed (2-1) to (2-15), (3-1) to (3-48) and (4-1) to (4-55), respectively. 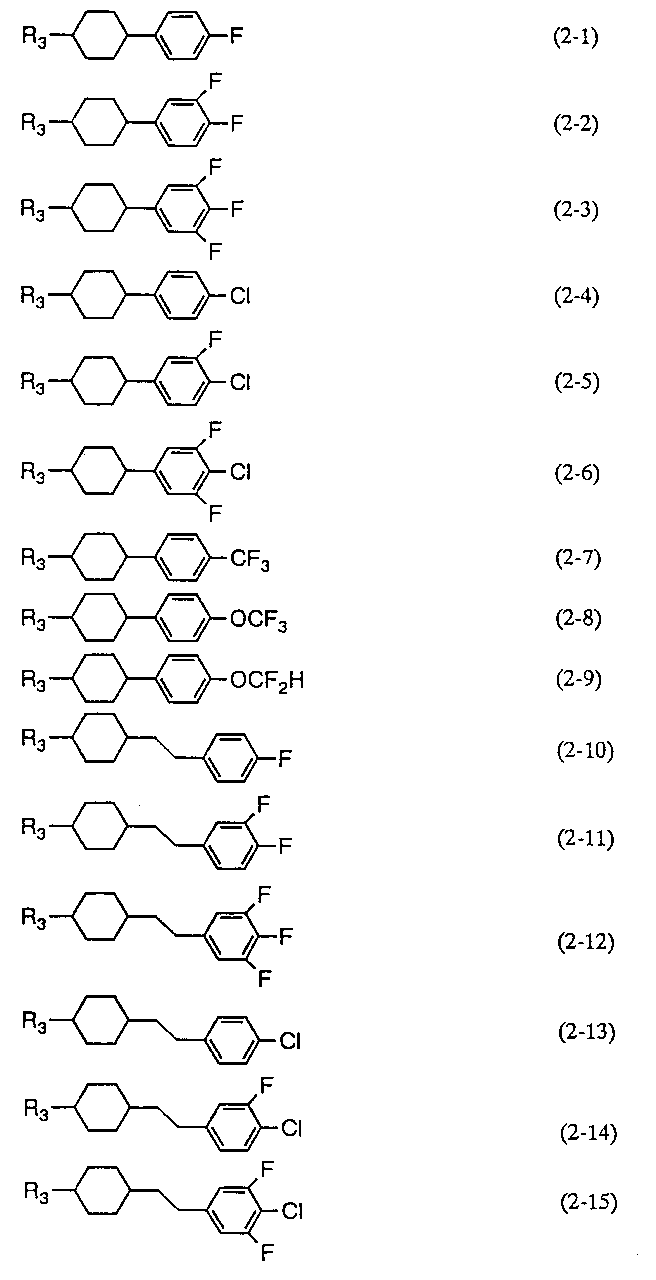 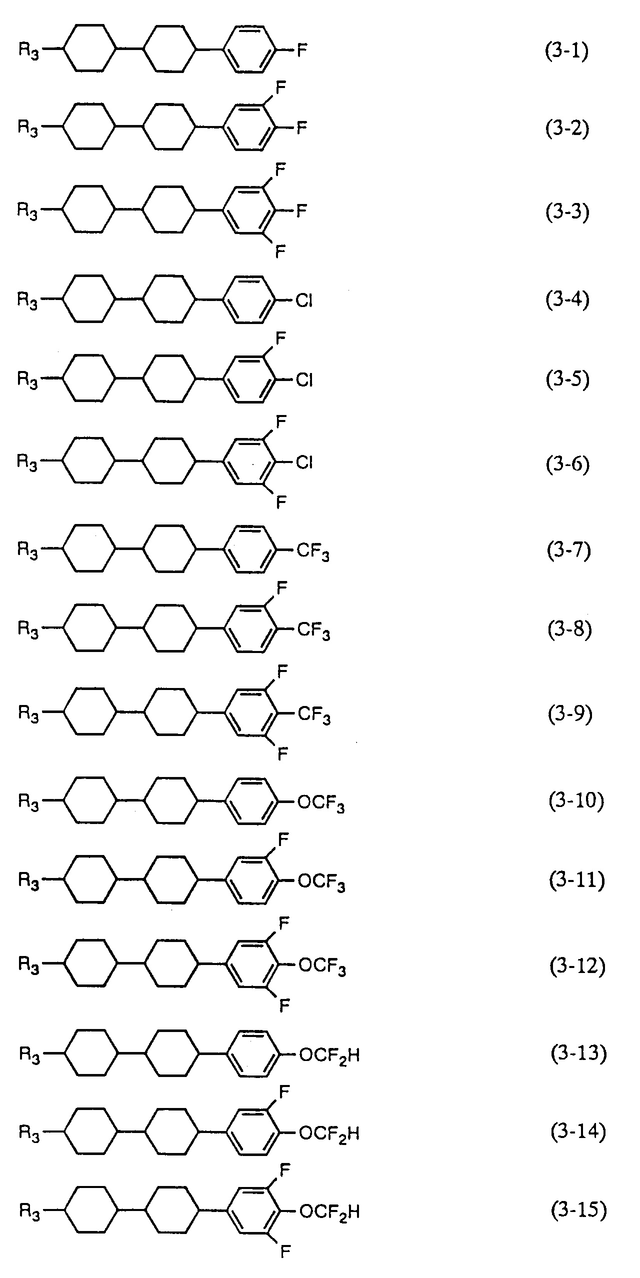 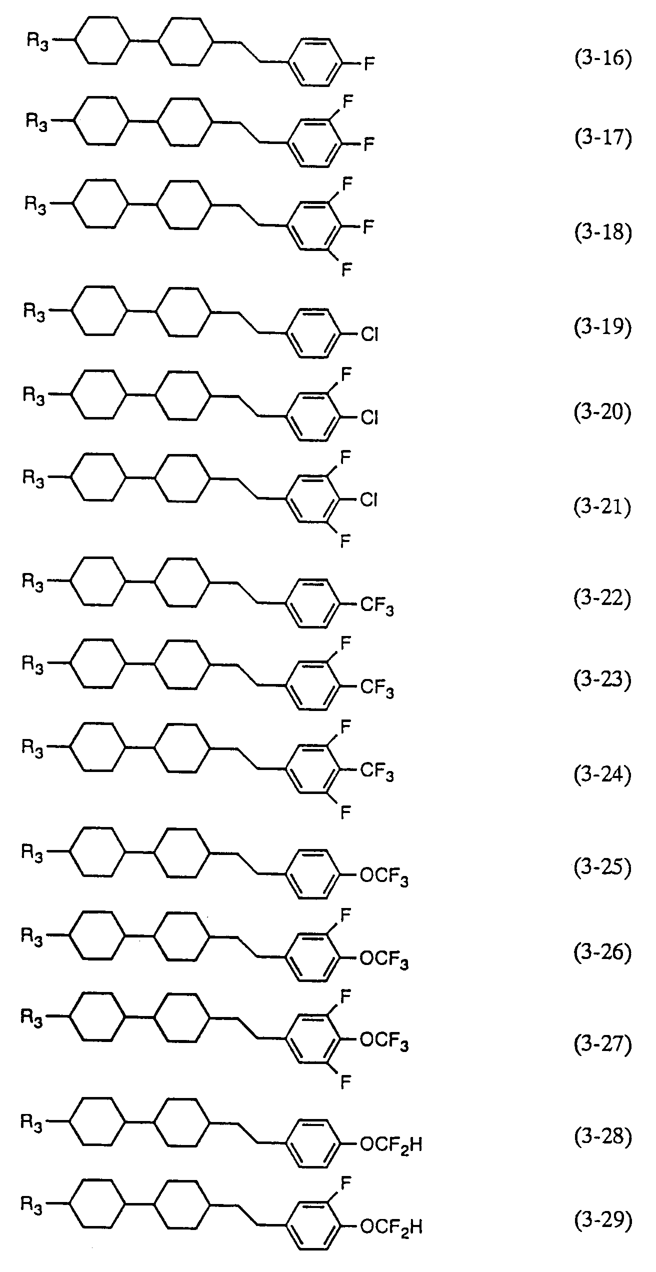 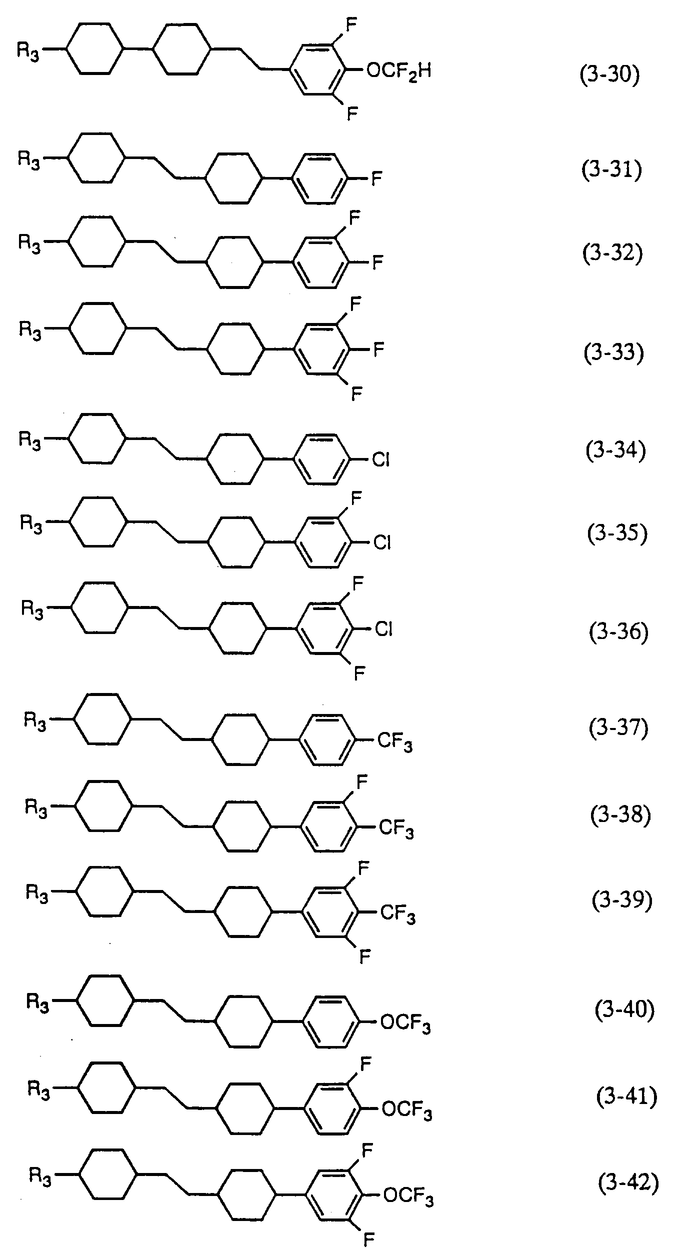 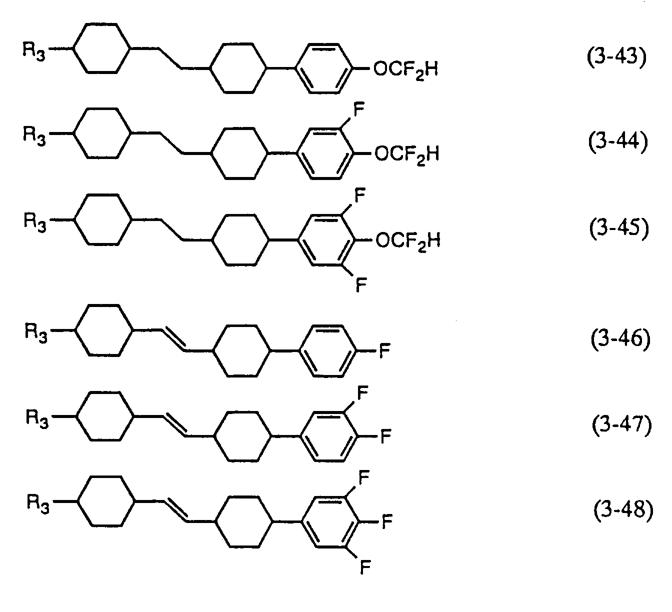 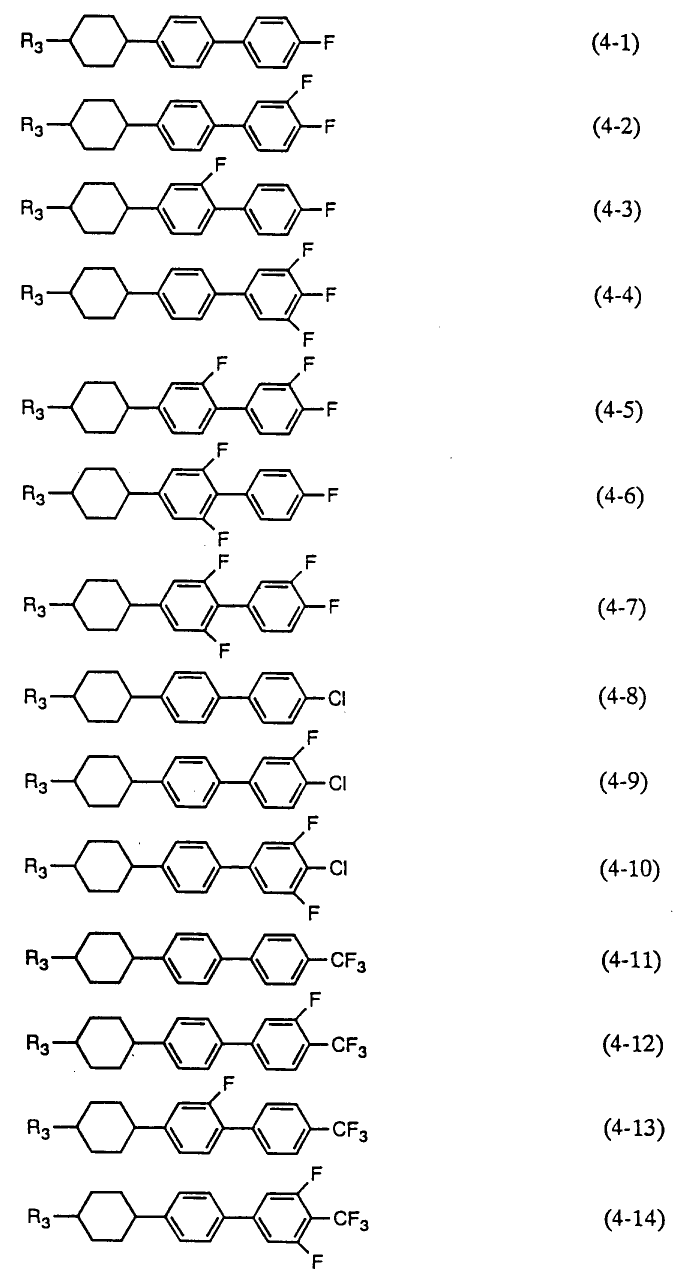 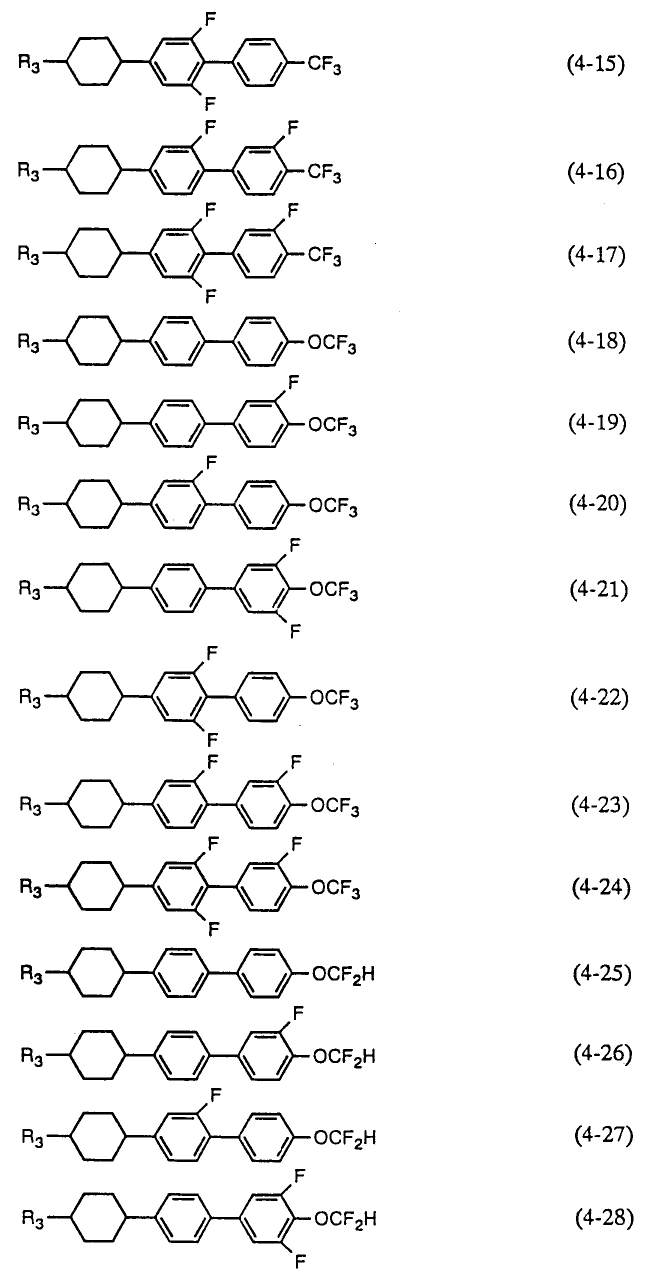 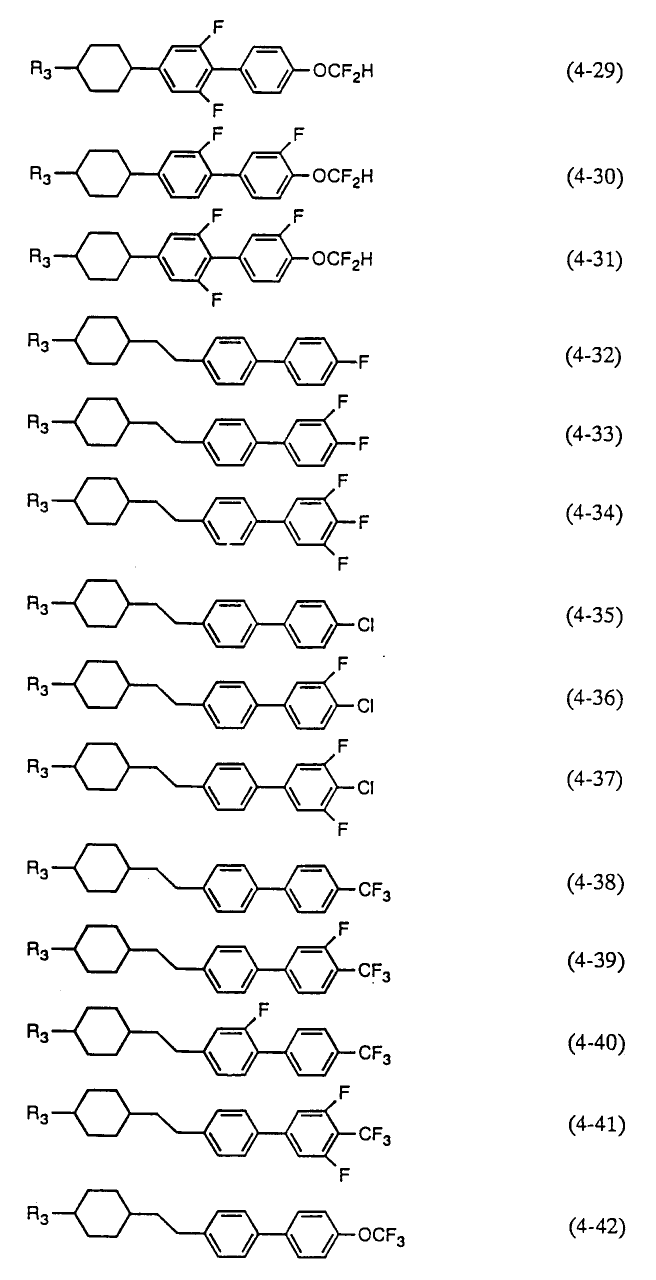 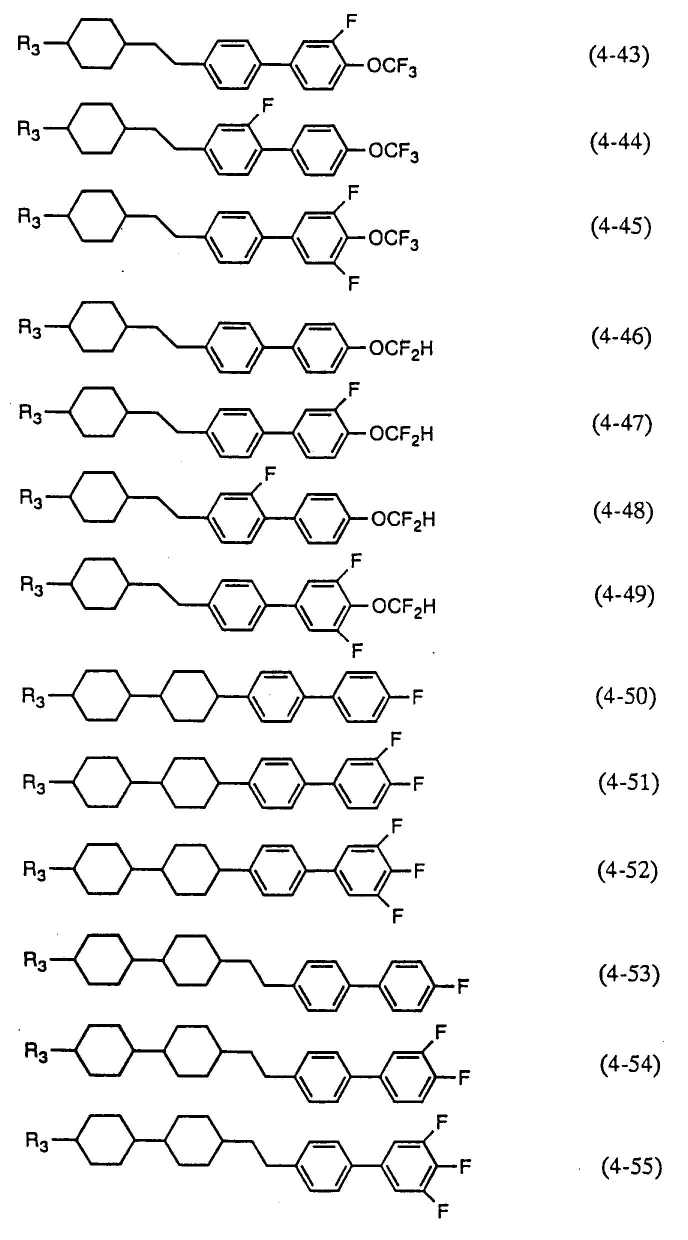 in which, R₃ denotes the same meaning as described above.

The compounds expressed by these general formulae (2) to (4) show positive dielectric anisotropy values and they are quite superior in heat stability and chemical stability.

The amount of the said compounds used are suitably within a range of 1 to 99% by weight, preferably 10 to 97% by weight, more preferably 40 to 95% by weight, to the total weight of the liquid crystal composition.

Then, as preferable examples of the compounds included in the general formulae (5), (6) and (7) of the above-mentioned second B components, there may be listed (5-1) to (5-24), (6-1) to (6-3) and (7-1) to (7-17), respectively. 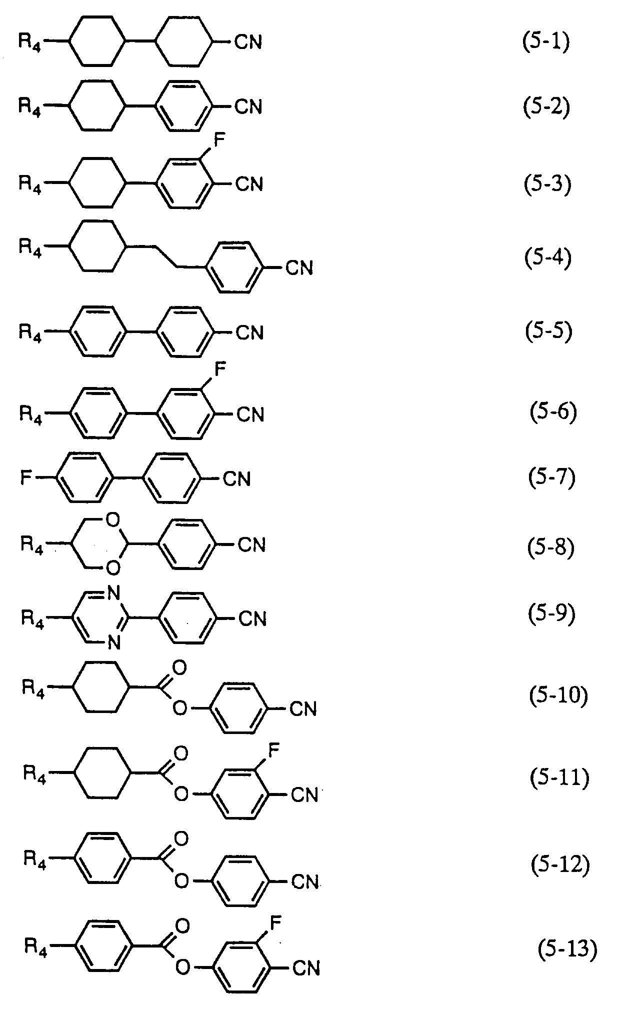 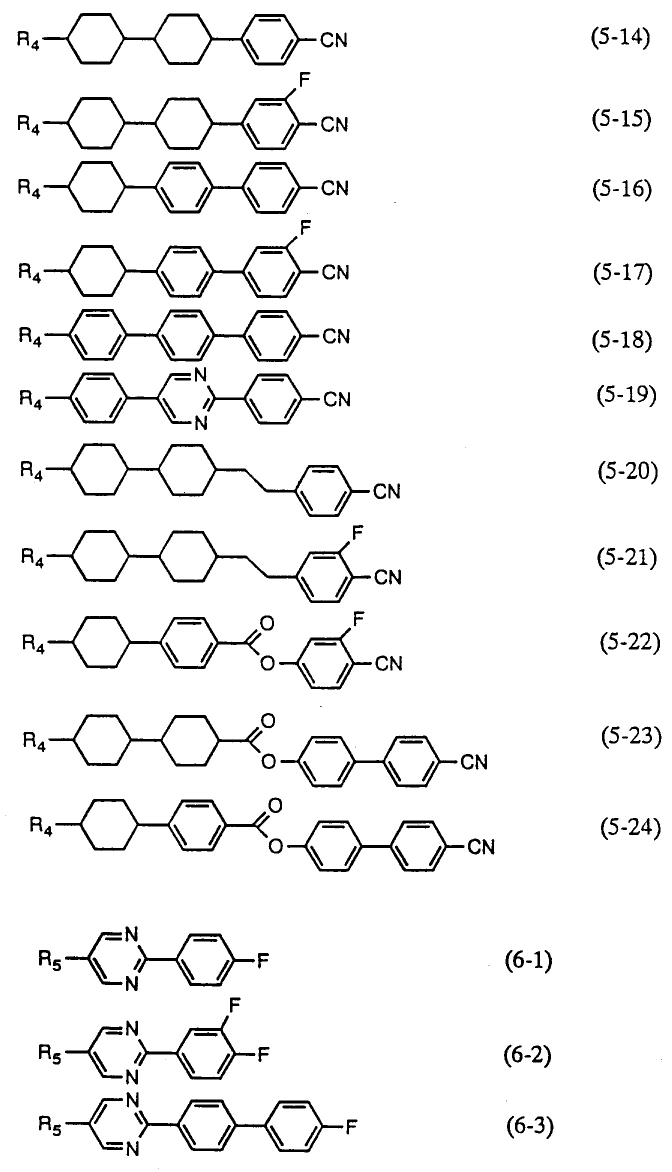 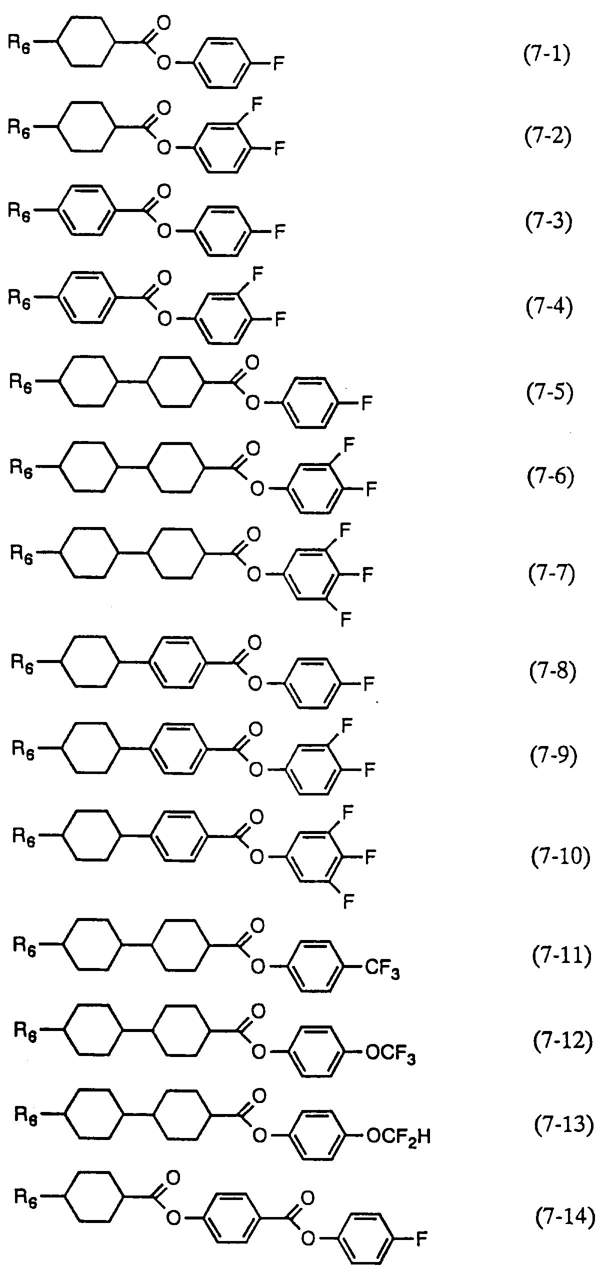 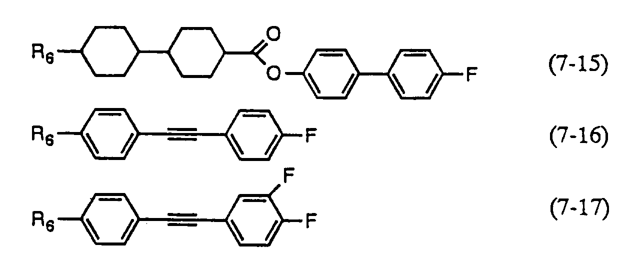 in which, R₄, R₅ and R₆ denote the same meanings as described above.

The compounds expressed by these general formulae (5) to (7) show high positive dielectric anisotropy values and they are used as components of the composition particularly with intentions to decrease a threshold voltage value. Also, they are used with intentions to control a viscosity, to control a refractive index anisotropy value and to enlarge a liquid crystal temperature range, as well as to improve a sharpness.

Also, as preferable examples of the compounds included in the general formulae (8) and (9) of the above-mentioned second B components, there may be listed (8-1) to (8-8) and (9-1) to (9-13), respectively. 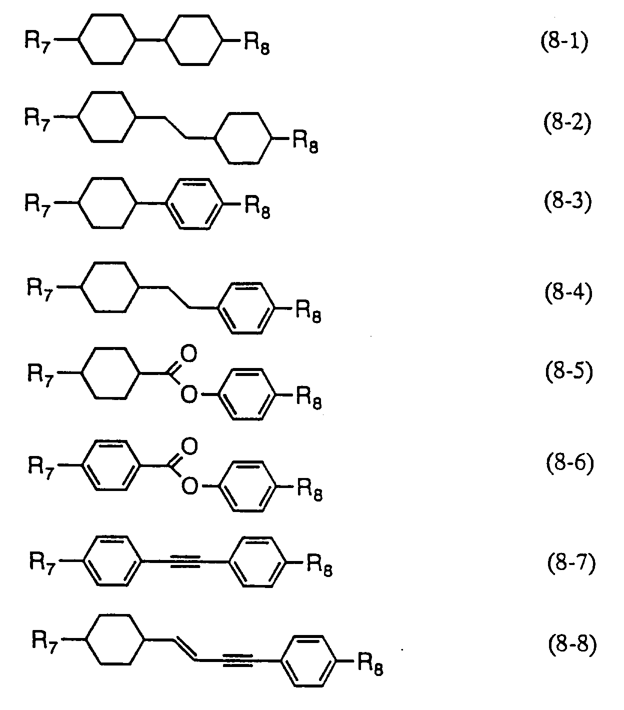 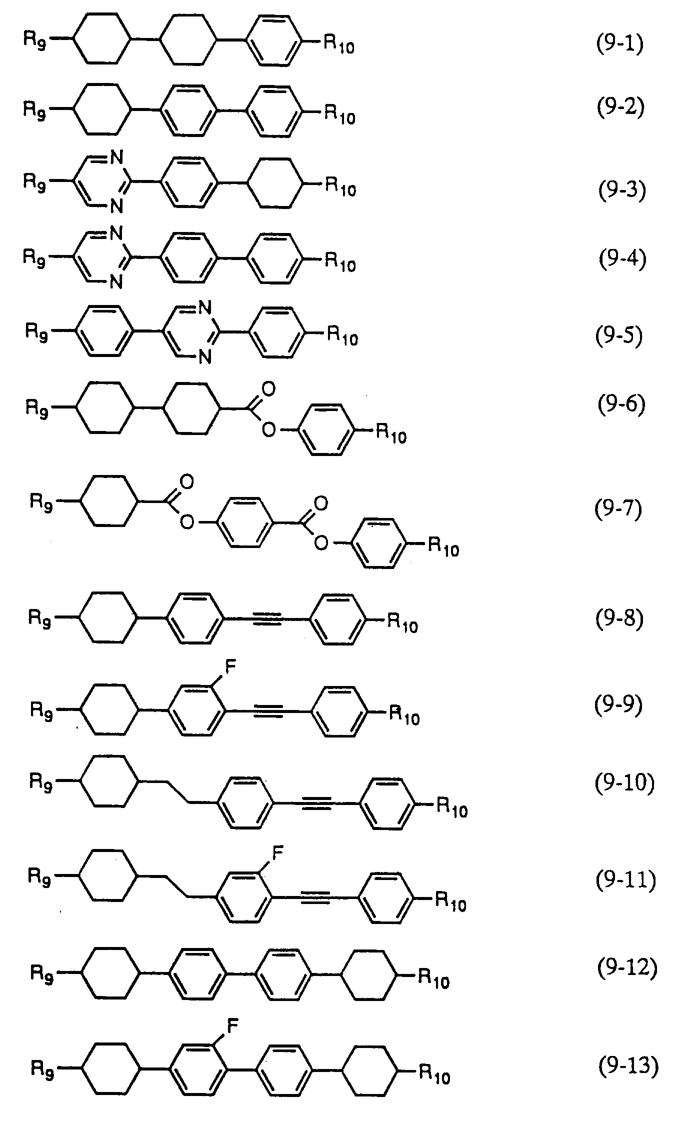 in which, R₇ and R₈ denote the same meanings as described above.

The compounds expressed by the general formulae (8) and (9) are the compounds having negative or slightly positive dielectric anisotropy values. The compound of the general formula (8) is used mainly with intentions to decrease a viscosity and/or to control a refractive index anisotropy value. Also, the compound of the general formula (9) is used with intentions to enlarge a nematic range such as an elevation of a transparent point and/or to control a refractive index anisotropy value.

The compound of the general formulae (5) to (9) are indispensable compounds, particularly in the case that liquid crystal compositions for STN display methods and conventional TN display methods being prepared.

The amount of the compound of the general formulae (5) to (9) used is optional one within a range of 1 to 99% by weight, preferably 10 to 97% by weight, and more preferably 40 to 95% by weight in the case that the liquid crystal compositions for conventional TN display methods and STN display methods being prepared. Additionally, the compounds of (2) to (4) may be used partly.

By using the liquid crystal composition according to the invention, a sharpness and a visible angle can be improved. Also, since the compound of the formula (1) is a low viscous compound, a response speed of a liquid display element with use of the compound is improved to be very high.

The liquid crystal composition used according to the invention is prepared by the customary method. Generally, there may be carried out a method for dissolving various components each other at a high temperature. However, after dissolving and mixing them into an organic solvent in which the liquid crystal being dissolved, the solvent may be removed off under a decreased pressure.

Also, the liquid crystal material according to the invention may be improved and optimized by means of appropriate additives in response to the aimed application. Such additives are known to one skilled in the art and described in detail in literatures etc.

For example, in order to use as a liquid crystal composition for a guest-host (G-H) mode, there may be used dichroic dyes such as a merocyan type, a styryl type, an azo type, an azomethine type, an azoxy type, a quinophthalone type, an anthraquinone type and a tetrazine type. Alternatively, they are used as liquid crystal compositions for polymer dispersion type liquid crystal display elements (PDLCD) represented by NCAP prepared by microcapsulating a nematic liquid crystal and a polymer network liquid crystal display element (PNLCD) with formed three-dimensional networks high molecules in the liquid crystal. Furthermore, they can be used as liquid crystal compositions for a refractive index control (ECB) mode or a dynamic scattering (DS) mode.

The compound according to the invention can be easily prepared by using general chemical methods for organic syntheses. It can be easily synthesized by combining suitably for example the method described in Organic Synthesis, Organic Reactions, Experimental Chemical Text etc.

Although the compound according to the invention can be easily synthesized by general chemical methods for organic syntheses, it can be prepared without any problem according to the representative examples shown as follows. 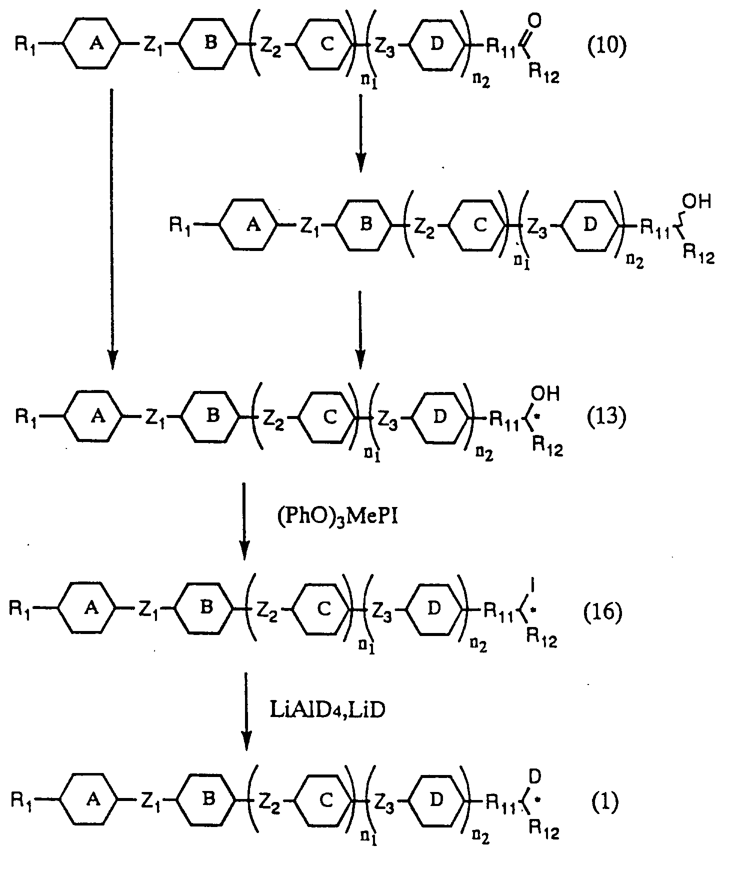 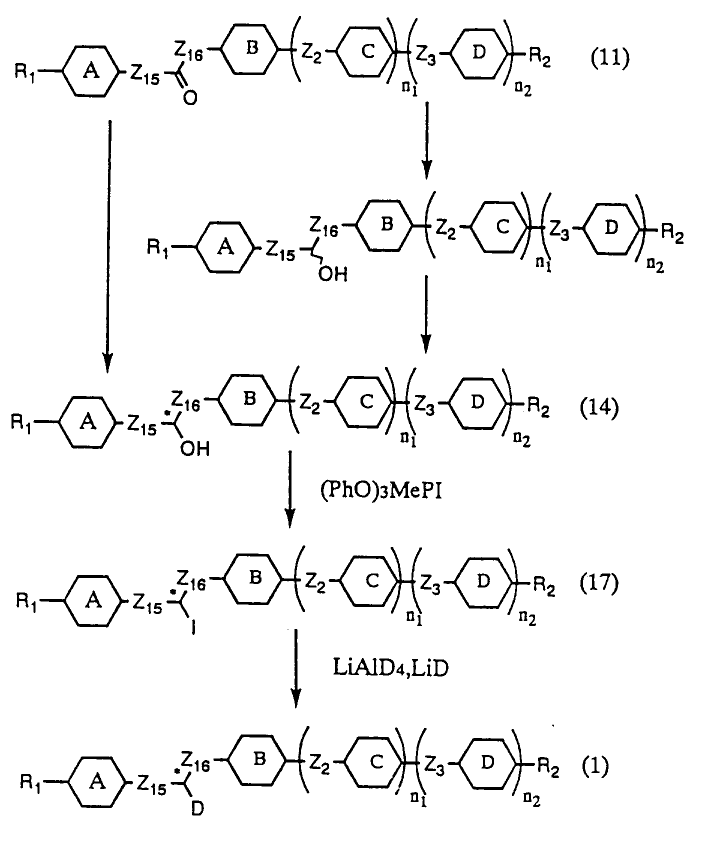 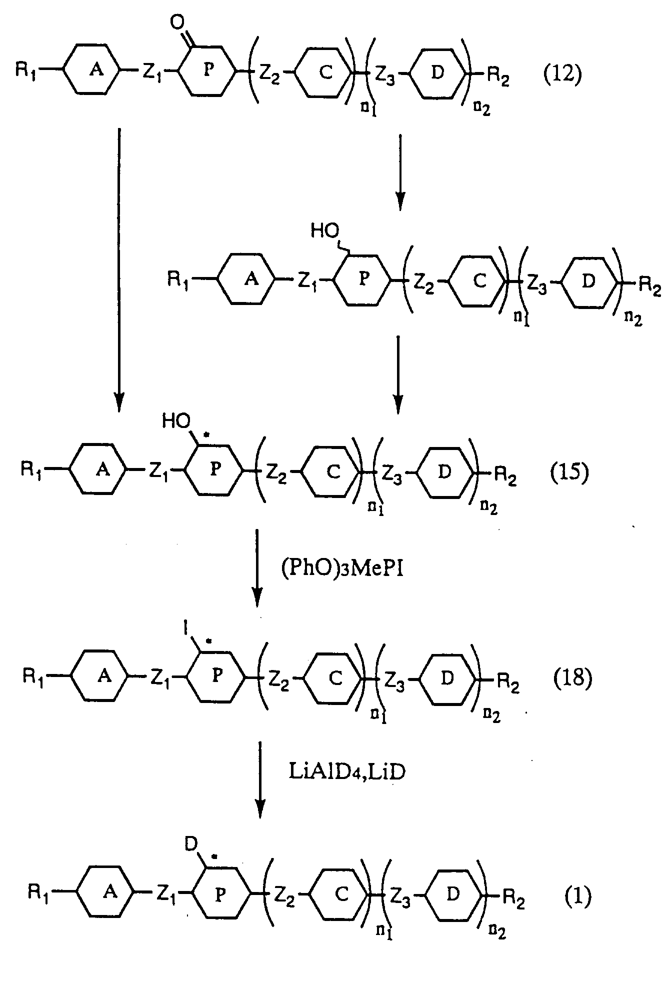 in which, each of R₁, R₂ and R₁₂ independently denotes a hydrogen atom, a cyano group, a halogen atom, an alkyl group and a halogenated alkyl group with 1 to 20 carbon atoms, R₁₁ denotes an alkylene group, wherein one or more than one methylene group in those groups may be substituted with an oxygen, a sulfur atom, CH=CH, C≡C, CO, CF=CF, CF₂, or a cycloalkane ring or a cycloalkene ring with 3 to 5 carbon atoms, but two or more than two methylene groups cannot be continuously substituted by oxygen atoms or sulfur atoms, each of Z₁, Z₂, Z₃, Z₁₅ and Z₁₆ independently denotes a covalent bond or an alkylene group with 1 to 4 carbon atoms, wherein one or more than one methylene group in the said groups may be substituted by an oxygen atom, a sulfur atom, CH=CH, C≡C, CO, CF=CF, CF₂, or a cycloalkane ring or a cycloalkene ring with 3 to 5 carbon atoms, but two or more than two methylene groups cannot be continuously substituted by oxygen atoms or sulfur atoms,
n₁ and n₂ are 0 or 1,
each of rings A, B, C and D independently denotes a benzene ring, a bicyclo[1.1.1]pentane ring, a bicyclo[2.1.1]hexane ring, a bicyclo[2.2.1]heptane ring, a bicyclo[2.2.2]octane ring, a naphthalene ring, a 1,2,3,4-tetrahydronaphthalene ring, a perhydronaphthalene ring, a fluorene ring, a phenanthrene ring, a 9,10-dihydrophenanthrene ring, an indane ring, an indene ring or a cycloalkane ring or cycloalkene ring with 4 to 20 carbon atoms, ring P denotes a bicyclo[1.1.1]pentane ring, a bicyclo[2.1.1]hexane ring, a bicyclo[2.2.1]heptane ring, a bicyclo[2.2.2]octane ring, a 1,2,3,4-tetrahydronaphthalene ring, a perhydronaphthalene ring, a fluorene ring, a 9,10-dihydrophenanthrene ring, an indane ring, an indene ring or a cycloalkane ring or cycloalkene ring with 4 to 20 carbon atoms, wherein the carbon atom(s) in the said rings may be substituted by (an) oxygen atom(s), (a) sulfur atom(s) or (a) nitrogen atom(s), one or more than one hydrogen atom in R₁, R₂, Z₁, Z₂, Z₃, rings A, B, C or D is substituted by a deuterium atom, and the carbon atom bonded to the deuterium atom is an asymmetric carbon atom and optically active, and the hydrogen(s) in the structure may be substituted by (a) halogen atom(s) or (a) cyano group(s).

That is, a ketone compound (10) obtained according to a method of Toku-Gan-Hei 6-6629, a derivative obtained by methods of P. H. Balkwill and et al (EP-117631A), and Y. Goto and et al (Toku-Gan-Hei 2-125489) or a cycloalkane substance (12) obtained according to a method of W. Sucrow and et al. (Chem. Ber., 119, 387 (1986)) is subjected to a stereoselective reduction method by means of various ways reported in H. B. Kagan and et al. (Comprehensive Organometallic Chemistry, vol. 8, Pergamon, 463 (1982)), Noe and et al. (Kagaku, 43, 146 (1989)), S. R. Landor and et al. (J. Chem. Soc., C, 1822 (1966)), A. I. Meyers and et al (J. Am. Chem. Soc., 104, 879 (1982), D. Valentine and et al (Synthesis, 329, (1978)), W. von E Doering and et al (J. Am. Chem. Soc., 72, 631 (1950)), M. M. Midland and et al (Asymmetric Synethesis, 2A, 45 (1983), Ota and et al. (Yuki Gosei Kagaku Kyokaishi, 41, 1018 (1983)), to obtain (13) to (15).

Alternatively, (13) to (15) can be obtained by reduction of (10) to (12) with use of a reductant represented by aluminium lithium hydride and boron sodium hydride followed by an optical resolution by means of a biochemical method with use of various enzymes such as those described in D. Bianchi and et al. (J. Org. Chem., 53, 5531 (1988)), C. J. Sih and et al. (Tetrahedron Lett., 29, 4927 (1988)) and C-S. Chen and et al. (Tetrahedron Lett., 30, 7165 (1989)) or a recrystallization method such as a method of Nohira and et al. (Nihon Kagakukaishi, 1381, (1984)).

Furthermore, after converting a hydroxyl group of (13) to (15) into a leaving group such as an iodine atom, a bromine atom, a methanesulphonyl group, a benzenesulphonyl group or a 4-substituted benzenesulphonyloxy group, lithium aluminium hydride (deuterium) and lithium hydride (deuterium) can be used for deriving to the compound of the formula (1).

Furthermore, in the above-mentioned preparation methods, both enantiomers of the formula (1) can be obtained by selecting a catalyst in the stereoselective reduction or by selecting a kind of enzyme in the biochemical method. Furthermore, both enantiomers of the formula (1) can be obtained by hydrolyzing the antipodes remained in the previous optical resolution reaction for the optical resolution.

### Examples

The preparation methods and use methods according to the invention are illustrated in more detail by means of examples as follows. Herein, C denotes (a) crystal(s), N denotes (a) chiral nematic phase(s), S denotes (a) smectic phase(s) and I denotes (an) isotropic liquid(s) in these examples. In the cases of different smectic phases contained, they are differentiated by S₁ and S₂. Phase transition temperatures are shown on °C.

### Example 1

### Synthesis of (+)-trans-4-(trans-4-n-propylcyclohexyl) -1-(3-D-pentyl)cyclohexane (Compound No.17) (in the general formula (1), R₁ denotes a propyl group, R₂ denotes a 3-D-pentyl group, n₁ and n₂ both denote 0, rings A and B both denote a trans-1,4-cyclohexylene group, and Z₁ denotes a covalent bond)

### (Step 1)

To 9.11g (0.24 mol) of lithium aluminium hydride, 150ml of tetrahydrofuran (hereinafter, referred to THF) was added dropwise at below 0°C with stirring. To it, a solution of 35.1g (0.12 mol) of trans-4-(trans-4-n-propylcyclohexyl)-1-( 3-oxopentyl)cyclohexane in 150ml of THF was added dropwise at below 0°C. After the dropwise addition, the solution was stirred for 2 hours and 150ml of ethyl acetate was added dropwise. Furthermore, 200ml of 6N hydrochloric acid was added dropwise.

Deposited precipatations were filtered off, and the reaction solution was extracted with diethyl ether (500ml X 3). The extracted organic layer was washed subsequently with an aqueous saturated sodium hydrogencarbonate solution (200ml X 3) and a saturated brine (200ml X 3), thereafter dried with anhydrous magnesium sulfate, and concentrated, to obtain 33.2g (0.11 mol) of trans-4-(trans-4-n-propylcyclohexyl)-1-( 3-hydroxypentyl)cyclohexane in racemic form.

### (Step 2)

33.3g (0.11 mol) of trans-4-(trans-4-n-propylcyclohexyl)-1-(3-hydroxypentyl)cyclohexane in racemic form was dissolved in 300ml of toluene, and 9.70g (0.11 mol) of vinyl acetate and 6.6g of Lipase PS made by Amano Seiyaku Sha were introduced and stirred for 21 hours at the room temperature.

The reaction mixture was concentrated and thereafter purified with a silica gel column chromatography (eluting solvent; toluene : vinyl acetate = 5:1), to obtain 16.4g (0.056 mol) of (+)-trans-4-(trans-4-n-propylcyclohexyl)-1-(3 -hydroxypentyl)cyclohexane.

The specific rotation of the compound was
[α] D28+5.33° (c 1.08, CHCℓ₃).

### (Step 3)

5.89g (0.020 mol) of (+)-trans-4-(trans-4-n-propylcyclohe xyl)-1-(3-hydroxypentyl)cyclohexane was dissolved in 50ml of dimethylformamide (hereinafter, referred to DMF). 18.1g (0.04 mol) of methyltriphenoxyphosphine iodide was added and heated to 50°C with stirring for 3 hours, and thereafter 60ml of methanol was added dropwise. To the reaction solution, 300ml of chloroform was added, then washed subsequently with an aqueous saturated sodium thiosulphate solution (100ml X 3) and a saturated brine (100ml X 3), dried with anhydrous magnesium sulphate and concentrated, to obtain 1.0g (0.0025 mol) of (-)-trans-4-(trans-4-n-propylcyclohexyl)-1-(3-iodopentyl)cyclohexane.

The specific rotation of the compound was
[α] D28-2.00° (c 0.40, CHCℓ₃).

### (Step 4)

To 0.1g (0.0024 mol) of lithium aluminium hydride (deuterium) and 0.09g (0.0099 mol) of lithium hydride (deuterium), 4ml of THF was added at below 0°C with ice cooling. To this, 1.0g (0.0025 mol) of (-)-trans-4-(trans-4-n-propylcyclohexyl)-1-(3-iodopentyl)cyclohexane which had been dissolved in 2ml of THF was added dropwise at below 0°C. After stirring at 0°C for 2 hours, 1ml of ethyl acetate was added dropwise. Furthermore, 10ml of 6N hydrochloric acid was added dropwise. Deposited precipitations were filtered off, and the reaction solution was extracted with 150ml of diethyl ether.

The extracted organic layer was washed subsequently with an aqueous sodium hydrogencarbonate solution (20ml X 3) and a saturated brine (20ml X 3), dried with magnesium sulphate and concentrated, to obtain 0.58g of crude (+)-trans-4-(tran s-4-n-propylcyclohexyl)-1-(3-D-pentyl)cyclohexane. It was further purified with a silica gel column chromatography (eluting solvent; n-heptane), to obtain 0.53g of (+)-trans-4 -(trans-4-n-propylcyclohexyl)-1-(3-D-pentyl)cyclohexane.

The specific rotation of the compound was
[α] D28+0.19° (c 1.02, CHCl3).

The compound showed liquid crystal properties, and S-I point thereof was 96.2 to 97.1°C. Also, various spectral data coincided well with the structure.
MS: 279 (M+)

### Example 2

### Synthesis of (+)-trans-4-(trans-4-(3-pentenyl)cyclohexyl) -1-(1-D-propyl)cyclohexane (Compound No.10) (in the general formula (1), R₁ denotes a 3-pentenyl group, R₂ denotes a 1-D-propyl group, n1 and n₂ both denote 0, rings A and B both denote a trans-1,4-cyclohexylene group, and Z₁ denotes a covalent bond)

### (Step 1)

367.8g (1.2 mol) of ethyl trans-4-(trans-4-(3-pentenyl) cyclohexyl)cyclohexane carboxylate obtained according to Toku-Kai-Hei 1-157925 was dissolved in 5l of toluene and then cooled to -55°C, and 1.32l (1.32 mol) of 1M solution of diisobutyl aluminium hydride in toluene was added dropwise. After the dropwise addition, the solution was stirred at the same temperature for 10 minutes and then warmed gradually to the room temperature.

Furthermore, 500ml of an aqueous saturated ammonium chloride solution was added dropwise with ice cooling. 1l of 10% sulphuric acid was added, and deposited precipitations were filtered off with Celite. The organic layer was washed with a saturated brine (500ml X 3). It was dried with anhydrous magnesium sulfate and concentrated, to obtain 258.4g (1.04 mol) of trans-4-(trans-4-(3-pentenyl)cyclohexyl )cyclohexane carboaldehyde.

### (Step 2)

1.1l (1.1 mol) of 1M THF solution of ethyl magnesium bromide was added dropwise at 40°C to 258.4g (1.04 mol) of trans-4-(trans-4-(3-pentenyl)cyclohexyl)cyclohexane carboaldehyde which had been dissolved in 1l of THF. After the dropwise addition, it was stirred at the same temperature for 1 hour. It was cooled with ice, and 1l of 6N hydrochloric acid was added. The reaction mixture was extracted with toluene (1l X 3), and the obtained toluene phase was washed with a saturated brine (500ml X 3).

It was dried with anhydrous magnesium sulphate and concentrated, to obtain 217.1g (0.78 mol) of trans-4-(trans-4-(3-pentenyl)cyclohexyl)-1-(1-hydroxypropyl) cyclohexane in racemic form.

### (Step 3)

217.1g (0.78 mol) of trans-4-(trans-4-(3-pentenyl)cyclohe xyl)-1-(1-hydroxypropyl)cyclohexane in racemic form was dissolved in 1l of toluene, and 67.2g (0.78 mol) of ethyl acetate and 40g of Lipase PS were introduced and stirred for 26 hours. The reaction mixture was concentrated and thereafter purified with a silica gel column chromatography (eluting solvent; toluene : ethyl acetate = 5:1), to obtain 111.3g (0.40 mol) of (+)-trans-4-(trans-4-(3-pentenyl)cycloh exyl)-1-(1-hydroxypropyl)cyclohexane.

The specific rotation of the compound was
[α] D24+5.24° (c 1.02, CHCl3).

### (Step 4)

111.3g (0.40 mol) of (+)-trans-4-(trans-4-(3-pentenyl)cyc lohexyl)-1-(1-hydroxypropyl)cyclohexane was dissolved in 1l of toluene. Furthermore, 79.0g (1.0 mol) of pyridine was added, and 59.5g (0.52 mol) of methane sulphonic chloride (mesyl chloride) was added dropwise at the room temperature. After stirring for 3 hours, 1l of toluene was added, and the reaction mixture was washed with a saturated brine (300ml X 3), dried with anhydrous magnesium sulphate and concentrated, to obtain 133.4g (0.39 mol) of optically active trans-4-(trans-4-(3-pentenyl)cyclohexyl)-1-(1-mesyloxypropyl)cyclohexane.

### (Step 5)

To 0.42g (0.01 mol) of lithium aluminium hydride (deuterium) and 0.27g (0.03 mol) of lithium hydride (deuterium), 10ml of THF was added at below 0°C with ice cooling. To this, 3.4g (0.01 mol) of trans-4-(trans-4-(3-pen tenyl)cyclohexyl)-1-(1-mesyloxypropyl)cyclohexane which had been dissolved in 4ml of toluene was added dropwise at below 0°C. After stirring at 0°C for 2 hours, 1ml of ethyl acetate was added dropwise. Furthermore, 10ml of 6N hydrochloric acid was added dropwise.

Then, deposited precipitations were filtered off with Celite, and the reaction solution was extracted with 150ml of diethyl ether. The extracted organic layer was washed subsequently with an aqueous sodium hydrogencarbonate solution (20ml X 3) and a saturated brine (20ml X 3), dried with anhydrous magnesium sulphate and concentrated, to obtain 2.5g of crude (+)-trans-4-(trans-4-(3-pentenyl)cycloh exyl)-1-(1-D-propyl)cyclohexane. It was further purified with a silica gel column chromatography (eluting solvent; n-heptane), to obtain 2.06g (0.0078 mol) of (+)-trans-4-(tra ns-4-(3-pentenyl)cyclohexyl)-1-(1-D-propyl)cyclohexane.

The specific rotation of the compound was
[α] D25+0.22° (c 1.05, CHCl3).

The compound showed liquid crystal properties, and C-S point, S-N point and N-I point thereof were 44.1°C, 72.3°C and 72.3°C, respectively. Also, various spectral data coincided wall with the structure.
MS: 277 (M+)

### Example 3

### Synthesis of (+)-trans-4-(4-cyanophenyl)-1-(1-D-pentenyl) cyclohexane (Compound No.33) (in the general formula (1), R₁ denotes a 1-D-3-pentenyl group, R₂ denotes a cyano group, n₁ and n₂ both denote 0, ring A denotes trans-1,4-cyclohexylene, ring B denotes a 1,4-phenylene, and Z₁ denotes a covalent bond)

### (Step 1)

Into a reactor, 25.54g (1.05 mol) of magnesium was introduced, 30ml of THF was added with stirring, and a solution of 108.7g (1.2 mol) of 1-bromo-2-butene dissolved in 200ml of THF was added dropwise at below 50°C. After the dropwise addition, 300ml of THF was added, and then 213.3g (1.0 mol) of trans-4-(4-cyanophenyl)cyclohexane carboaldehyde dissolved in 200ml of THF was added dropwise at 40°C. After the dropwise addition, it was stirred at the same temperature. The reaction solution was cooled with ice, and 1l of 6N hydrochloric acid was added dropwise.

The reaction solution was extracted with toluene (1l X 3), and the obtained toluene phase was washed with a saturated brine (500ml X 3). It was dried with anhydrous magnesium sulfate, concentrated and furthermore purified with a silica gel column chromatography (eluting solvent; n-heptane : ethyl acetate = 5:1), to obtain 174.4g (0.65 mol) of trans-4-(4-cyanophenyl)-1-(1-hydroxy-3-pentenyl)cycl ohexane in racemic form.

### (Step 2)

174.4g (0.65 mol) of trans-4-(4-cyanophenyl)-1-(1-hydroxy -3-pentenyl)cyclohexane in racemic form was dissolved in 1l of toluene, and 56.0g (0.65 mol) of vinyl acetate and 35g of Lipase PS were introduced and stirred for 78 hours. The reaction mixture was concentrated and thereafter purified with a silica gel column chromatography (eluting solvent; toluene : ethyl acetate = 5:1), to obtain 90.7g (0.34 mol) of (+)-trans-4-(4-cyanophenyl)-1-(1-hydroxy-3-pentenyl)cyclo hexane.

The specific rotation of the compound was
[α] D25+4.38° (c 1.04, CHCl3).

### (Step 3)

13.4g (0.05 mol) of (+)-trans-4-(4-cyanophenyl)-1-(1-hydr oxy-3-pentenyl)cyclohexane was dissolved in 100ml of toluene. Furthermore, 7.9g (0.1 mol) of pyridine was added and 6.0g (0.53 mol) of methane sulphonic acid chloride (mesyl chloride) was added dropwise. After stirring for 3 hours, 100ml of toluene was added, then the reaction mixture was washed with a saturated brine (100ml X 3), dried with anhydrous magnesium sulphate and concentrated, to obtain 14.7g (0.042 mol) of optically active trans-4-(4-cyanophenyl)-1-(1-methoxy-3-pentenyl) cyclohexane.

### (Step 4)

To 0.42g (0.01 mol) of lithium aluminium hydride (deuterium) and 0.27g (0.03 mol) of lithium hydride (deuterium), 10ml of THF was added at below 0°C with ice cooling. 3.5g (0.01 mol) of (+)-trans-4-(4-cyanophenyl)-1-(1 -methoxy-3-pentenyl)cyclohexane which had been dissolved in 4ml of THF was added dropwise to it at below 0°C.

After stirring at 0°C for 2 hours, 1ml of ethyl acetate was added dropwise. Furthermore, 10ml of 6N hydrochloric acid was added dropwise. Deposited precipitations were filtered off with Celite, and the reaction mixture was extracted with 150ml of diethyl ether. The extracted organic layer was washed subsequently with an aqueous sodium hydrogencarbonate solution (20ml X 3) and a saturated brine (20ml X 3). It was dried with anhydrous magnesium sulphate and concentrated, to obtain 2.34g of crude (+)-trans-4-(4-cy anophenyl)-1-(1-D-3-pentenyl)cyclohexane. It was further purified with a silica gel column chromatography (eluting solvent; n-heptane), to obtain 2.0g (0.0078 mol).

The specific rotation of the compound was
[α] D28+0.18° (c 1.04, CHCl3). The compound showed liquid crystal properties, and C-N point and N-I point thereof were 59.8 and 73.5°C, respectively.

Also, various spectral data coincided well with the structure.
MS: 254 (M+)

### Example 4

### Synthesis of (+)-4-(trans-4-(trans-4-(2-D-propyl)cyclohex yl)cyclohexyl)-3,5-difluoro-4-trifluoromethylbenzene (Compound No.130) (in the general formula (1), R₁ denotes a 2-D-propyl group, R₂ denotes a trifluoromethyl group, n₁ denotes 1, n₂ denotes 0, rings A and B both denote a trans-1,4-cyclohexylene group, ring C denotes a 3,5-difluoro-1,4-phenylen, and Z₁ and Z₂ denote a covalent bond)

### (Step 1)

124.6g (0.556 mol) of ethyl diethylphosphonoacetate, 1l of THF and 62.3g (0.556 mol) of t-butoxy potassium were introduced and stirred for 40 minutes. To it, 117g (0.447 mol) of trans-4-(1,4-dioxy-spiro[4,5]dec-8-yl)cyclohexanone which had been dissolved in 400ml of THF was added dropwise at below 10°C. It was stirred for 20 hours at the room temperature. Thereafter, 1300ml of toluene was added, and the organic layer was washed with water and a saturated brine (300ml X 2, respectively). After it was dried with anhydrous magnesium sulfate, it was concentrated, to obtain 218.2g of an Horner-Wittig adduct.

### (Step 2)

218.2g of the adduct obtained in the above-mentioned step was dissolved in 400ml of Solmix A-11, 25g of Raney nickel catalyst was added, and hydrogenation was carried out for 3 hours at a hydrogen pressure of 1Kg. After filtering off the catalyst, the reaction mixture was purified with a silica gel column chromatography (eluting solvent; toluene), to obtain 144g (0.464 mol) of trans-4-(1,4-dioxy-spiro[4,5]dec-8-yl)-(1-methoxycarbonylethyl)cyclohexane.

### (Step 3)

144g (0.464 mol) of trans-4-(1,4-dioxy-spiro[4,5]dec-8-yl )-(1-methoxycarbonylethyl)cyclohexane was dissolved in 2l of added toluene. A reactor was cooled to -55°C, and 670ml (0.67 mol) of 1M diisobutyl aluminium hydride solution in toluene was added dropwise. After the dropwise addition, the reaction mixture was stirred at the same temperature for 10 minutes. Thereafter, the reactor was heated gradually to the room temperature.

Furthermore, 300ml of an aqueous saturated ammonium chloride solution was added dropwise with ice cooling. 700ml of 10% sulphuric acid was added, and deposited precipitations were filtered off with Celite. The mixed liquid was separated and the organic layer was washed with a saturated brine (300ml X 3). It was dried with anhydrous magnesium sulphate and thereafter concentrated, to obtain 112.3g (0.418 mol) of trans-4-(1,4-dioxy-spiro[4,5]dec-8-yl)cyclohe xane carboaldehyde.

### (Step 4)

460ml (0.460 mol) of 1M methyl magnesium bromide solution in THF was added dropwise to 112.3g (0.418 mol) of trans-4-(1,4-dioxy-spiro[4,5]dec-8-yl)cyclohexane carboaldehyde which had been dissolved in 500ml of THF. After the dropwise addition, the reaction mixture was stirred at the room temperature for 1 hour. Thereafter, the reactor was cooled with ice, and 700ml of 6N hydrochloric acid was added. The reaction mixture was extracted with toluene (500ml X 3), and the obtained toluene layer was washed with a saturated brine (300ml X 3).

It was dried with anhydrous magnesium sulphate and thereafter concentrated, to obtain 78.8g (0.293 mol) of trans-4-(1,4-dioxy-spiro[4,5]dec-8-yl)-1-(2-hydroxypropyl)cy clohexane in racemic form.

### (Step 5)

78.8g (0.293 mol) of trans-4-(1,4-dioxy-spiro[4,5]dec-8-y l)-1-(2-hydroxypropyl)cyclohexane in racemic form was dissolved in 500ml of toluene, and 25.2g (0.293 mol) and 15g of Lipase PS were introduced and stirred for 24 hours. The reaction mixture was concentrated and thereafter purified with a silica gel column chromatography (eluting solvent; toluene : ethyl acetate = 5:1), to obtain 40.93g (0.152 mol) of (+)-trans-4-(1,4-dioxy-spiro[4,5]dec-8-yl)-1-(2-hydroxypr opyl)cyclohexane.

The specific rotation of the compound was
[α] D28+5.59° (c 1.04, CHCl3).

### (Step 6)

40.93g (0.152 mol) of (+)-trans-4-(1,4-dioxy-spiro[4,5]de c-8-yl)-1-(2-hydroxypropyl)cyclohexane was dissolved in 500ml of DMF. 161.7g (0.4 mol) of methyltriphenoxyphosphine iodide was added and heated to 50°C with stirring for 3 hours. Furthermore, 500ml of methanol was added dropwise.

Thereafter, 500ml of chloroform was added and the reaction mixture was washed with an aqueous saturated sodium thiosulphate solution (300ml X 3) and a saturated brine (300ml X 3). It was dried with anhydrous magnesium sulphate and concentrated, to obtain 30.34g (0.08 mol) of optically active trans-4-(1,4-dioxy-spiro[4,5]dec-8-yl)-1-(2-iodopropyl)cyclohexane.

### (Step 7)

To 3.36g (0.08 mol) of lithium aluminium hydride (deuterium) and 2.15g (0.24 mol) of lithium hydride (deuterium), 15ml of THF was added with ice cooling at below 0°C. To it, 30.34g (0.08 mol) of optically active trans-4-(1,4-dioxy-spiro[4,5]dec-8-yl)-1-(2-iodopropyl)cyclo hexane which had been dissolved in 30ml of THF was added dropwise at below 0°C.

Furtheremore, 50ml of 6N hydrochloric acid was added dropwise. Deposited precipitations were filtered off with Celite, and the reaction mixture was extracted with 500ml of diethyl ethre. The extracted organic layer was washed with an aqueous saturated sodium hydrogencarbonate solution (100ml X 3) and a saturated brine (20ml X 3). It was dried with magnesium sulphate and concentrated, to obtained 19.25g of crude (+)-trans-4-(1,4-dioxy-spiro[4,5]dec-8-yl)-1-(2-D-p ropyl)cyclohexane. It was furthermore purified with a silica gel column chromatography (eluting solvent; n-heptane), to obtain 18.3l (0.072 mol) of (+)-trans-4-(1,4-dioxy-spiro[4,5 ]dec-8-yl)-1-(2-D-propyl)cyclohexane.

### (Step 8)

A mixture of 18.3l (0.072 mol) of (+)-trans-4-(1,4-dioxy-spiro[4,5]dec-8-yl)-1-(2-D-propyl)cyclohexane and 100ml of formic acid was heated under reflux for 3 hours. After cooling to the room temperature, 500ml of toluene was added, and the reaction mixture was washed subsequently with water (200ml X 2), an aqueous saturated sodium hydrogencarbonate solution (200ml X 2) and a saturated brine (200ml X 2). It was dried with magnesium sulphate and concentrated, to obtain 17.2g of crude (+)-trans-4-(2-D-propyl)cyclohexyl)cyc lohexane.

Thereafter, it was purified with a silica gel column chromatography (eluting solvent; n-heptane : ethyl acetate = 4:1), to obtain 12.87g (0.058 mol) of (+)-4-(trans-4-(2-D-pr opyl)cyclohexyl)cyclohexane.

### (Step 9)

1.78g (0.073 mol) of magnesium was introduced in a reactor, 5ml of THF was added, and 18.12g (0.70 mol) of 3,5-difluoro-4-trifluoromethyl bromide which had been dissolved in 20ml of THF was added dropwise at below 50°C. After the dropwise addition, 20ml of THF was added and 12.87g (0.058 mol) of (+)-4-(trans-4-(2-D-propyl)cyclohexyl) cyclohexane which had been dissolved in 20ml of THF was added dropwise. After the dropwise addition, the reaction mixture was stirred for 1 hour.

The reactor was cooled with ice and 150ml of 6N hydrochloric acid was added. The reaction mixture was extracted with toluene (300ml X 3), and the obtained toluene layer was washed with a saturated brine (200ml X 3). It was dried with anhydrous magnesium sulphate, concentrated and purified with a silica gel column chromatography (eluting solvent; n-heptane), to obtain 15.81g (0.039 mol) of (+)-4-( 4-(trans-4-(2-D-propyl)cyclohexyl)-(1-hydroxy)cyclohexyl)-3, 5-difluoro-4-trifluoromethylbenzene.

### (Step 10)

A mixture of 15.81g (0.039 mol) of (+)-4-(4-(trans-4-(2-D -propyl)cyclohexyl)-(1-hydroxy)cyclohexyl)-3,5-difluoro-4-tr ifluoromethylbenzene, 100ml of toluene and 1g of Umberlist was refluxed for 3 hours. After cooling, Umberlist was filtered off, and the mixture was washed with a saturated brine (50ml X 3). It was dried with anhydrous magnesium sulphate and concentrated, to obtain 14.81g (0.038 mol) of (+)-4-(4-(trans-4-(2-D-propyl)cyclohexyl)cyclohexen-1-yl)-3, 5-difluoro-4-trifluoromethylbenzene.

### (Step 11)

14.81g (0.038 mol) of (+)-4-(4-(trans-4-(2-D-propyl)cyclo hexyl)cyclohexen-1-yl)-3,5-difluoro-4-trifluoromethylbenzene was dissolved in 100ml of ethyl acetate, 0.5g of 5% palladium-on-carbon catalyst was added, and then stirred at 1 atmosphere of hydrogen pressure for 5 hours. After filtering off the catalyst, the reaction mixture was purified with a silica gel column chromatography (eluting solvent; toluene), to obtain 6.10g of (+)-4-(4-(trans-4-(2-D -propyl)cyclohexyl)cyclohexyl)-3,5-difluoro-4-trifluoromethy lbenzene.

Furthermore, by repeating recrystallization with 20ml of n-heptane three times, 5.18g (0.13 mol) of (+)-4-(trans-4-(t rans-4-(2-D-propyl)cyclohexyl)cyclohexyl)-3,5-difluoro-4-tri fluoromethylbenzene was obtained.

The specific rotation of the compound was
[α] D25+0.16° (c 1.02, CHCl3). The compound showed liquid crystal properties, and C-N point and N-I point thereof were 64.1 and 92.5°C, respectively.

Also, various spectral data coincided well with the structure.
MS: 389 (M+)

### Example 5

### Synthesis of (+)-trans-4-(trans-4-(2-D-propyl)cyclohexyl) -1-(3-butenyl)cyclohexane (Compound No.13) (in the general formula (1), R₁ denotes a 2-D-propyl group, R₂ denotes a 3-butenyl group, n₁ and n₂ both denote 0, rings A and B denote trans-1,4-cyclohexylene, and Z₁ denotes a covalent bond)

### (Step 1)

To 55.42g (0.125 mol) of (2-(1,3-dioxan-2-yl)ethyl) triphenyl phosphonium bromide, 200ml of DMF and 14.15g (0.125 mol) of t-butoxy potassium were added and stirred for 40 minutes. To it, 11.17g (0.05 mol) of (+)-4-(trans-4-(2-D-propyl)cyclohexyl)cyclohexane obtained according to the method of Example 4 (till Step 8) which had been dissolved in 200ml of DMF was added dropwise and heated at 50°C for 3 hours with stirring.

300ml of methanol was added dropwise, 600ml of chloroform was added, and the reaction mixture was washed with an aqueous saturated sodium thiosulphate solution (200ml X 3) and a saturated brine (200ml X 3). It was dried with anhydrous magnesium sulphate and concentrated, to obtain 12.3g (0.04 mol) of (+)-4-(trans-4-(2-D-propyl)cyclohexyl)-(1,3-dioxan-2-yl)ethynylidene cyclohexane.

### (Step 2)

12.3g (0.04 mol) of (+)-4-(trans-4-(2-D-propyl)cyclohexyl )-(1,3-dioxan-2-yl)ethynylidene cyclohexane was dissolved in 100ml of ethyl acetate, 0.5g of 5% palladium-on-carbon catalyst, and the reaction mixture was stirred at 1 atmosphere of hydrogen pressure for 3 hours. After filtering off the catalyst, it was purified with a silica gel column chromatography (eluting solvent; toluene), to obtain 14.3g of (+)-4-(trans-4-(2-D-propyl)cyclohexyl)-(2-(1,3-dioxan-2-y l)ethyl)cyclohexane.

Furthermore, by repeating the recrystalization with 20ml of n-heptane three times, 4.09g (0.013 mol) of (+)-trans-4-( trans-4-(2-D-propyl)cyclohexyl)-(2-(1,3-dioxan-2-yl)ethyl)cy clohexane was obtained.

### (Step 3)

4.09g (0.013 mol) of (+)-trans-4-(trans-4-(2-D-propyl)cyc lohexyl)-(2-(1,3-dioxan-2-yl)ethyl)cyclohexane was obtained was dissolved in 50ml of THF, 50ml of 6N hydrochloric acid was added and the reaction mixture was heated under reflux for 2 hours. After cooling to the room temperature, 500ml of toluene was added, and the reaction mixture was washed with water (200ml X 2), an aqueous saturated sodium hydrogencarbonate solution (200ml X 2) and a saturated brine (200ml X 2).

It was dried with anhydrous magnesium sulphate, and concentrated, to obtain 3.11g (0.0117 mol) of crude (+)-trans-4-(trans-4-(2-D-propyl)cyclohexyl)cyclohexylpropyl aldehyde.

### (Step 4)

A mixture of 5.9g (0.0146 mol) of iodomethyl triphenyl phosphine, 50ml of THF and 1.65g (0.0146 mol) of t-butoxy potassium was stirred for 40 minutes. To it, a solution of 3.11g (0.0117 mol) of (+)-trans-4-(trans-4-(2-D-propyl)cyclo hexyl)cyclohexylpropyl aldehyde in 10ml of THF was added dropwise at below 10°C. Furthermore, the reaction mixture was stirred at the room temperature for 20 hours. Thereafter, 300ml of toluene was added and the organic layer was washed with water and a saturated brine (100ml X 2, respectively).

It was dried with anhydrous magnesium sulphate, and concentrated, to obtain 3.11g of (+)-trans-4-(trans-4-(2-D-propyl)cyclohexyl)-1-(3-butenyl)cyclohexane. Furthermore, by repeating recrystallization with 5ml of n-heptane twice, 2.47g (0.0094 mol) of (+)-trans-4-(trans-4-(2-D-propyl)cyclo hexyl)-1-(3-butenyl)cyclohexane was obtained.

The specific rotation of the compound was
[α] D30+0.25° (c 1.12, CHCl3).

The compound showed liquid crystal properties, and C-N point and N-I point thereof were 31.1°C and 47.6°C, respectively.

Also, various spectral data coincided well with the structure.
MS: 264 (M+)

### Example 6

Compound numbers 1 to 194 were synthesized according to the methods of Examples 1 to 5. Herein, Compound numbers 1 to 5 were also listed.

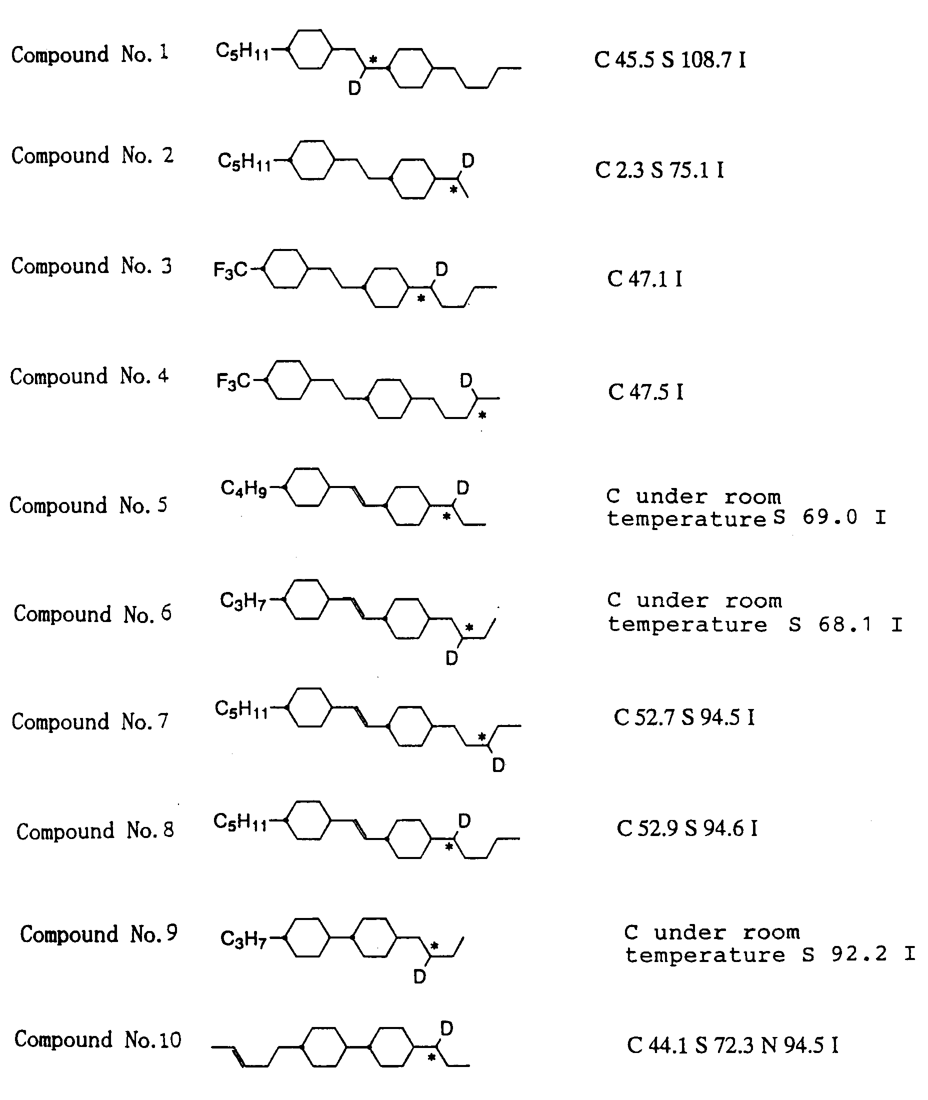

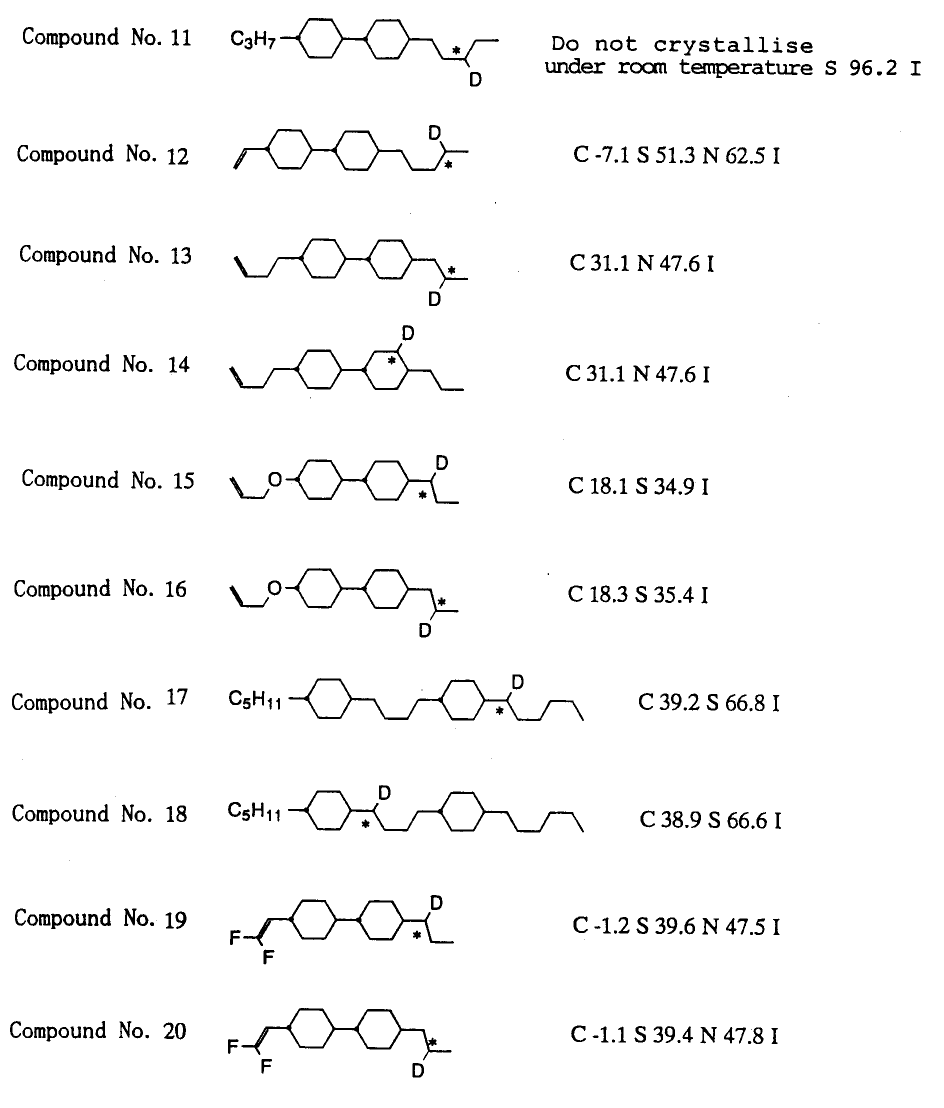

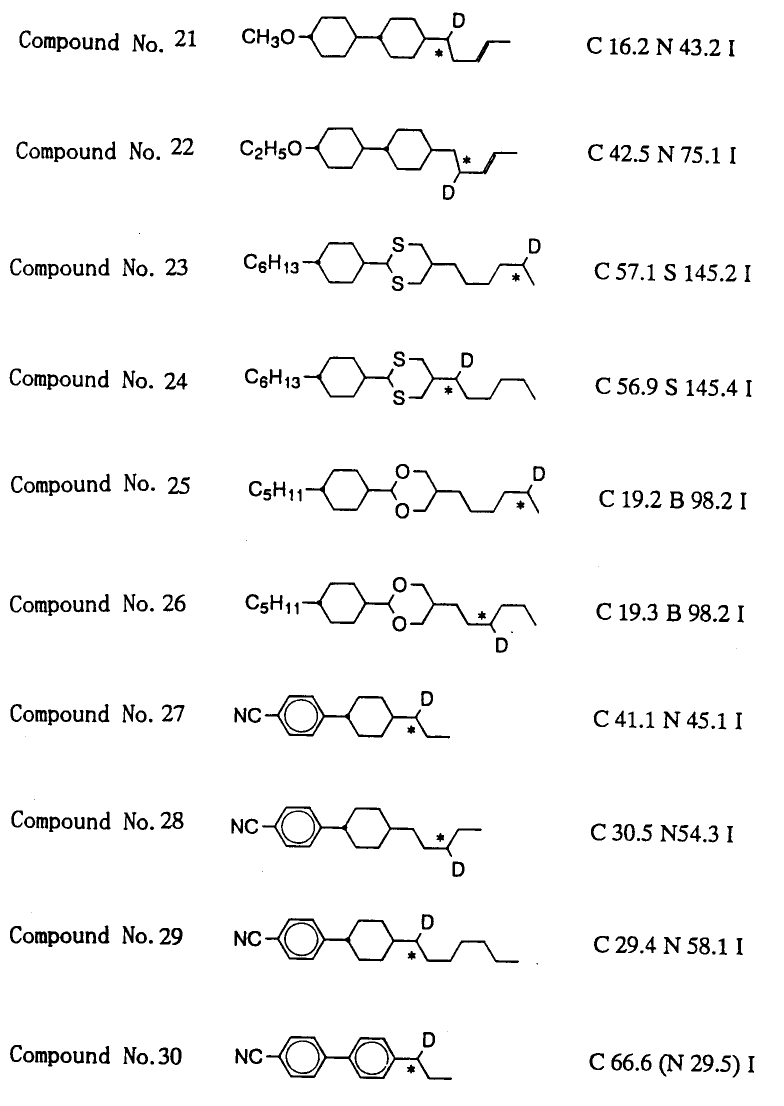

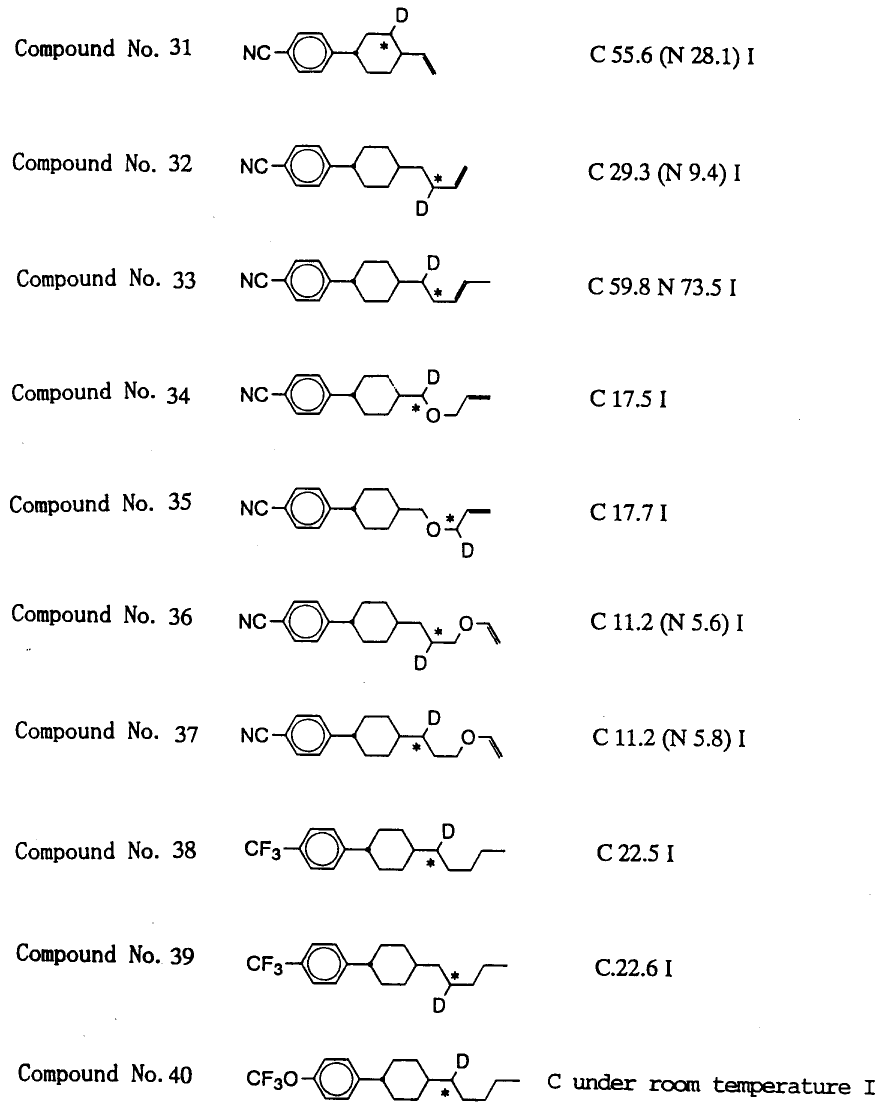

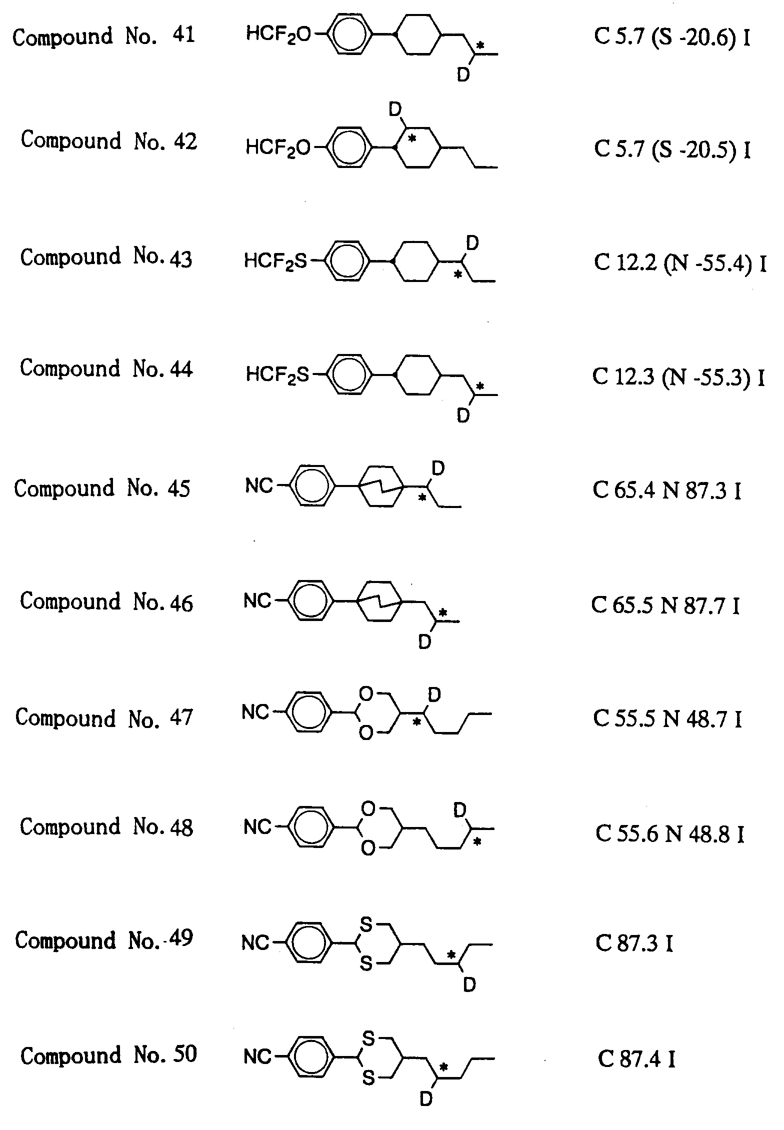

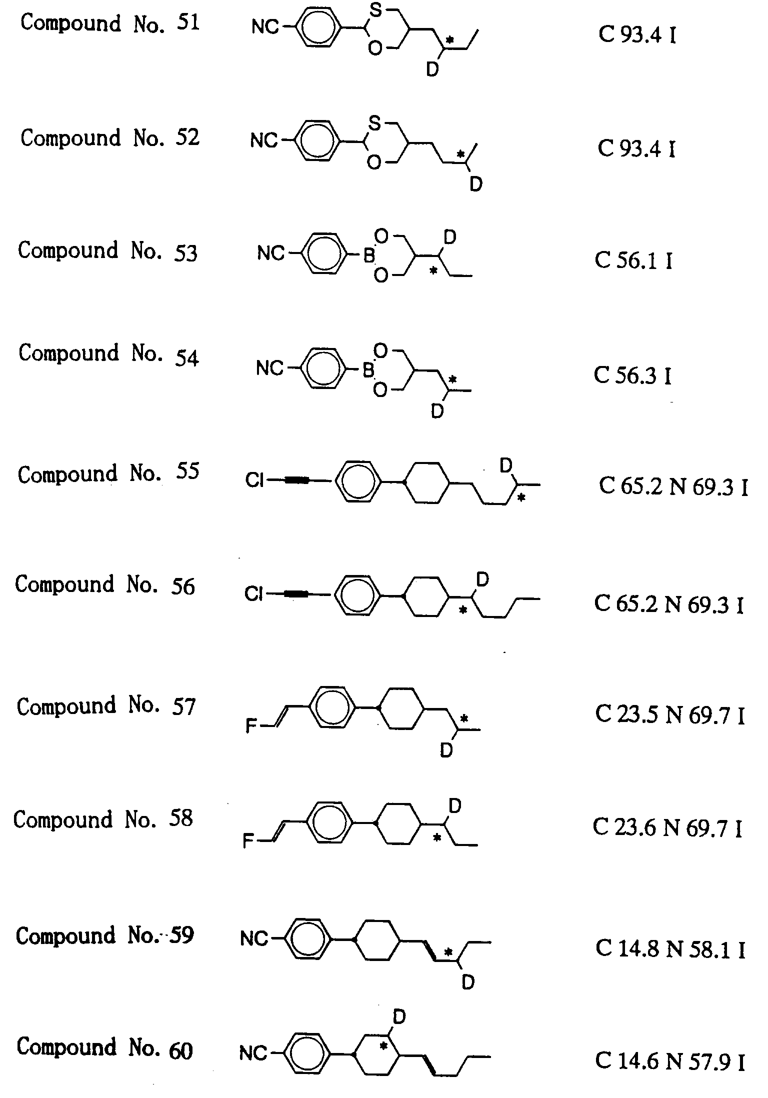

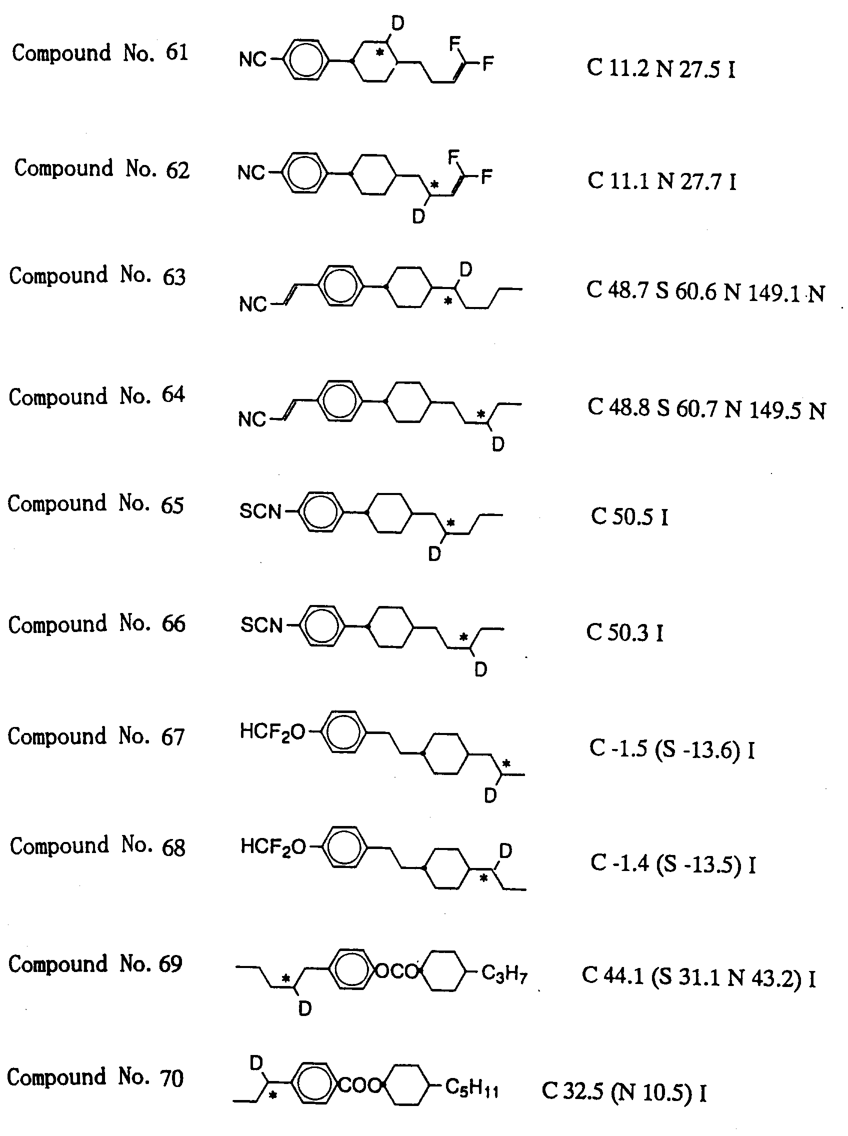

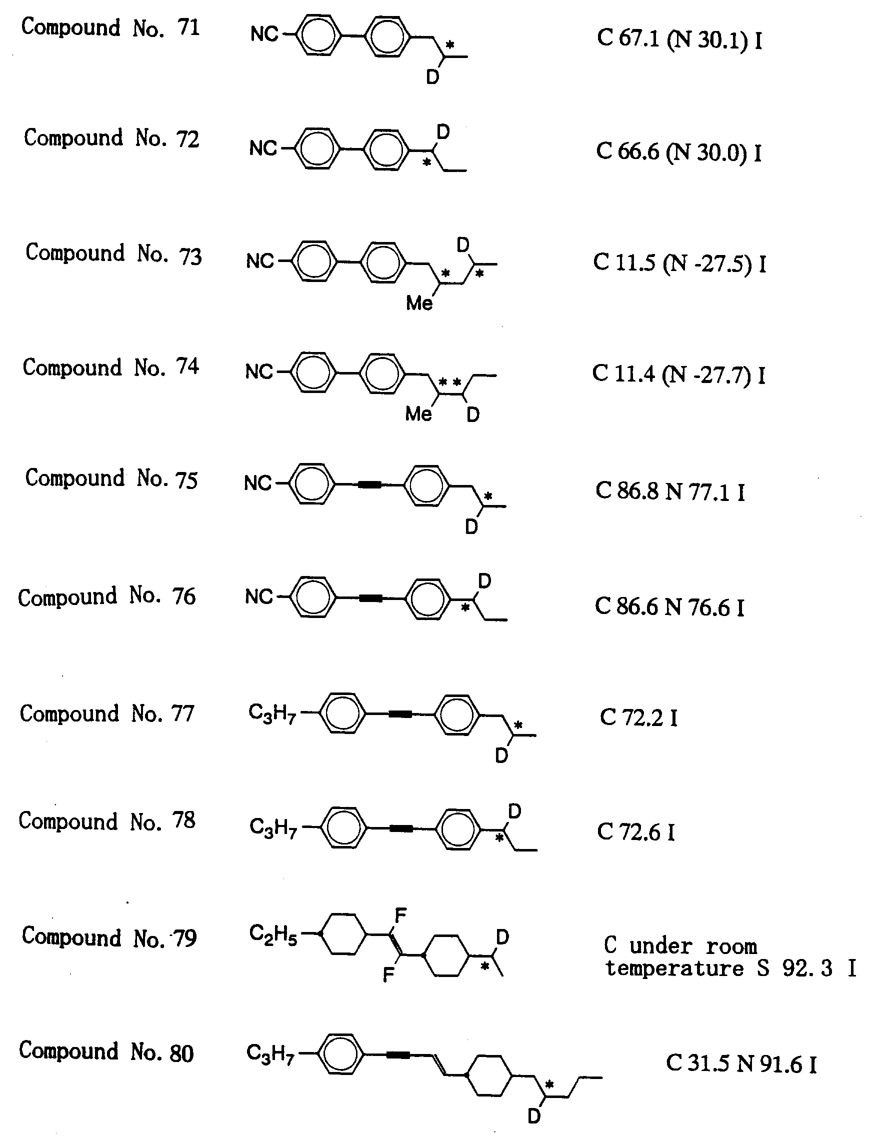

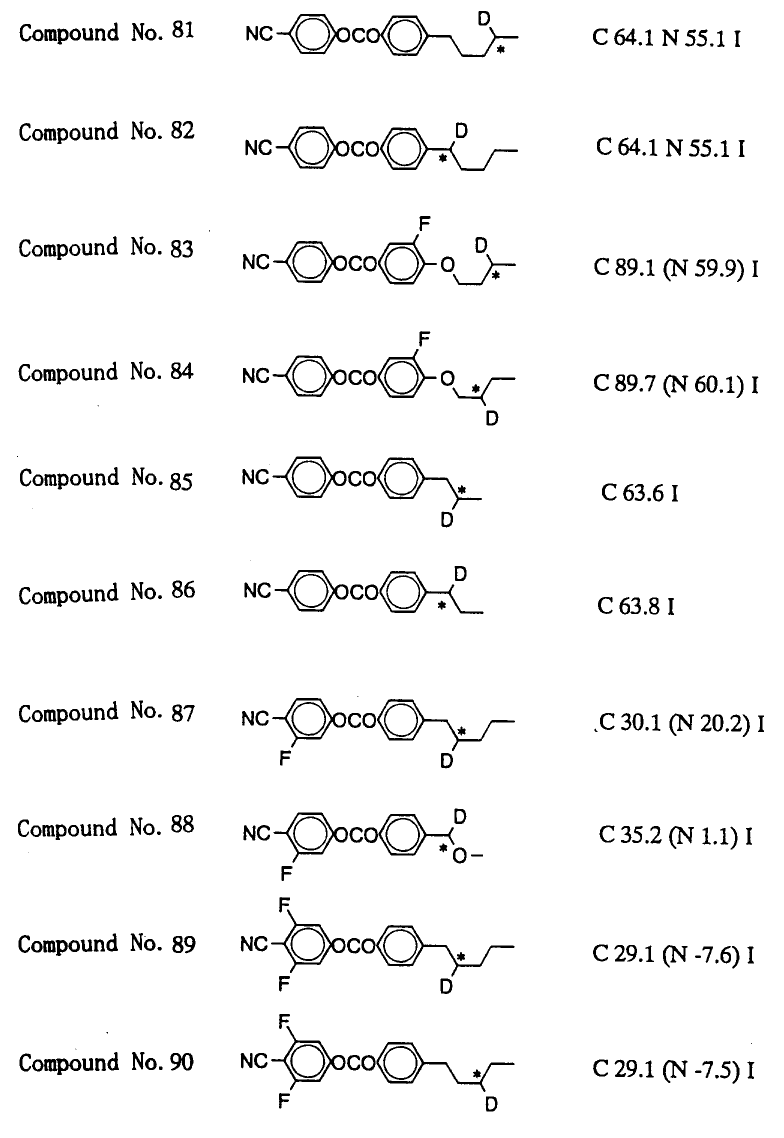

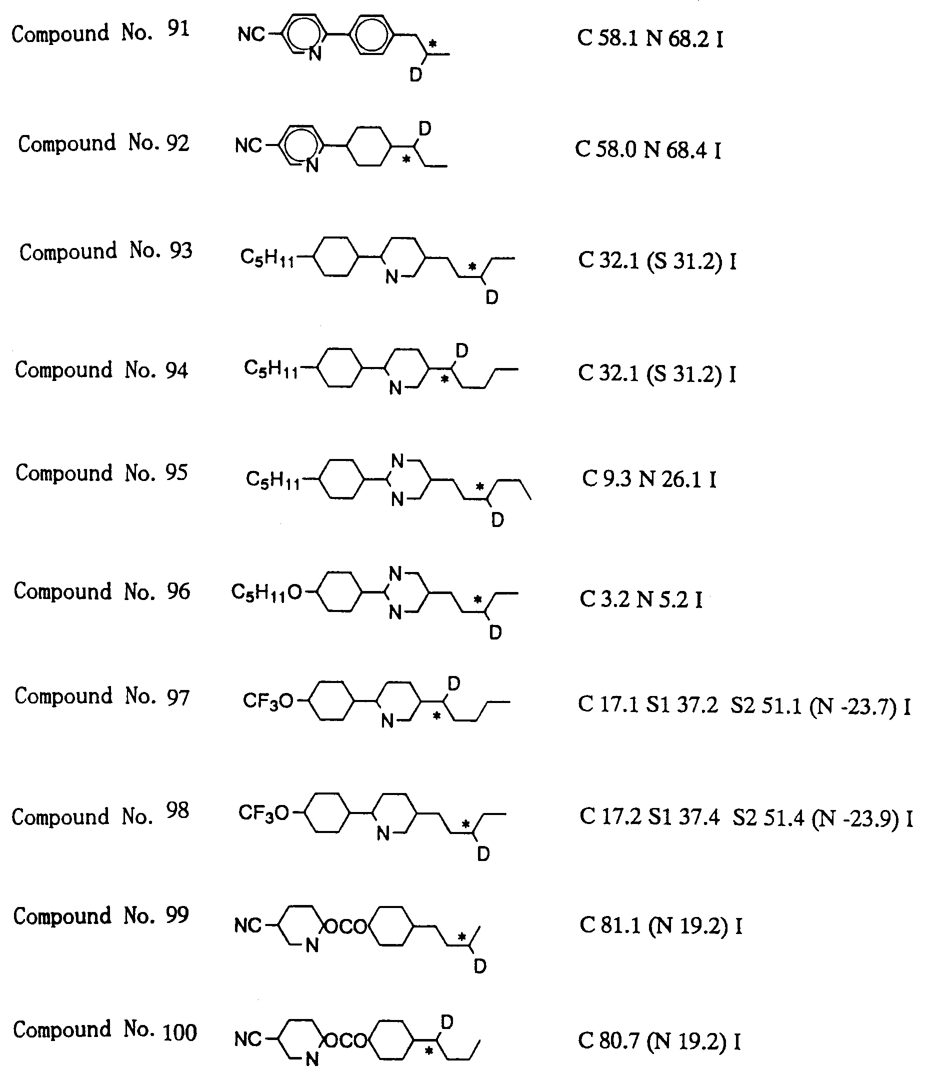

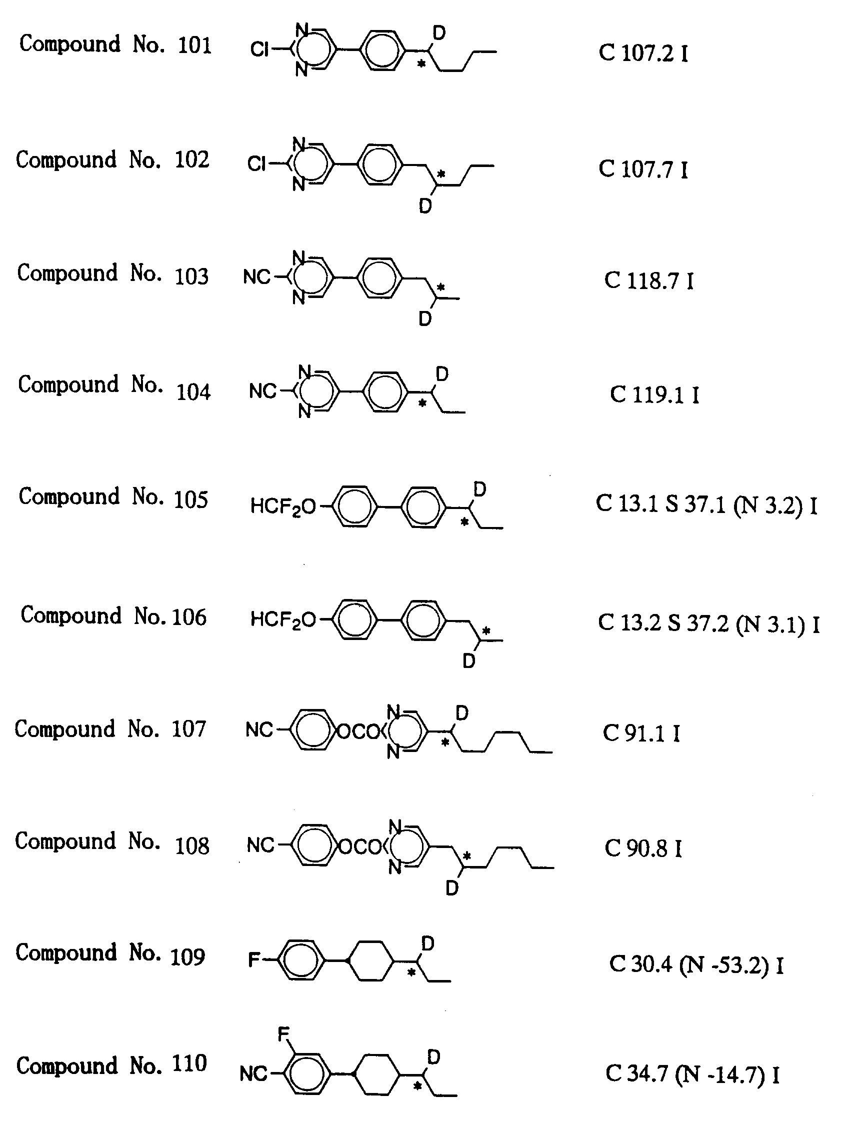

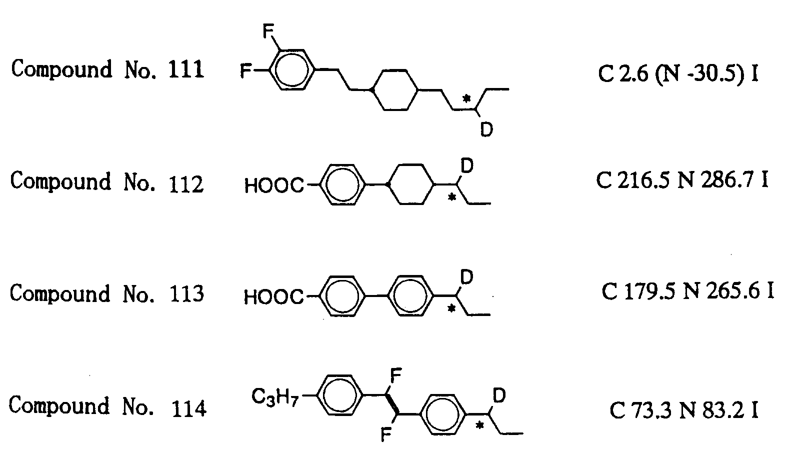

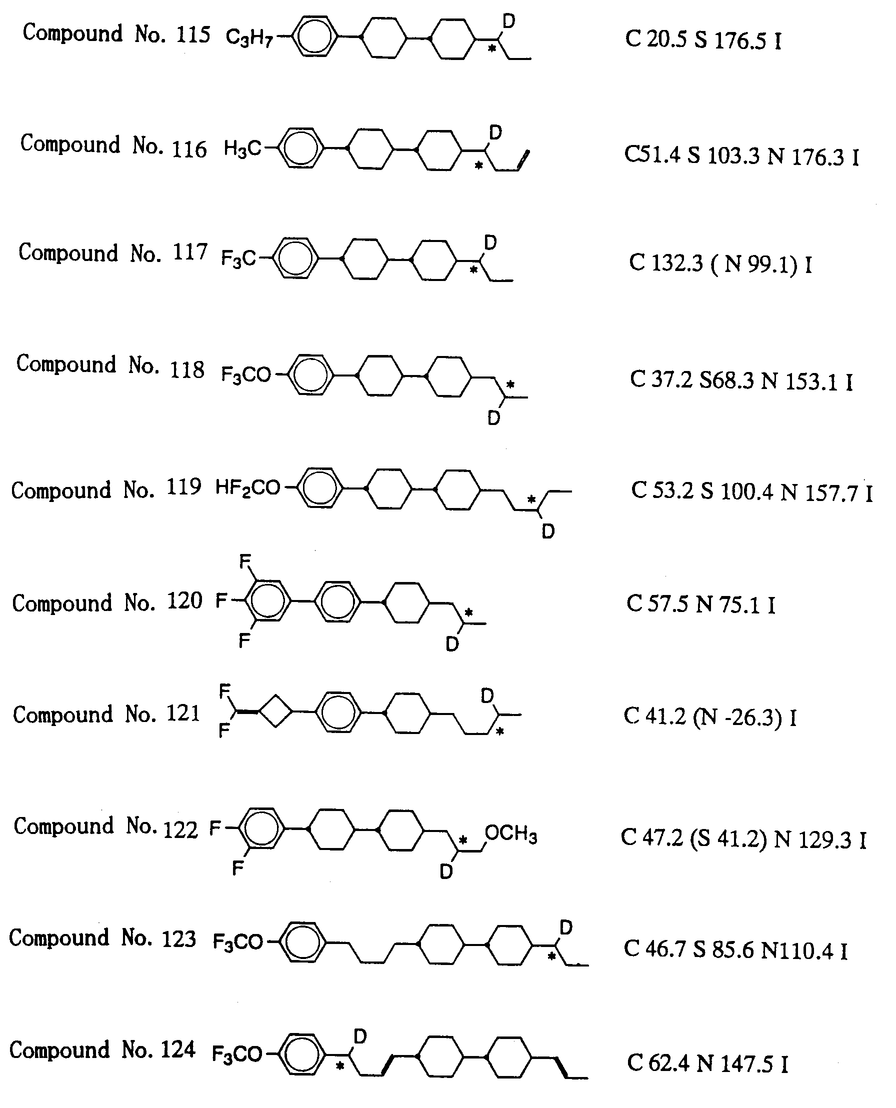

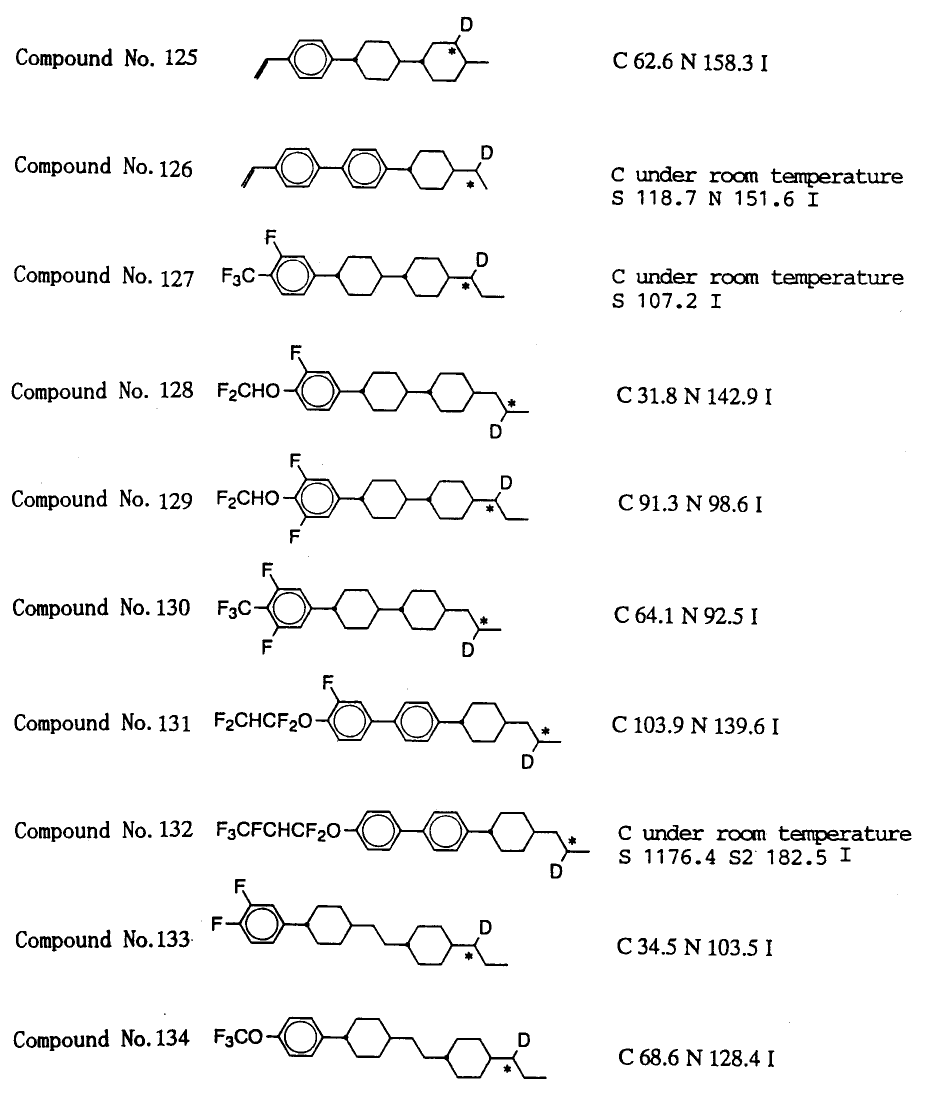

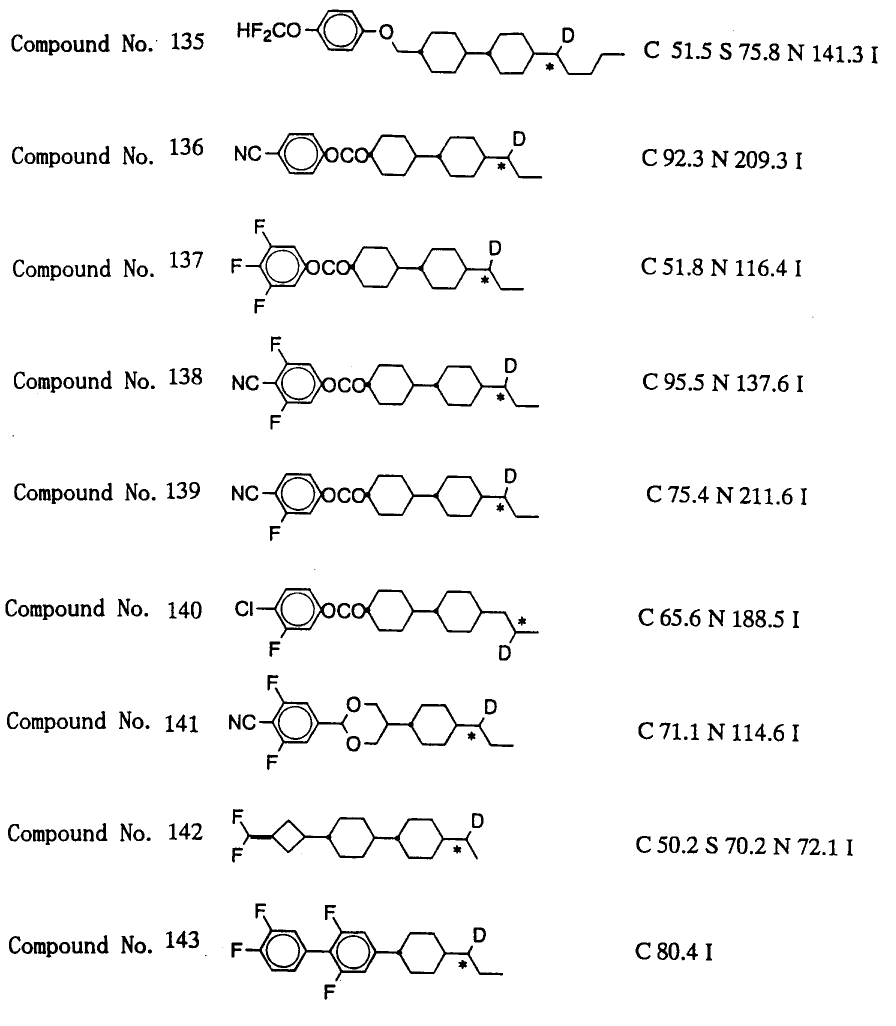

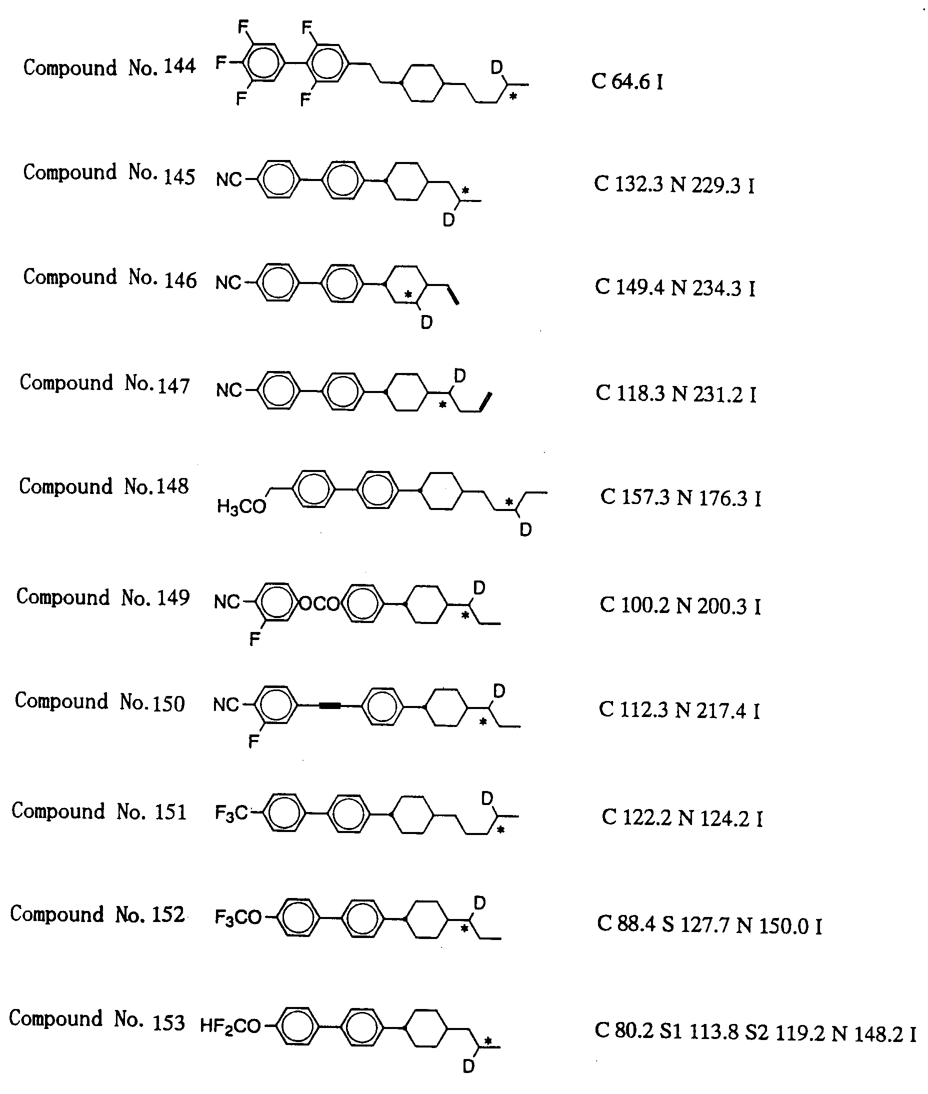

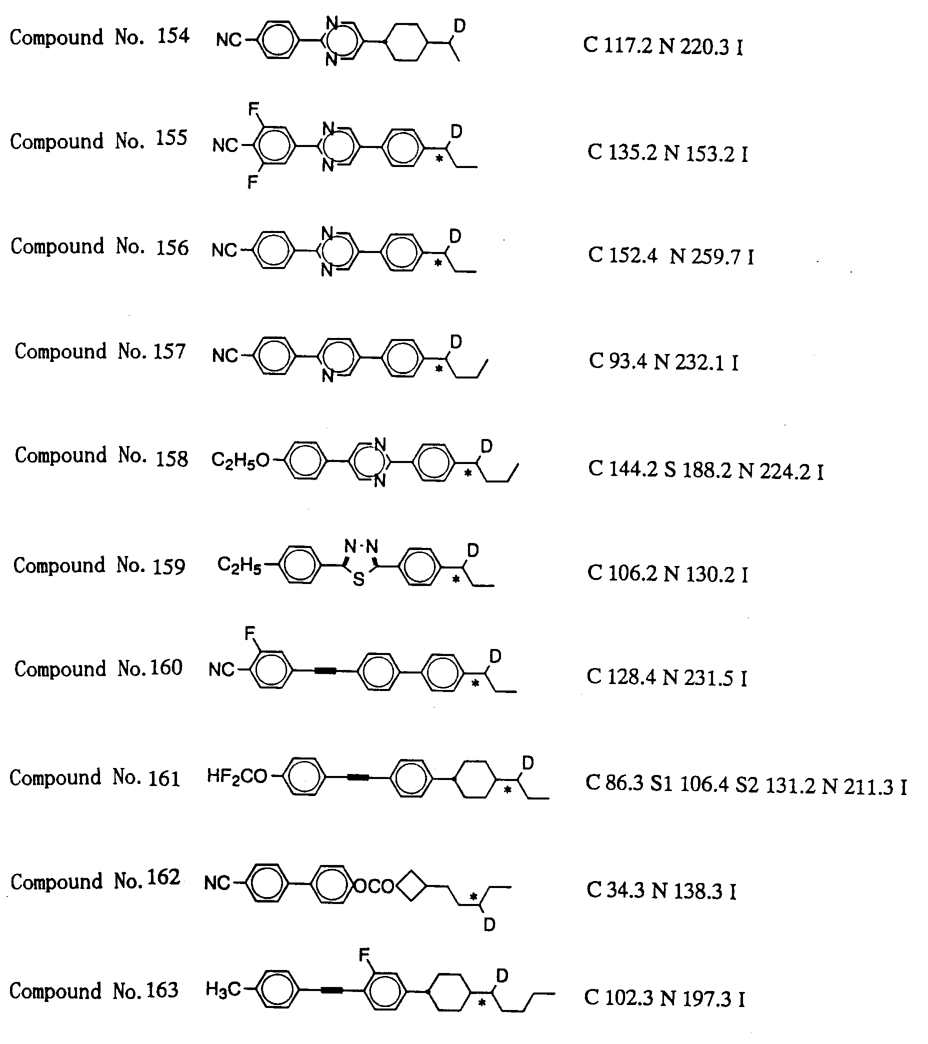

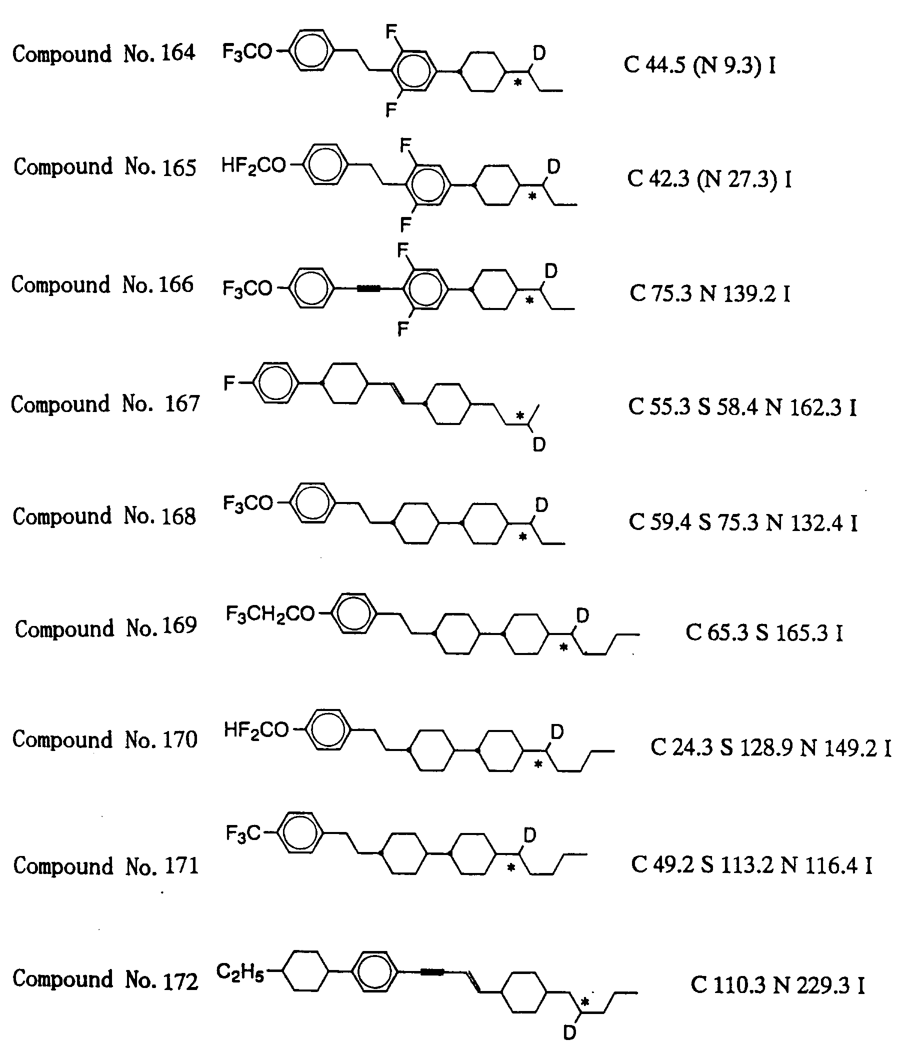

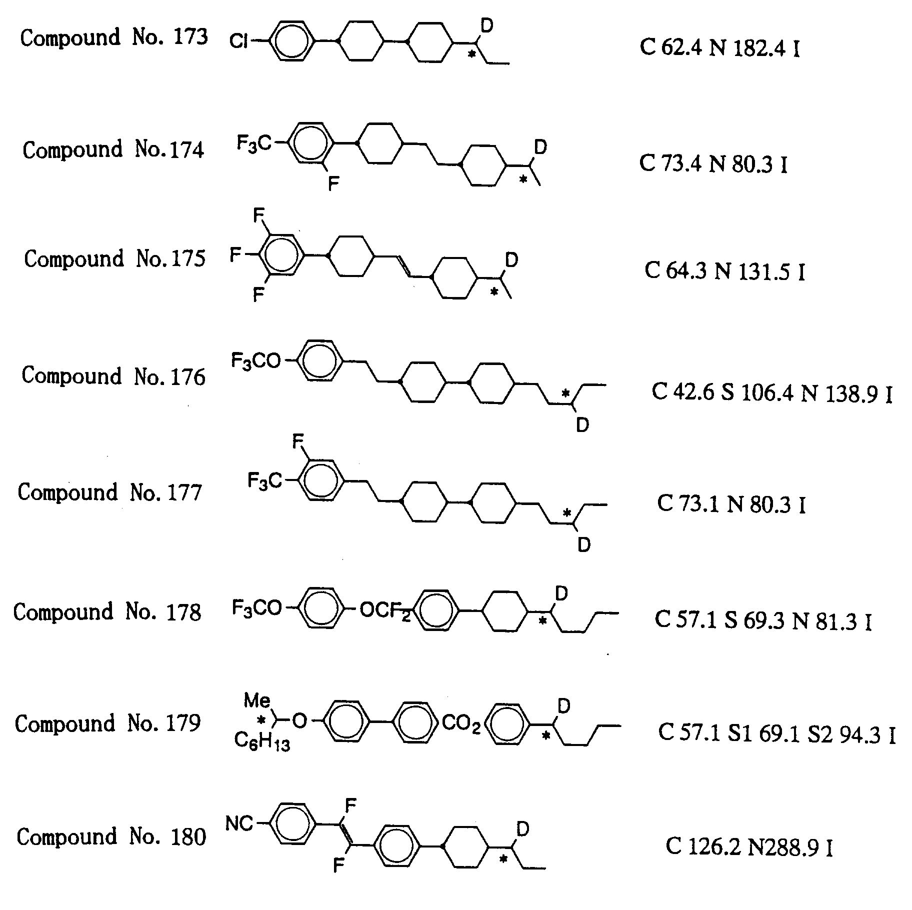

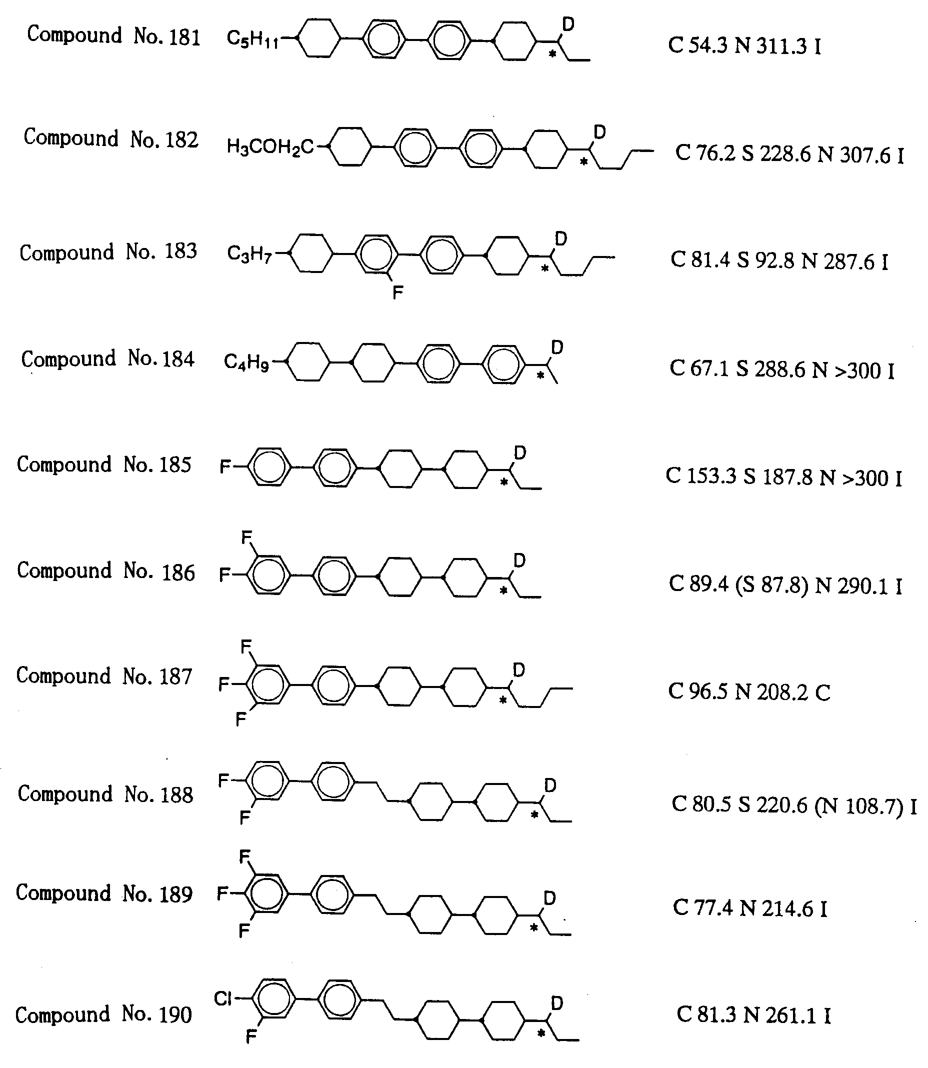

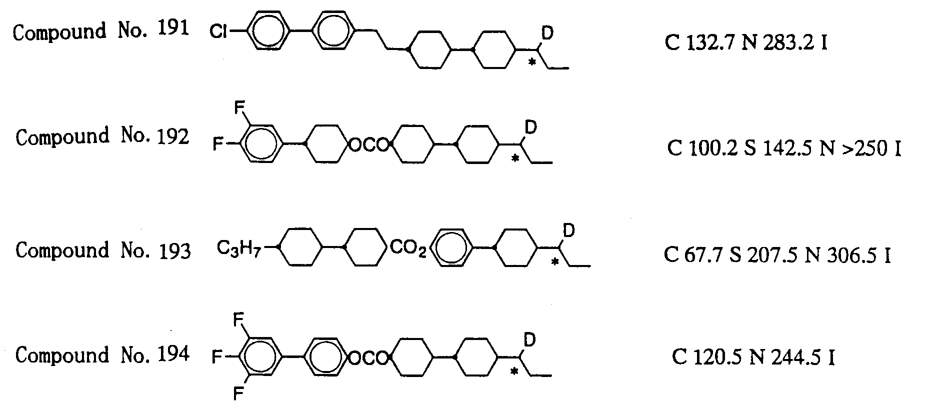

### Example 7

Liquid crystal composition examples 1 to 29 shown by

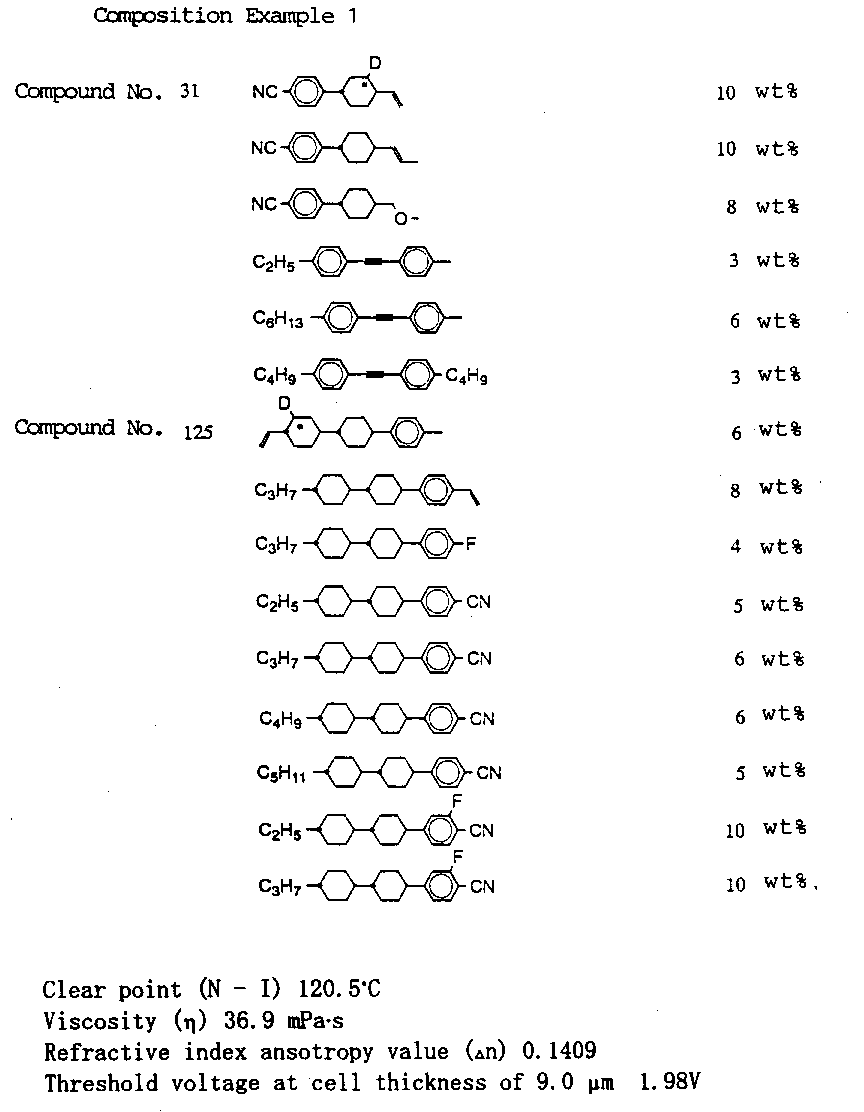

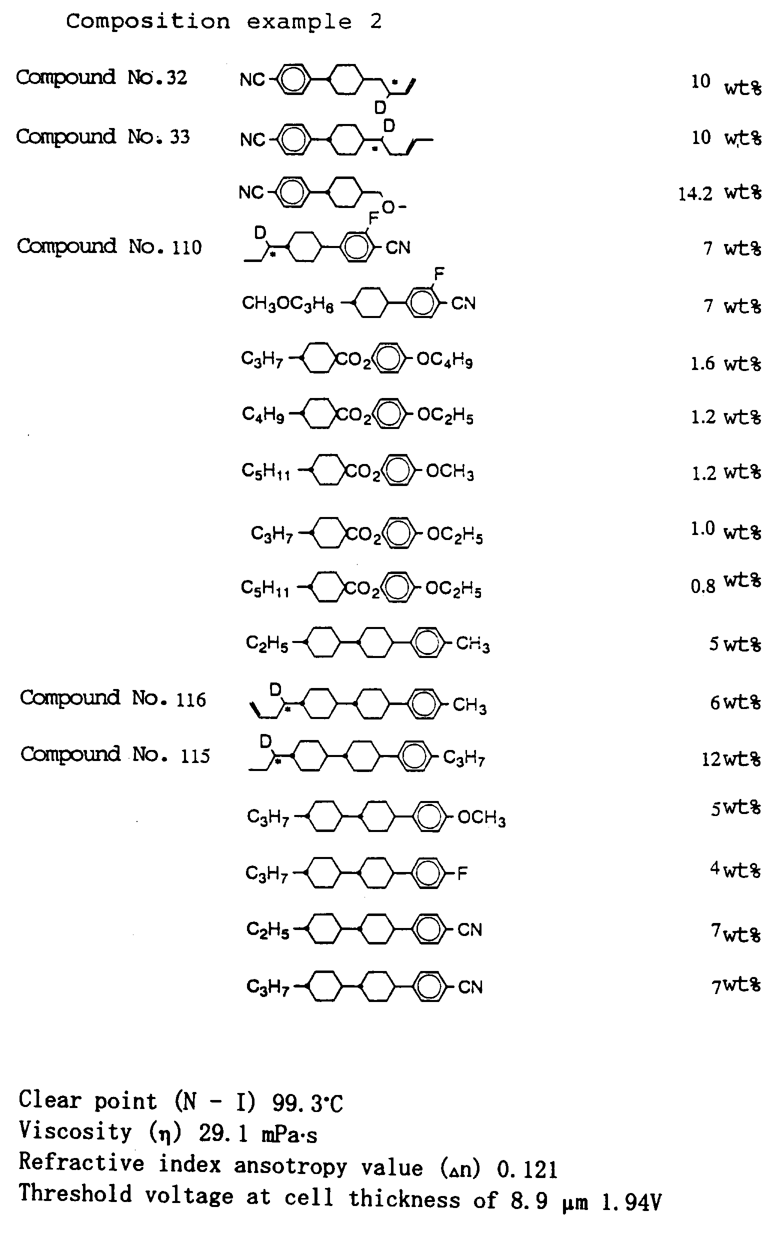

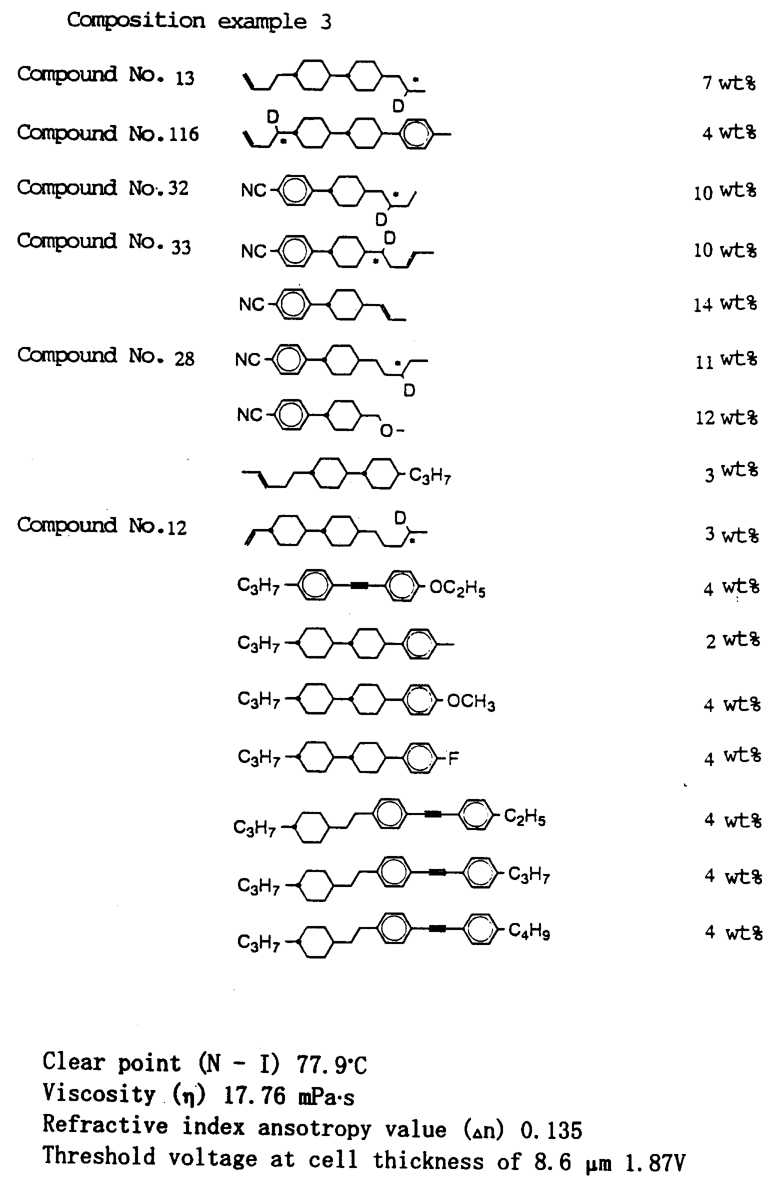

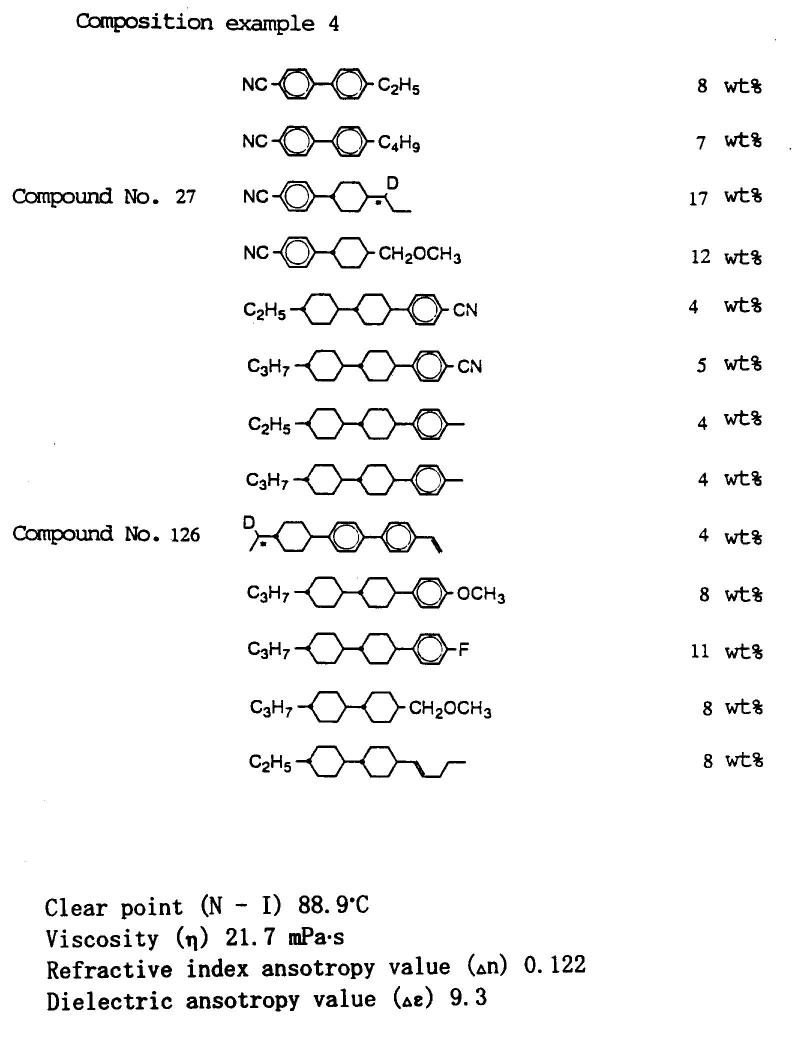

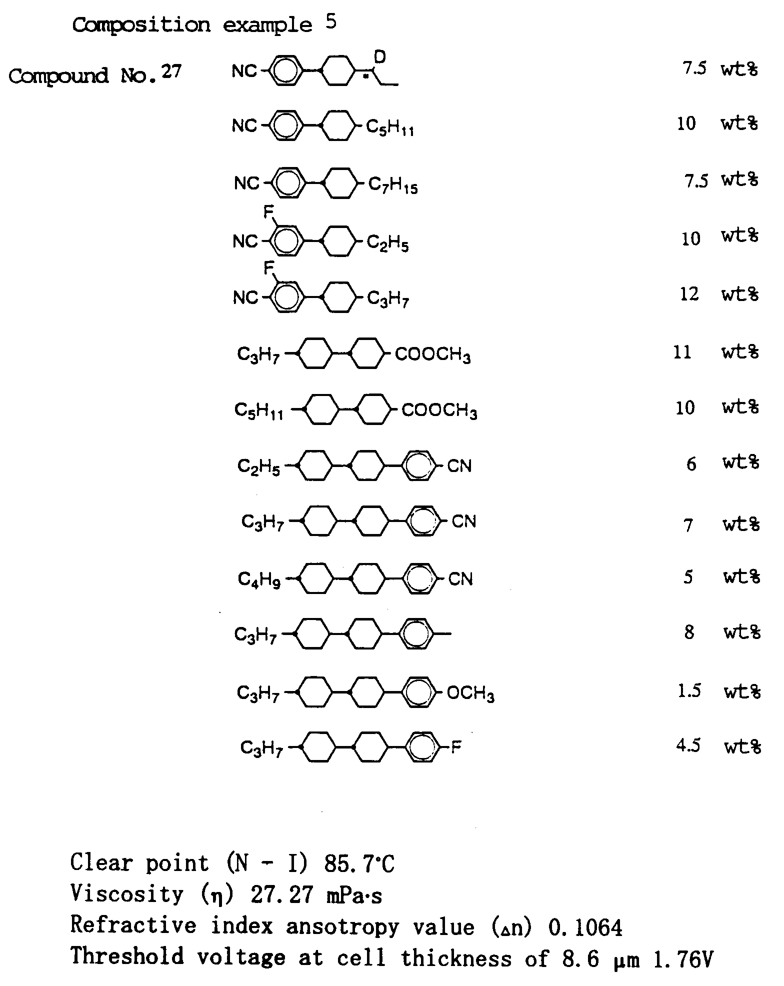

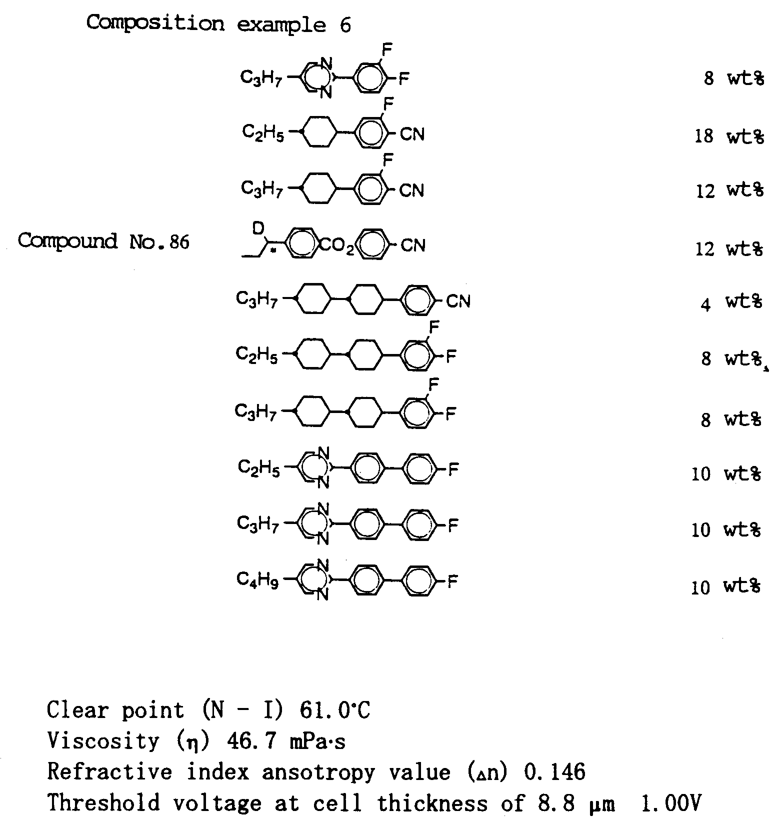

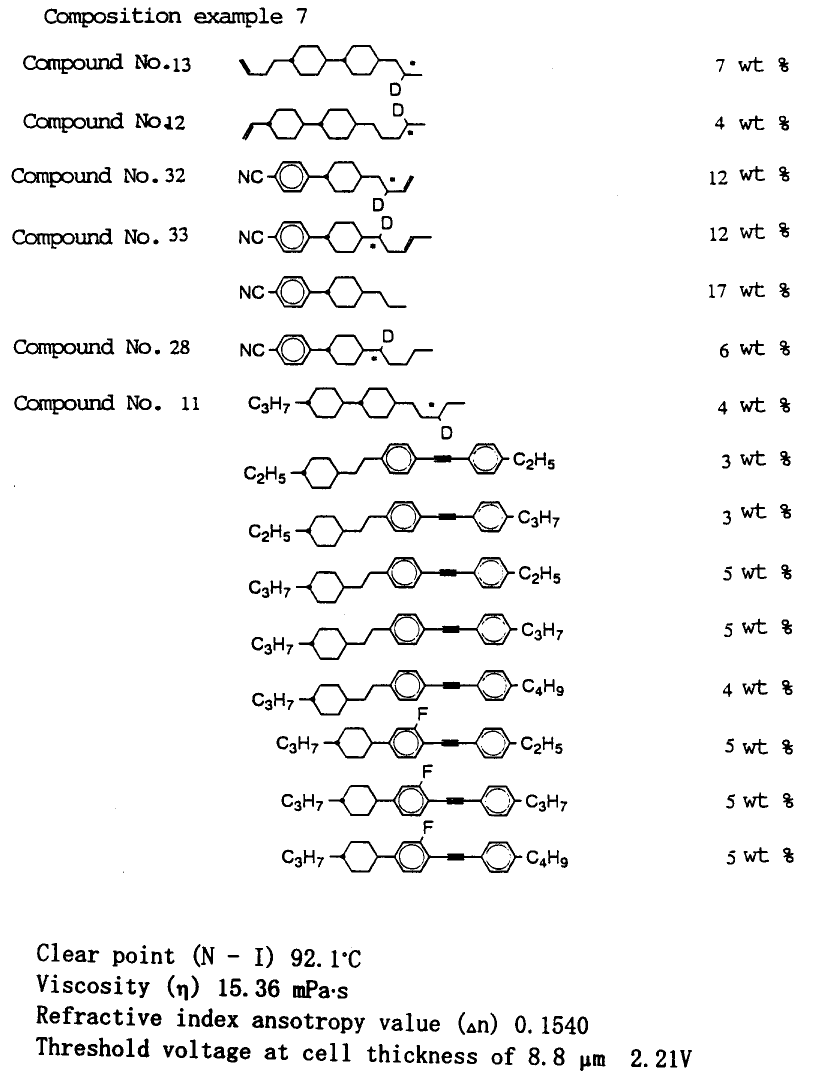

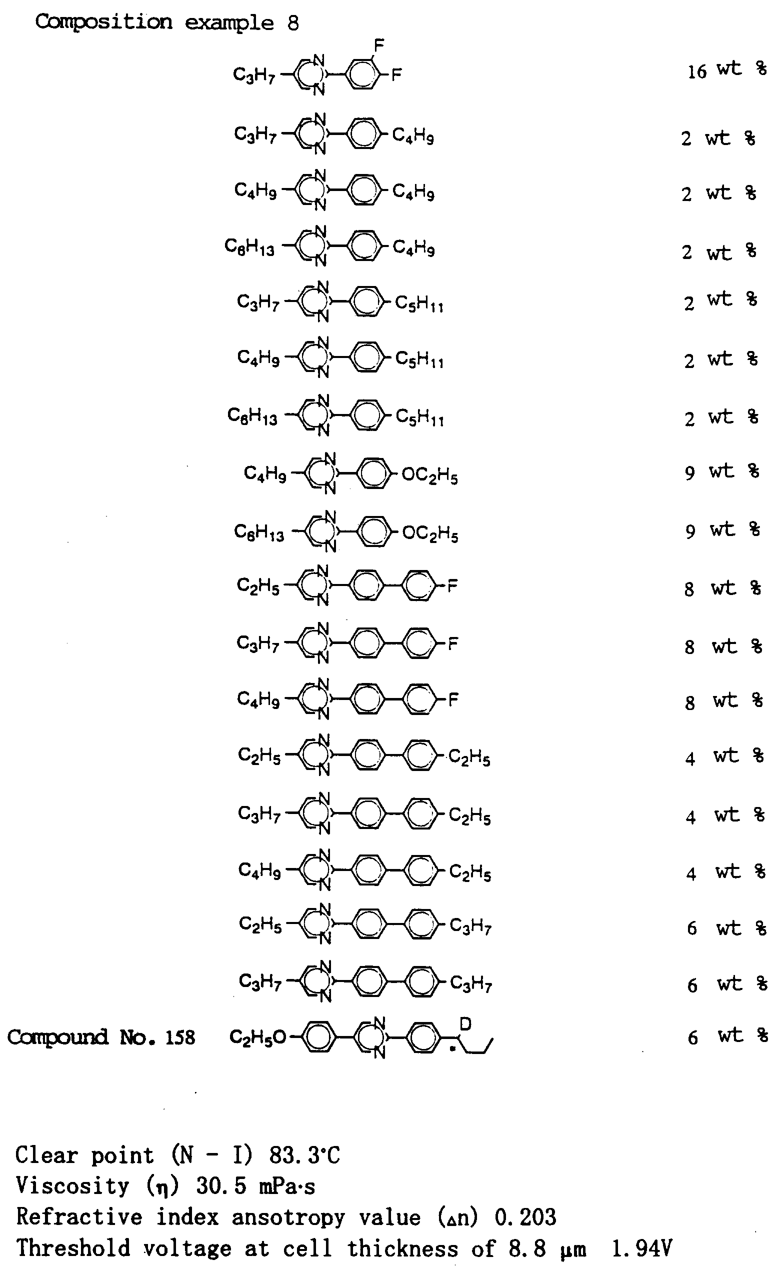

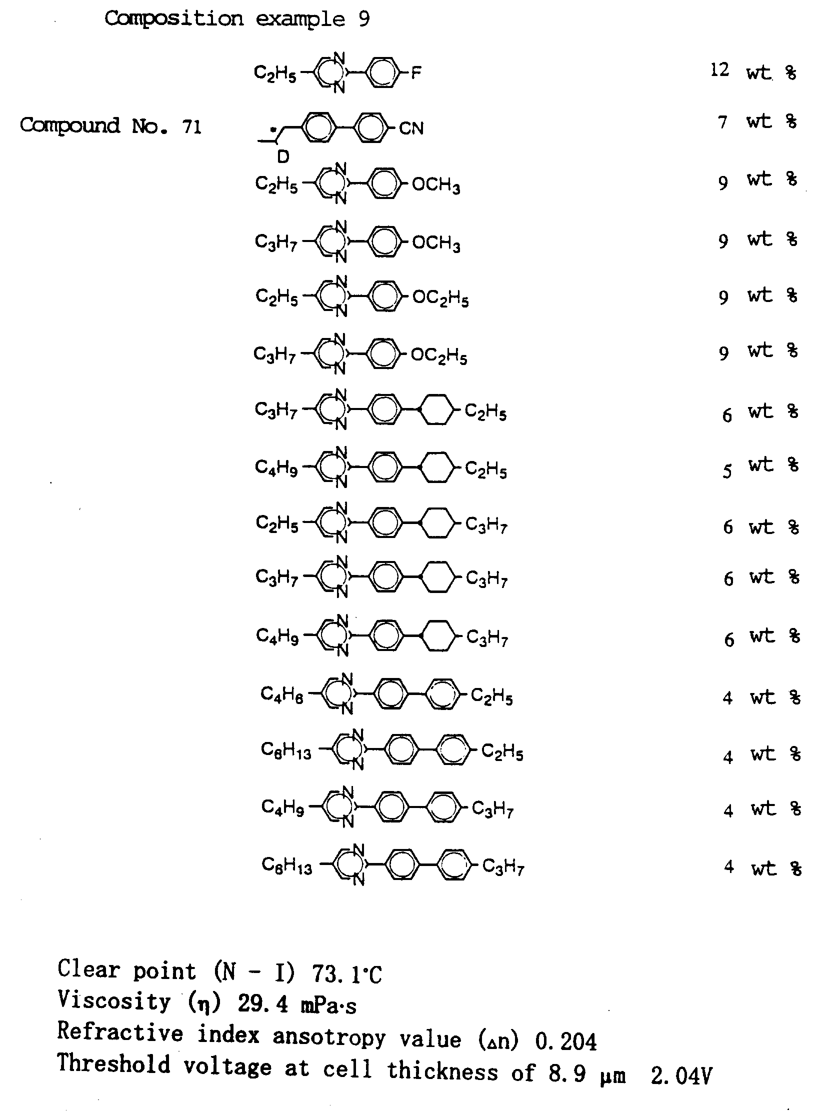

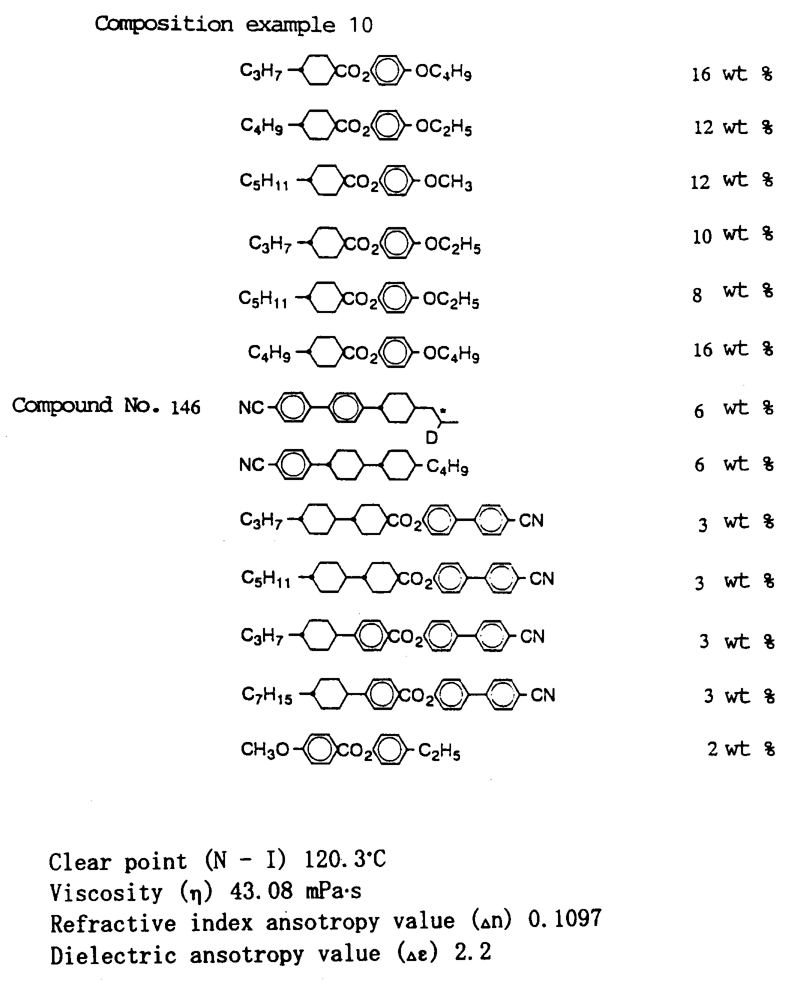

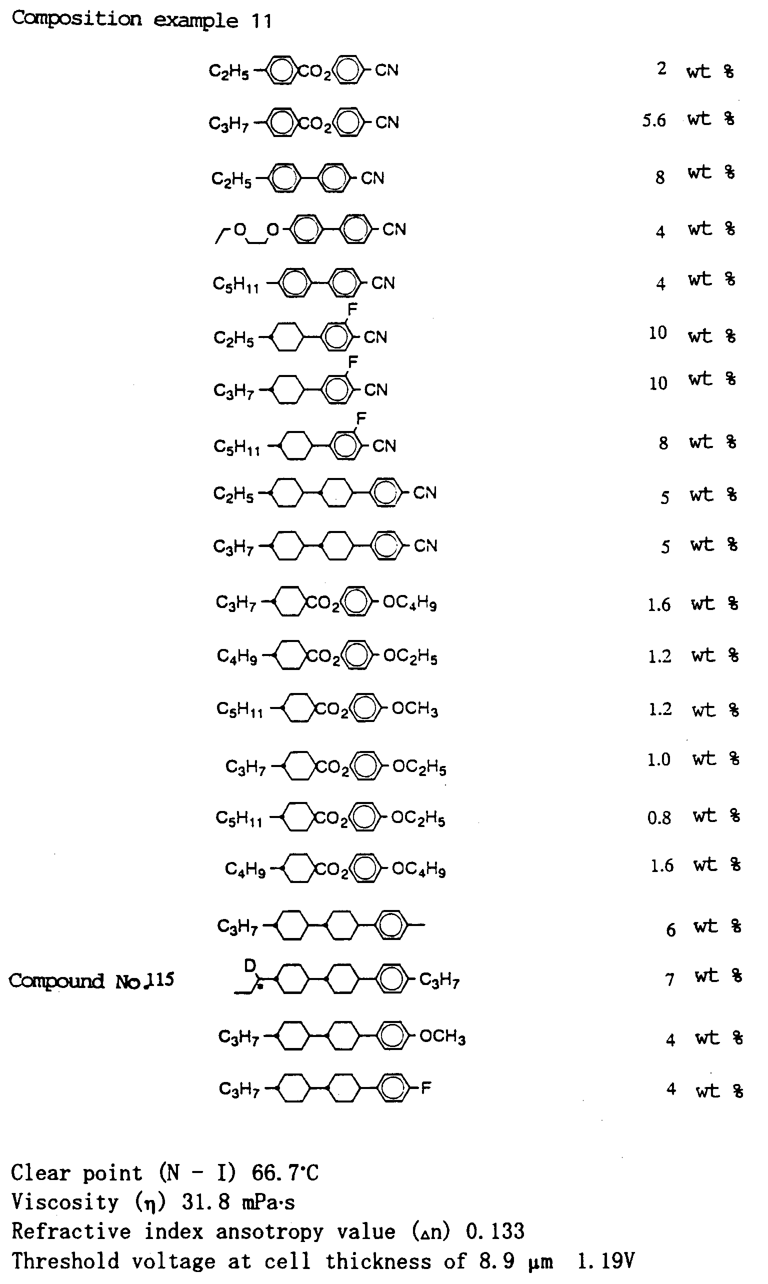

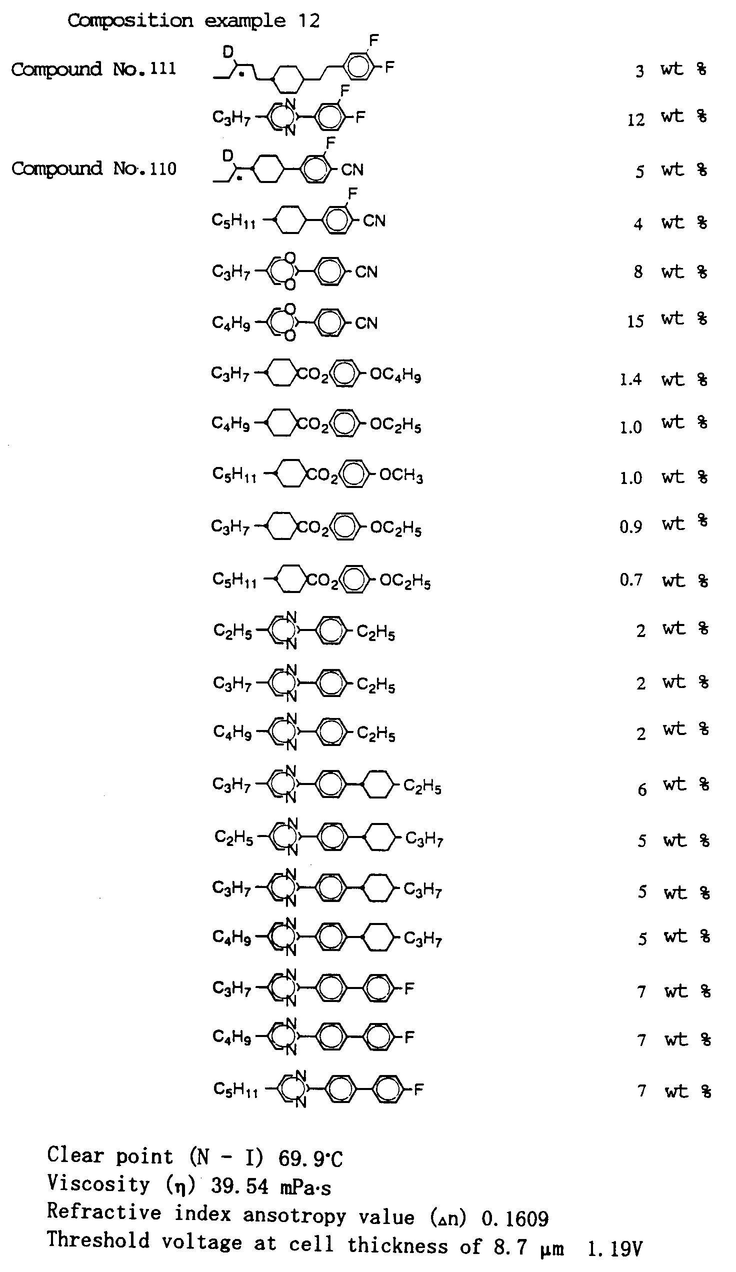

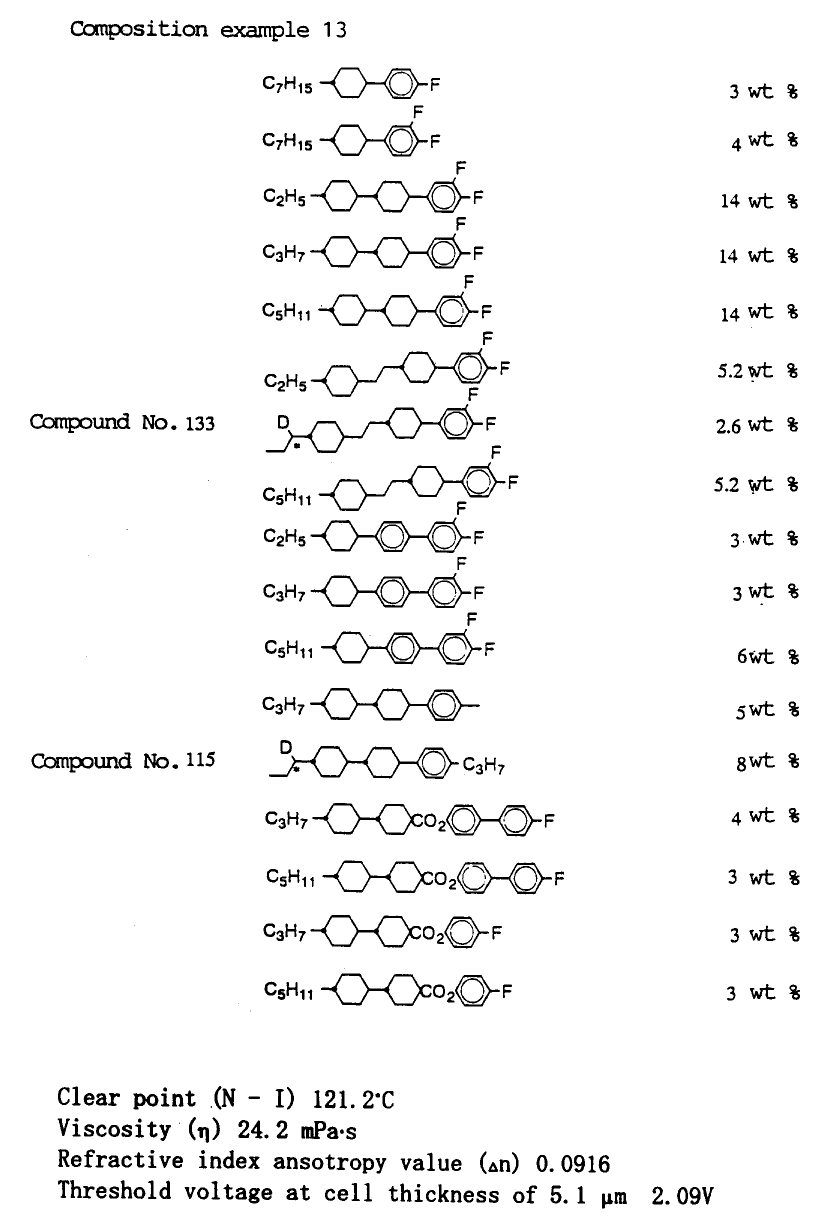

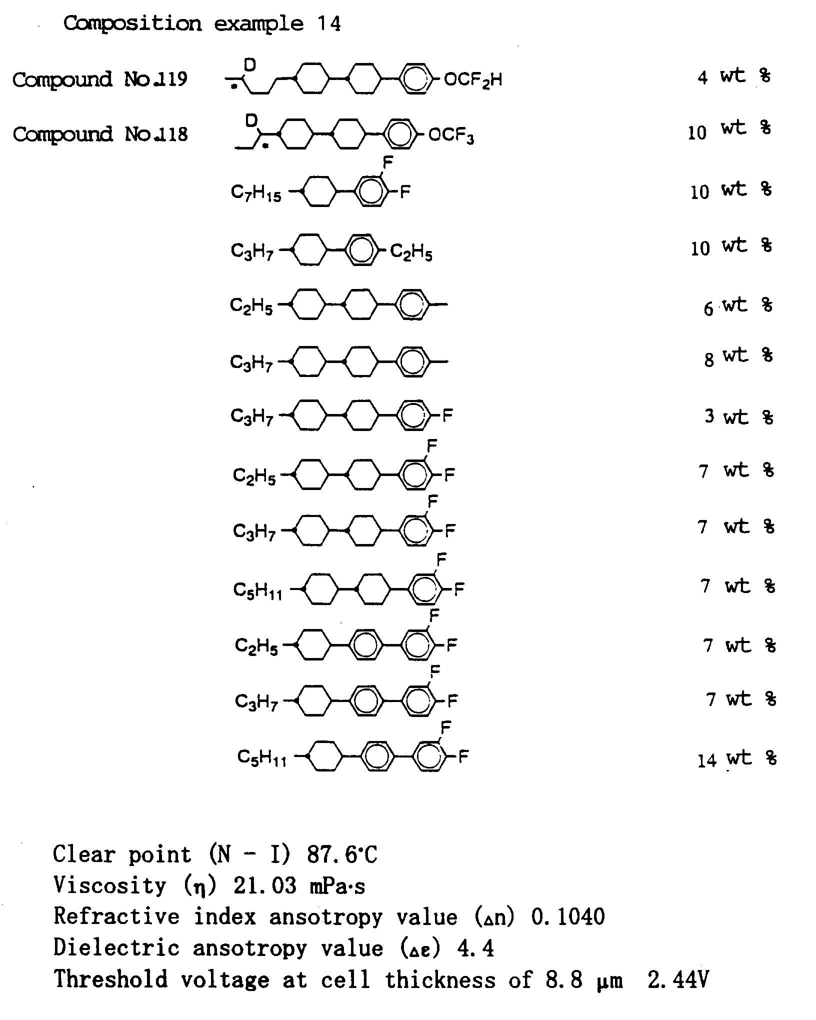

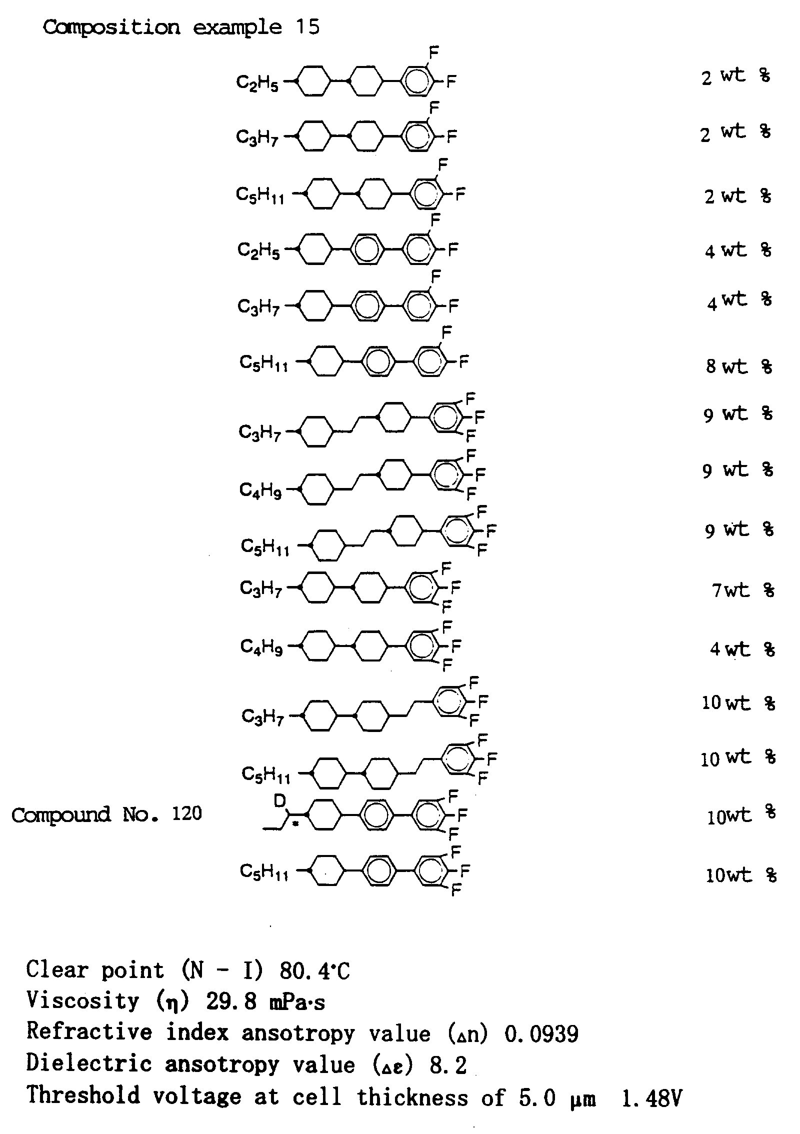

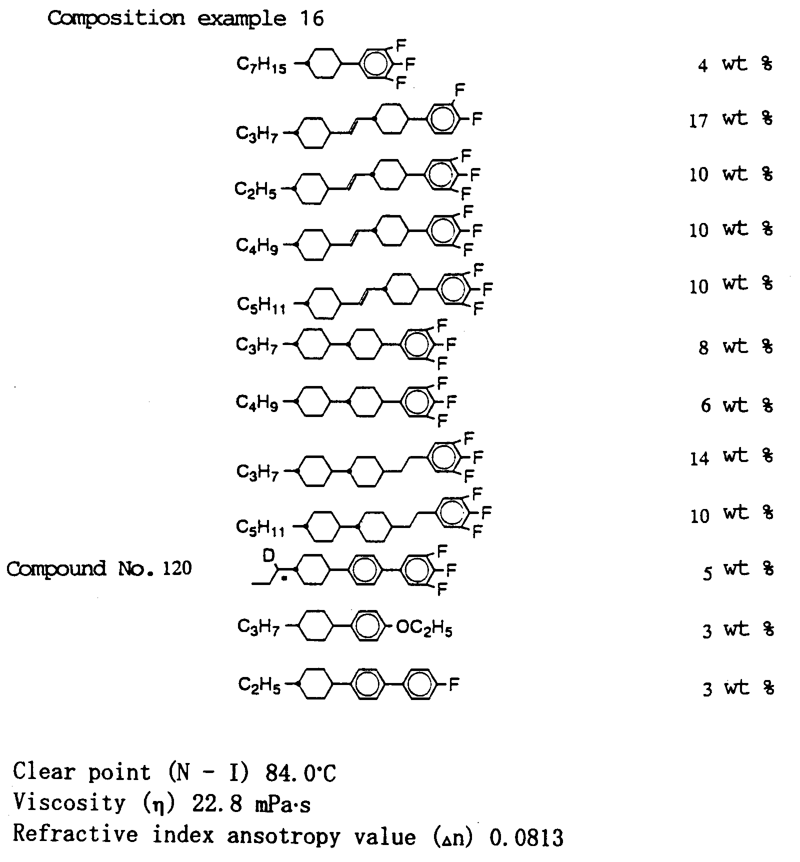

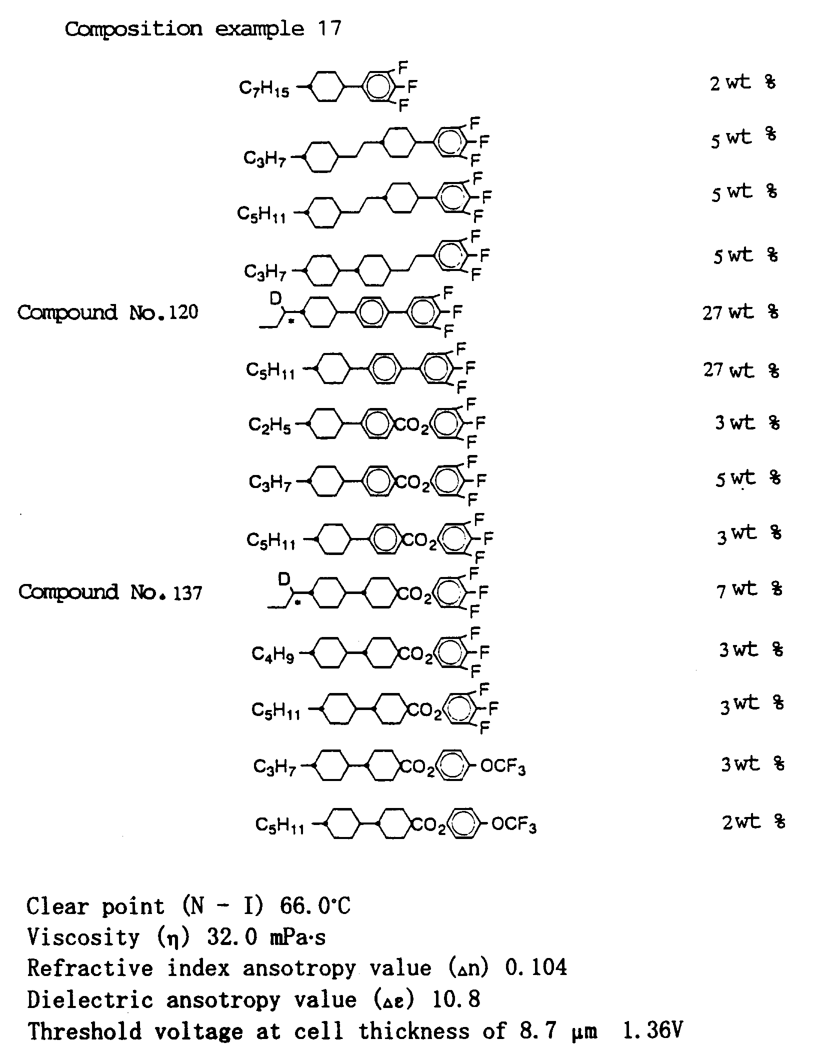

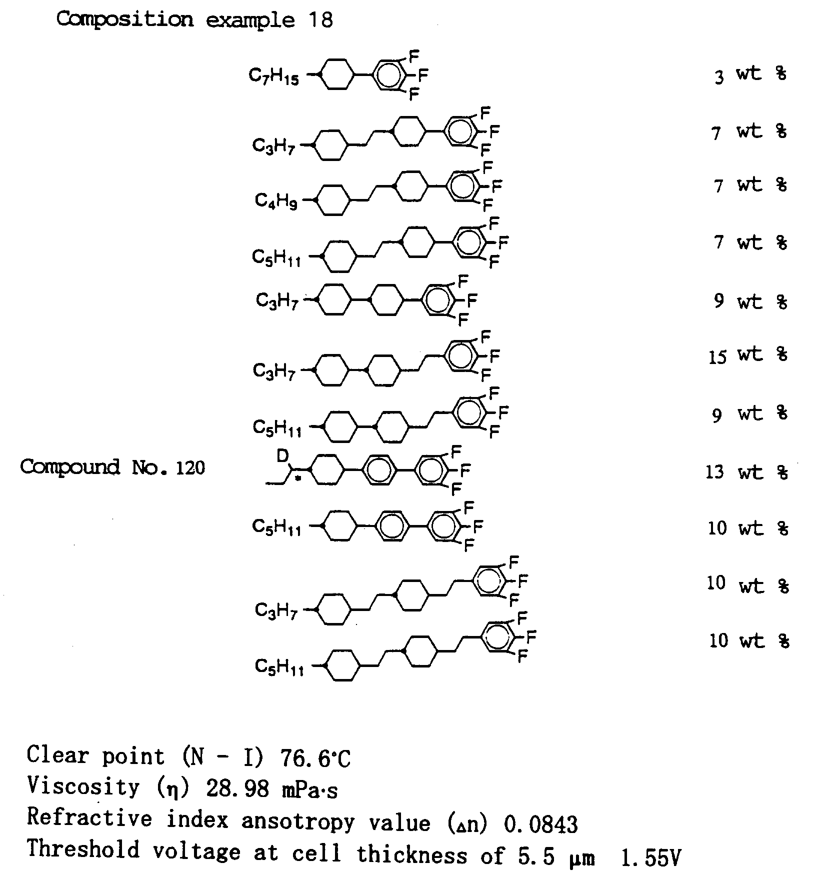

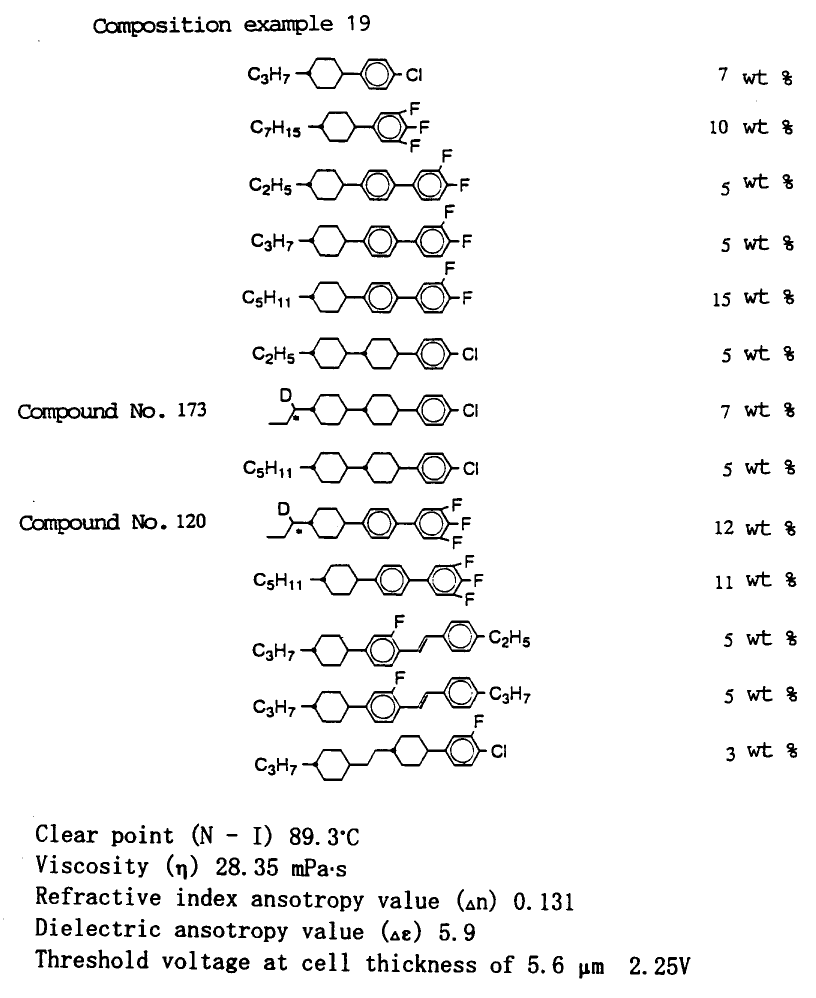

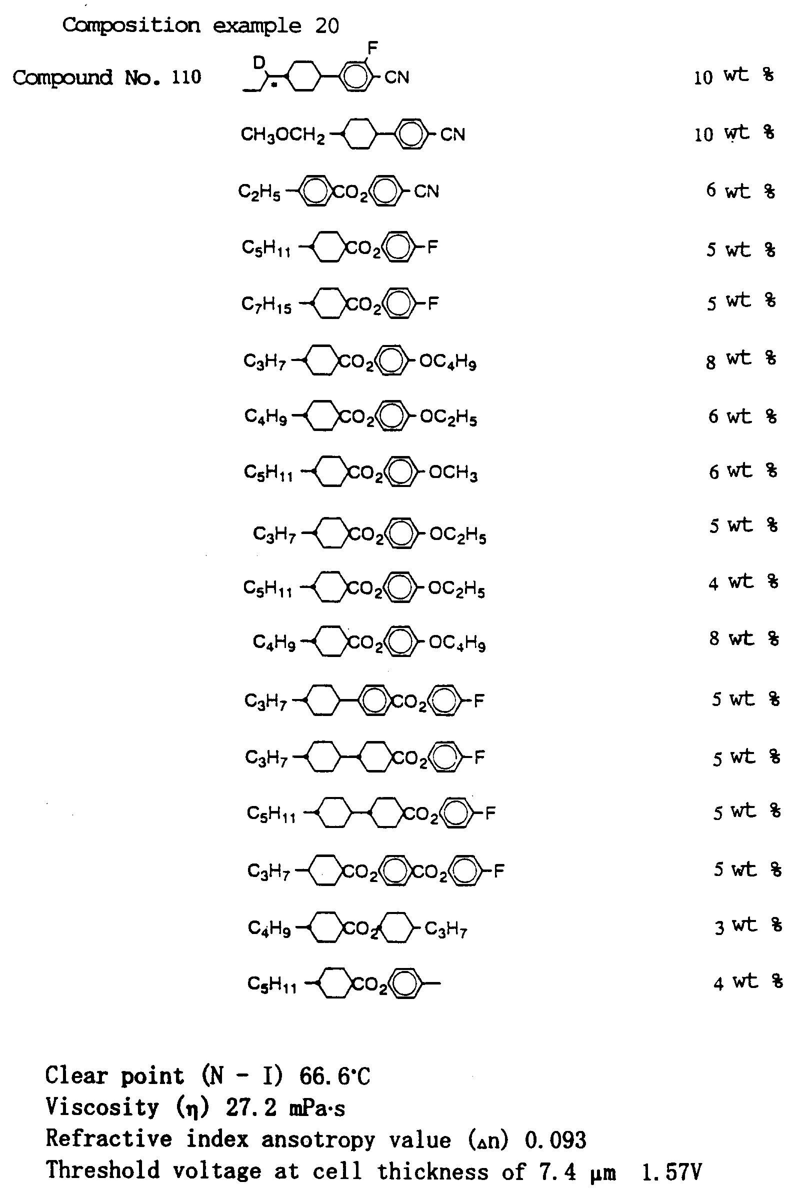

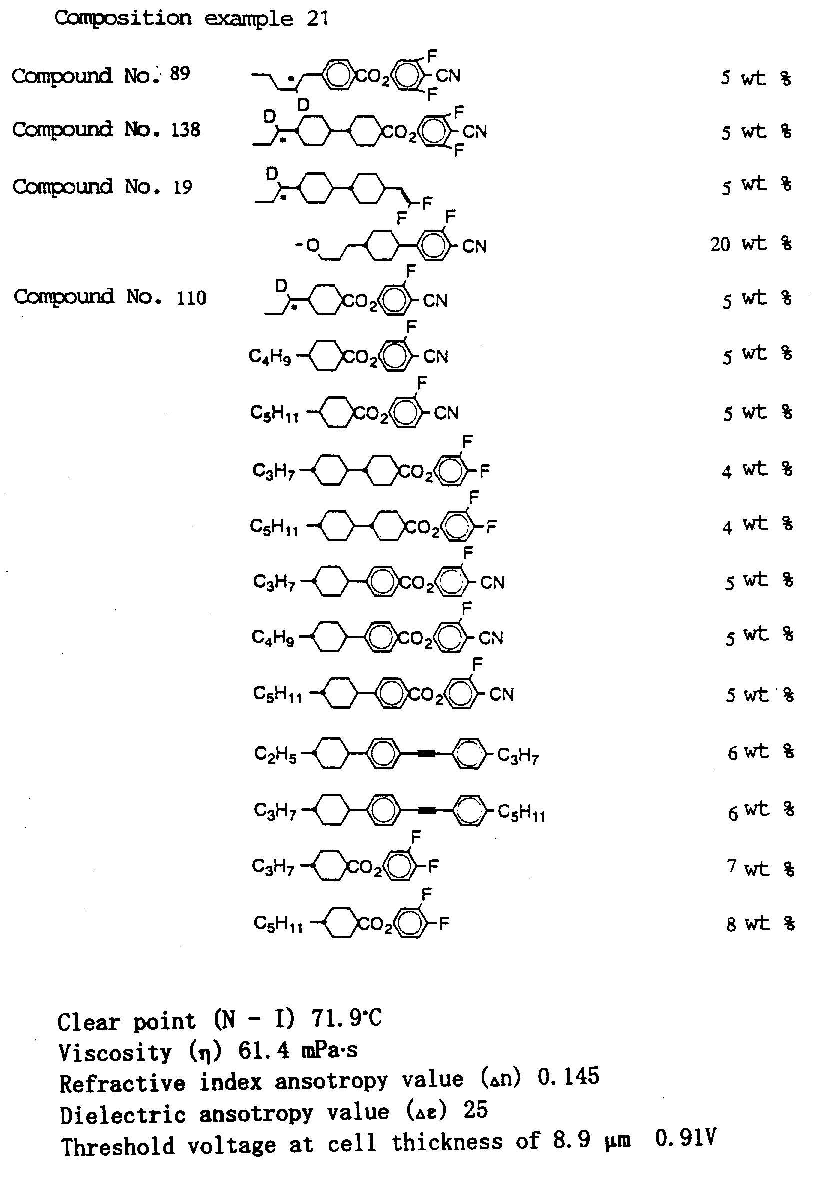

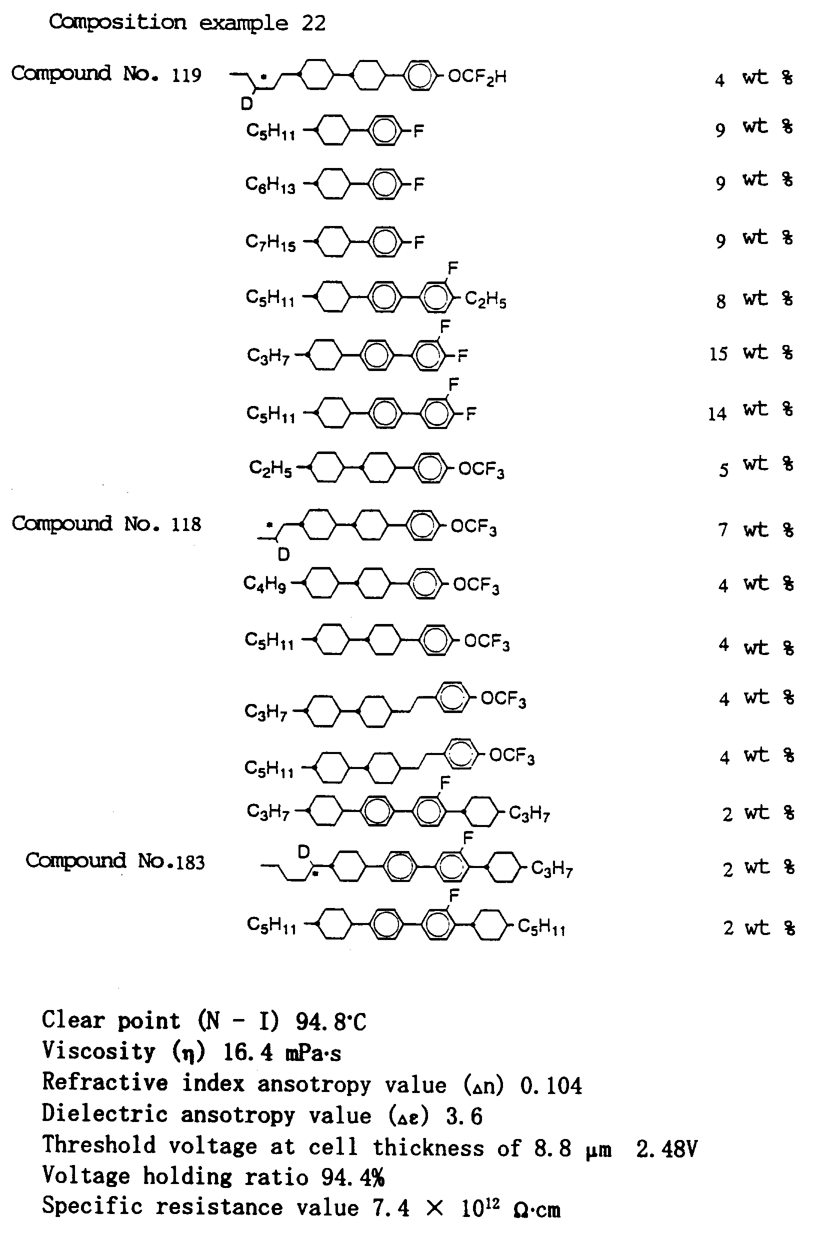

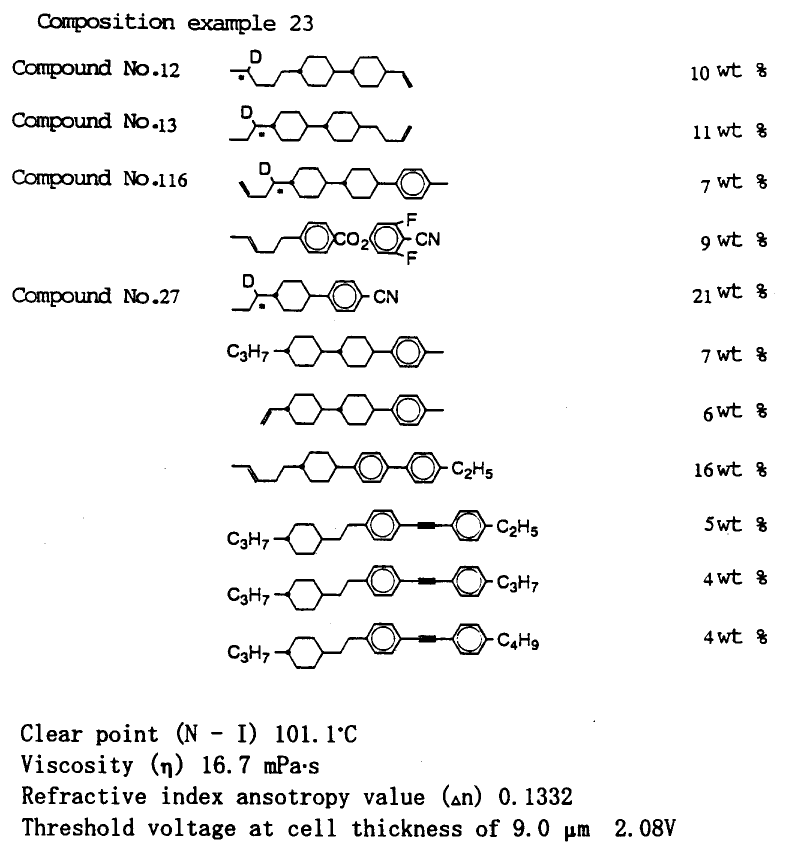

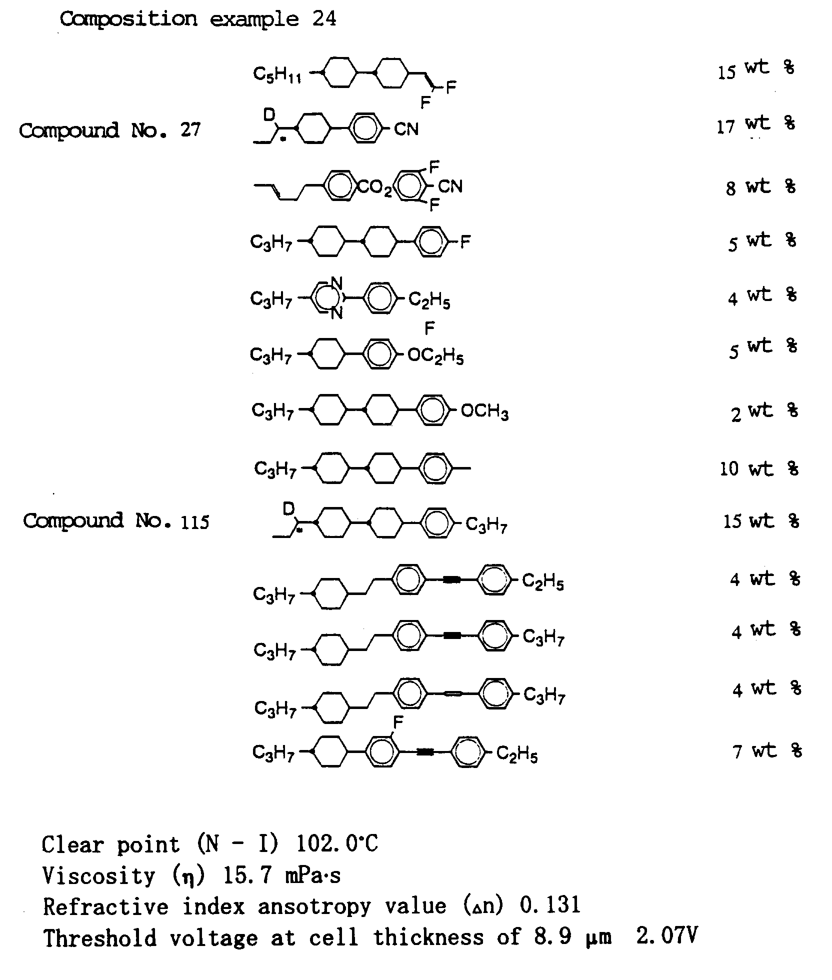

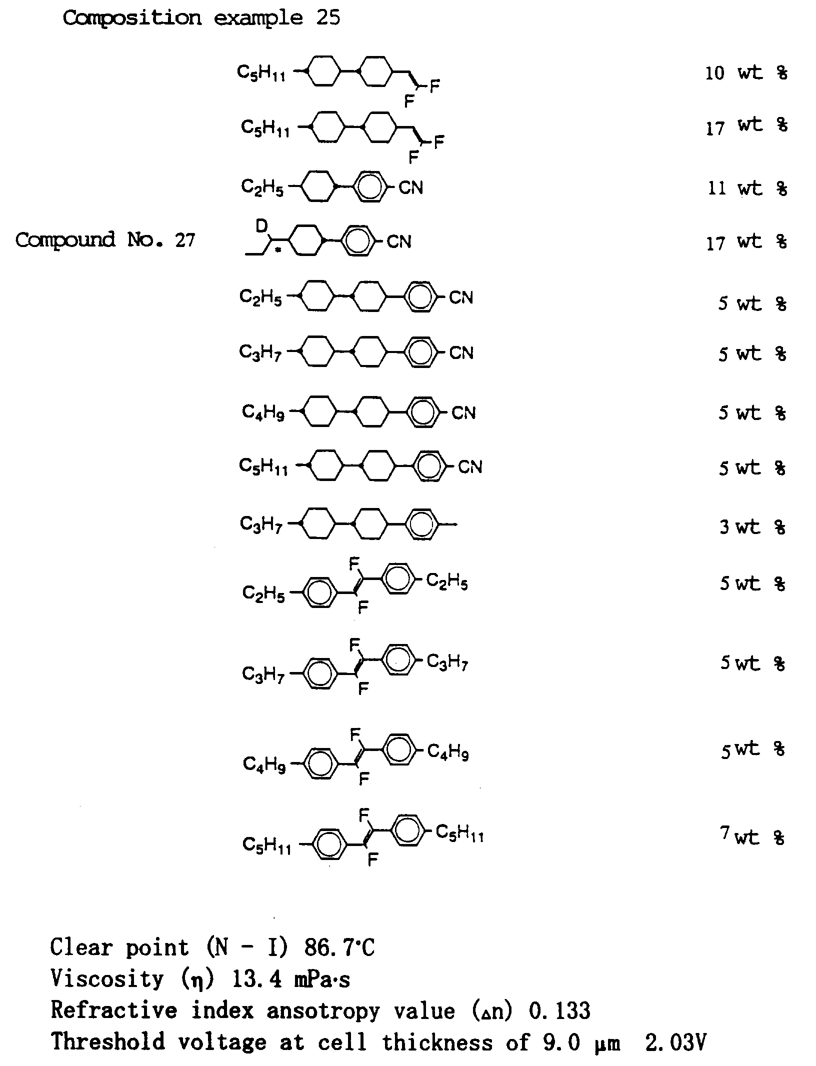

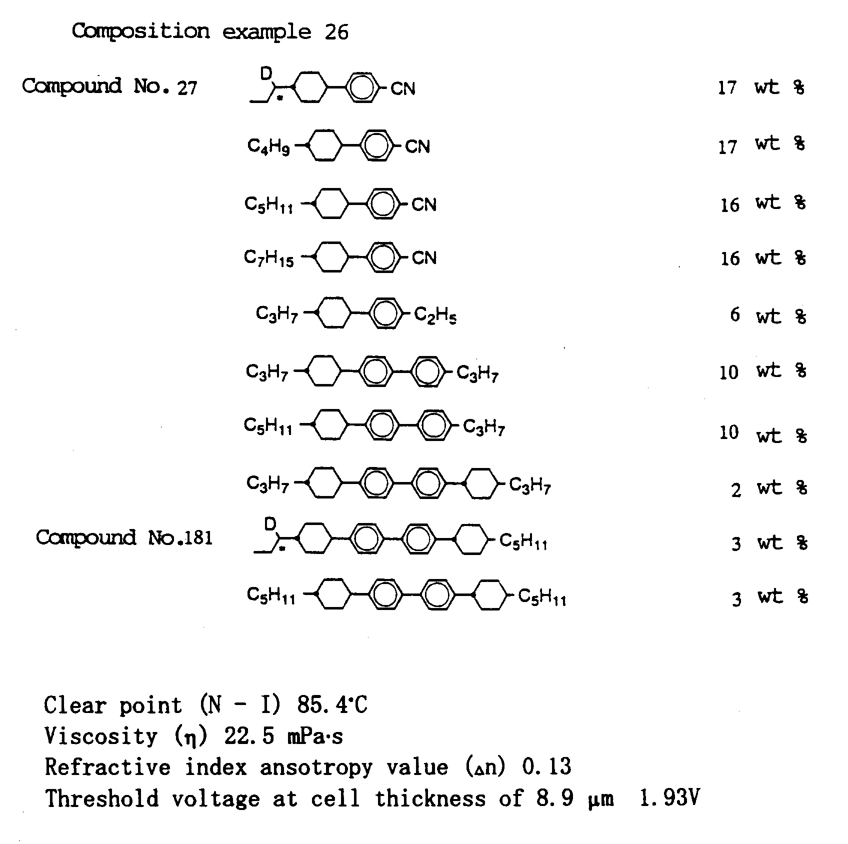

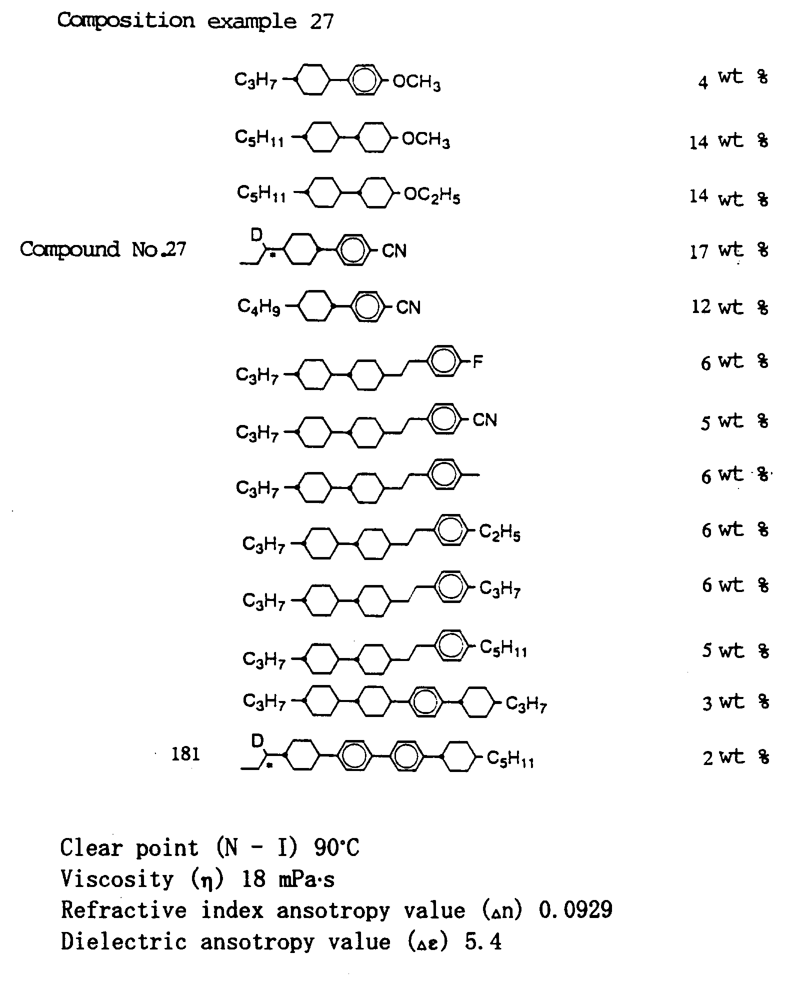

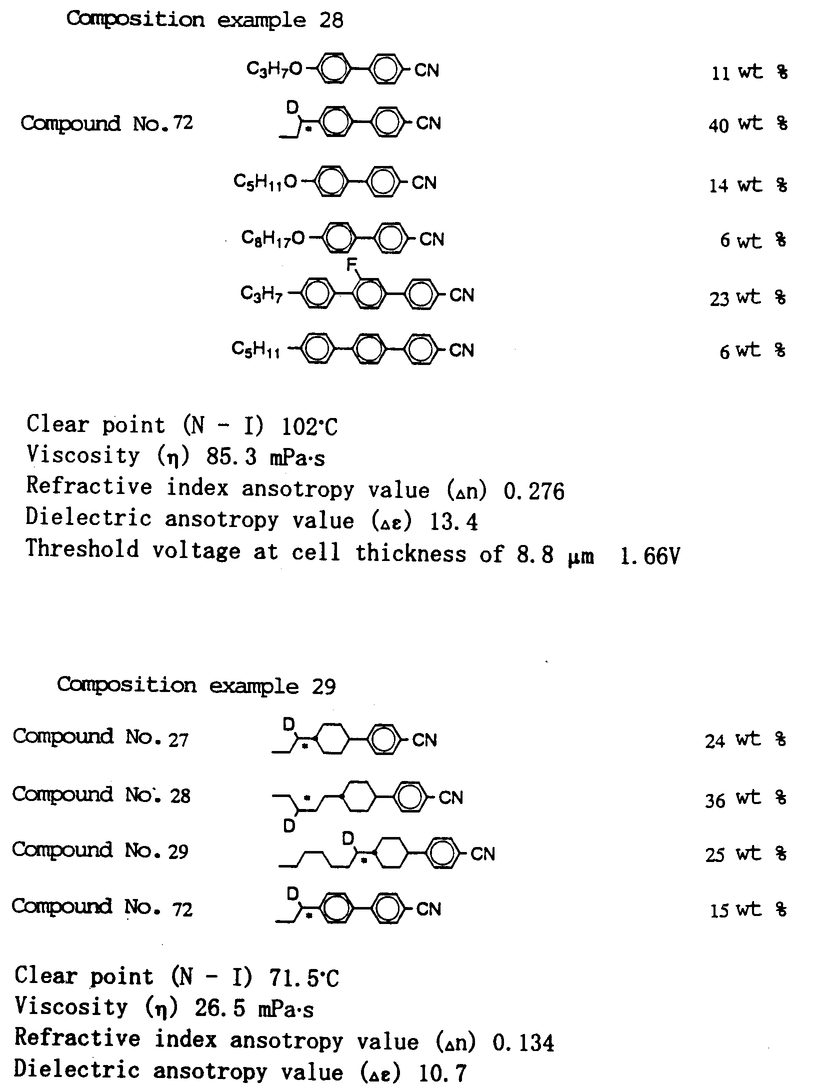

were produced by using the compounds shown in Examples 1 to 6 according to the invention.

### Advantage of the Invention

The liquid crystal compound expressed by the general formula (1) according to the invention with (an) optically active carbon atom (a) bonded to (a) deuterium atom(s) can be prepared industrially from the known compounds as shown in Examples.

As advantages of the compound expressed by the general formula (1) according to the invention, the following superior characteristics may be mentioned.
1) It is a compound having a characteristic as a chiral dopant, also. Thus, it is possible to prepare a liquid crystal composition without use of any conventional chiral dopant.
2) It is superior in its temperature-dependency of a pitch.
3) It is possible to control optionally a pitch length and a spiral direction by selecting a stereochemistry of the compound and (a) substitution position(s) of (a) deuterium atom(s).
4) It can be used as a ferroelectric liquid crystal composition.
5) It is superior in compatibility with other liquid crystal materials compared to the corresponding compounds without any deuterium substituent.

Thus, the compound expressed by the general formula (1) according to the invention is quite useful as a constitutive component of a practical liquid crystal material.

## Claims

1. An optically active compound with (an) optically active carbon atom(s) bonded to (a) deuterium atom(s).

2. A liquid crystal compound expressed by the general formula (1) 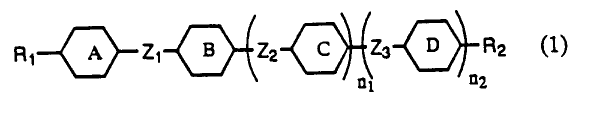 in which,
each of R₁ and R₂ independently denotes a hydrogen atom, a cyano group, a halogen atom, or an alkyl group or a halogenated alkyl group with 1 to 20 carbon atoms, wherein one or more than one methylene group in the said groups may be substituted by an oxygen atom, a sulfur atom, CH=CH, C≡C, CO, CF=CF, CF₂, or a cycloalkane ring or a cycloalkene ring with 3 to 5 carbon atoms, but two or more than two methylene groups cannot be continuously substituted by (an) oxygen atom(s) or (a) sulfur atom(s),
each of Z₁, Z₂ and Z₃ independently denotes a covalent bond or an alkylene group with 1 to 4 carbon atoms, wherein one or more than one methylene group in the said groups may be substituted by an oxygen atom, a sulfur atom, CH=CH, C≡C, CO, CF=CF, CF₂, or a cycloalkane ring or a cycloalkene ring with 3 to 5 carbon atoms, but two or more than two methylene groups cannot be continuously substituted by (an) oxygen atom(s) or (a) sulfur atom(s),
n₁ and n₂ are 0 or 1,
each of rings A, B, C and D independently denotes a benzene ring, a bicyclo[1.1.1]pentane ring, a bicyclo[2.1.1]hexane ring, a bicyclo[2.2.1]heptane ring, a bicyclo[2.2.2]octane ring, a naphthalene ring, a 1,2,3,4-tetrahydronaphthalene ring, a perhydronaphthalene ring, a fluorene ring, a phenanthrene ring, a 9,10-dihydrophenanthrene ring, an indane ring, an indene ring or a cycloalkane ring or (a) cycloalkene ring with 4 to 20 carbon atoms, wherein the carbon atom in the said rings may be substituted by a oxygen atom, a sulfur atom or a nitrogen atom,
one or more than one hydrogen atom in R₁, R₂, Z₁, Z₂, Z₃, rings A, B, C or D is substituted by (a) deuterium atom(s), and the carbon atom bonded to the deuterium atom(s) is(are) (an) asymmetric carbon atom(s) and optically active, and the hydrogen atom(s) in the structure may be substituted by (a) halogen atom(s) or a cyano group.

3. A liquid crystal compound according to Claim 2, wherein one or more than one hydrogen atom in R₁ or R₂ is(are) substituted by (a) deuterium atom(s).

4. A liquid crystal compound according to Claim 2, wherein one or more than one hydrogen atom in Z₁, Z₂ or Z₃ is(are) substituted by (a) deuterium atom(s).

5. A liquid crystal compound according to Claim 2, wherein one or more than one hydrogen atom in rings A, B, C or D is(are) substituted by (a) deuterium atom(s).

6. An intermediate for synthesizing a liquid crystal compound expressed by the general formula (1), wherein one or more than one hydrogen atom is substituted by (a) leaving group(s) such as an iodine atom, a bromine atom, a chlorine atom, a fluorine atom, a hydroxyl group, an alkanoyloxy group, a methanesulfonyl group, a benzenesulfonyl group or a 4-substituted benzenesulfonyloxy group, and wherein the carbon atom(s) bonded to the leaving group(s) is (an) asymmetric carbon atom(s).

7. A liquid crystal composition which is consistuted by using at least one liquid crystal compound according to any of Claims 1 to 5.

8. A liquid crystal composition characterized in that at least one liquid crystal compound according to any of Claims 1 to 5 is contained as the first component and at least one compound selected from the group consisting of general formulae (2), (3) and (4) 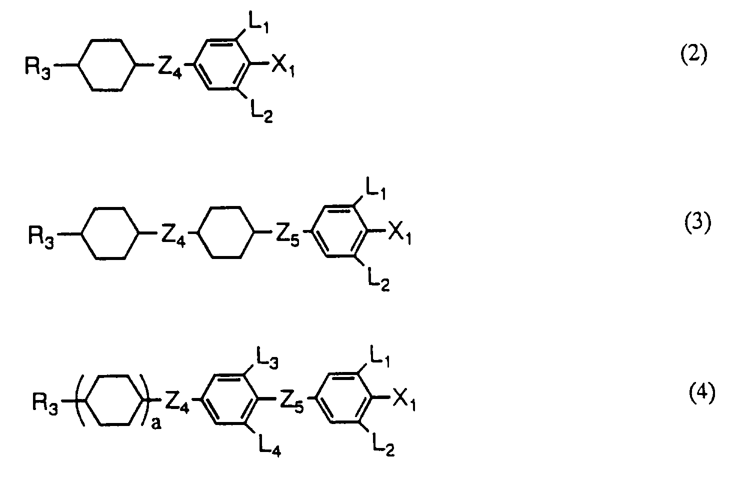 in which, R₃ denotes an alkyl group with 1 to 10 carbon atoms, X₁ denotes F, Cℓ, OCF₃, OCF₂H, CF₃, CF₂H or CFH₂, each of L₁, L₂, L₃ and L₄ independently denotes H or F, each of Z₄ and Z₅ independently denotes (CH₂)₂, CH=CH or a covalent bond, and a denotes 1 or 2,
is contained as the second component.

9. A liquid crystal composition characterized in that at least one liquid crystal compound according to any of Claims 1 to 5 is contained as the first component and at least one compound selected from the group consisting of general formulae (5), (6), (7), (8) and (9) 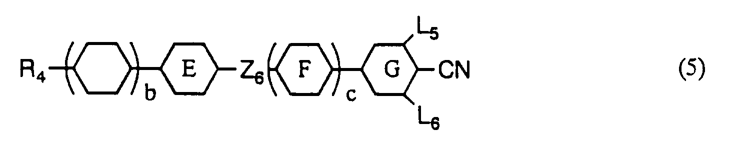 in which, R₄ denotes F, an alkyl group with 1 to 10 carbon atoms or an alkenyl group with 2 to 10 carbon atoms, wherein an optional methylene group in the said alkyl group or the alkenyl group may be substituted by (an) oxygen group(s) but two or more than two methylene groups cannot be continuously substituted by (an) oxygen atom(s), ring E denotes a cyclohexane ring, a benzene ring, a pyrimidine ring or a 1,3-dioxane ring, ring F denotes a cyclohexane ring, a benzene ring or a pyrimidine ring, ring G denotes a cyclohexane ring or a benzene ring, Z₆ denotes (CH₂)₂, COO or a covalent bond, each of L₅ and L₆ independently denotes H or F, and each of b and c independently denotes 0 or 1, 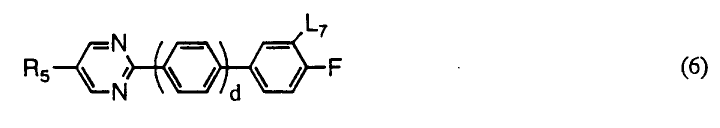 in which, R₅ denotes an alkyl group with 1 to 10 carbon atoms, R₇ denotes H or F, and d denotes 0 or 1, 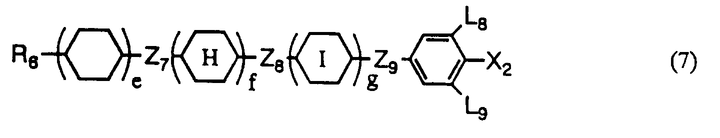 in which, R₆ denotes an alkyl group with 1 to 10 carbon atoms, each of rings H and I independently denotes a cyclohexane ring or a benzene ring, each of Z₇ and Z₈ independently denotes COO or a covalent bond, Z₉ denotes COO or C≡C, each of L₈ and L₉ independently denotes H or F, X₂ denotes F, OCF₃, OCF₂H, CF₃, CF₂H or CFH₂, but in the case that X₂ denotes OCF₃, OCF₂H, CF₃, CF₂H or CFH₂, both of L₈ and L₉ denote H, and each of e, f and g independently denotes 0 or 1, 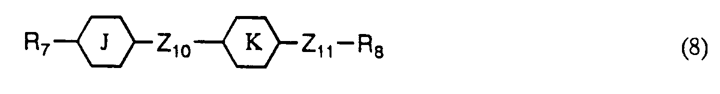 in which, each of R₇ and R₈ independently denotes an alkyl group with 1 to 10 carbon atoms or an alkenyl group with 2 to 10 carbon atoms, wherein (an) optional methylene group(s) thereof may be substituted by (an) oxygen atom(s) but two or more than two methylene groups cannot be continuously substituted by oxygen atoms, ring J denotes a cyclohexane ring, a benzene ring or a pyrimidine ring, ring K denotes a cyclohexane ring or a benzene ring, Z₁₀ denotes C≡C, COO, (CH₂)₂, CH=CH-C≡C or a covalent bond, and Z₁₁ denotes COO or a covalent bond, 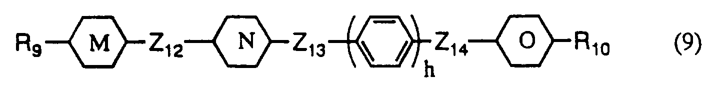 in which, each of R₉ and R₁₀ independently denotes an alkyl group with 1 to 10 carbon atoms or an alkenyl group with 2 to 10 carbon atoms, wherein (an) optional methylene group(s) thereof may be substituted by (an) oxygen atom(s) but two or more than two methylene groups cannot be continuously substituted by oxygen atoms, ring M denotes a cyclohexane ring, a benzene ring or a pyrimidine ring, ring N denotes a cyclohexane ring or a benzene ring in which one or more than one hydrogen atom may be substituted by F or (a) pyrimidine ring(s), ring O denotes a cyclohexane ring or a benzene ring, each of Z₁₂ and Z₁₄ independently denotes COO, (CH₂)₂ or a covalent bond, and Z₁₃ denotes CH=CH,
C≡C, COO or a covalent bond, and h denotes 0 or 1, is contained as the second component.

10. A liquid crystal composition characterized in that at least one liquid crystal compound according to any of Claims 1 to 5 is contained as the first component, at least one compound selected from the group consisting of general formulae (2), (3) and (4) is contained as a part of the second component, and at least one compound selected from the group consisting of general formulae (5), (6), (7), (8) and (9) is contained as another part of the second component.

11. A liquid crystal display element which is constituted by using a liquid crystal composition according to any of Claims 7 to 10.

12. A method for producing a compound of the general formula (1), characterized in that a racemic compound in which (a) hydrogen atom(s) being substituted by (a) hydroxyl group(s) is resoluted optically to obtain an optically active alcoholic substance, and that a hydroxyl part thereof is converted to (a) halogen or leaving group(s), and thereafter substituted by (a) deuterium atom(s).
